# EUROPEAN PATENT APPLICATION

(11) **EP 4 840 454 A2**
(43) Date of publication of application: **18.11.2026**
(21) Application number: 26204308.6
(22) Date of filing: 29.03.2024
(51) Int. Cl.: A61K 35/17

(54) **BISPECIFIC CHIMERIC ANTIGEN RECEPTORS TARGETING CD20 AND BCMA**

(30) Priority: 31.03.2023 US 202363493495 P; 21.06.2023 US 202363509371 P
(62) Document number(s) of the earlier application(s) in accordance with Art. 76 EPC: 24782064.0 / 4 626 447
(71) Applicant: AbelZeta Inc., Rockville, MD 20850 (US)
(72) Inventor: YAO, Yihong, Rockville, Maryland 20850 (US); HUANG, Jiaqi, Rockville, Maryland 20850 (US); ZHU, Shigui, Rockville, Maryland 20850 (US); YAO, Xin, Rockville, Maryland 20850 (US); LUO, Xiaobing, Rockville, Maryland 20850 (US); WEI, Yutian, Rockville, Maryland 20850 (US)
(74) Representative: Hoffmann Eitle

(57) **Abstract**

The present disclosure provides bispecific chimeric antigen receptors targeting CD20 and BCMA. The CAR may comprise an scFv targeting CD20 and an scFv targeting BCMA, a hinge region, a transmembrane domain, a co-stimulatory region, and a cytoplasmic signaling domain. The chimeric antigen receptors can be used to treat autoimmune disorders or cancer.

## Description

### CROSS REFERENCE TO RELATED APPLICATIONS

The present application claims priority to U.S. Provisional Patent Application Nos. 63/493,495 (filed on March 31, 2023) and 63/509,371 (filed on June 21, 2023), each of which is hereby incorporated by reference in its entirety.

### INCORPORATION-BY-REFERENCE OF SEQUENCE LISTING

A sequence listing, filed as the XML file "11299_011840-WO0_SL.xml" which was created on March 26, 2024 and is 168,342 bytes in size, is incorporated herein by reference in its entirety.

### FIELD OF THE INVENTION

The present disclosure relates to the field of immunotherapy, and more particularly to bispecific chimeric antigen receptors (CARs) targeting CD20 and BCMA.

### BACKGROUND

Autoimmune diseases are conditions caused by the immune system's response to the body itself, resulting in damage to its own tissues. These are typically divided into two main categories: systemic autoimmune diseases, such as systemic lupus erythematosus (SLE), rheumatoid arthritis, and systemic vasculitis; and organ-specific autoimmune diseases, such as autoimmune hepatitis and type I diabetes. Most autoimmune diseases are difficult to cure and often require long-term or lifelong medication. Treatment primarily involves corticosteroids and immunosuppressants, greatly impacting the patient's quality of life and presenting a significant unmet clinical need (Wang et al., Human autoimmune diseases: a comprehensive update, J. Intern. Med. 2015, 278(4):369-95).

The etiology of autoimmune diseases is unclear. In patients, abnormal activation of humoral immunity occurs, leading to the production of a large number of antibodies against self-antigens. These combine to form pathogenic immune complexes, which then deposit locally and cause inflammatory reactions. B cells play an important role in the pathogenesis of autoimmune diseases, promoting the occurrence of autoimmune diseases through various mechanisms such as producing autoantibodies, releasing cytokines, and presenting autoantigens. Autoantibodies, as a key factor, can bind with autoantigens to form immune complexes, which can activate innate immune system cells to produce type I interferon and other pro-inflammatory cytokines resulting in organ damage. Therefore, the depletion or removal of lymphocytes has become a potential treatment strategy.

SLE is a prototypic autoimmune disease that is known to be associated with polyclonal B-cell hyperreactivity (Dorner et al., Mechanisms of B cell autoimmunity in SLE, Arthritis Res. Ther. 13, 243 (2011)). As such, one of the immunological hallmarks of SLE is the production of antinuclear antibodies (ANAs), which can mediate SLE pathogenesis by binding to respective autoantigens, resulting in deposition of immune complexes and induction of inflammation and organ damage (for example, lupus nephritis) (Salmon, J.E., Arming T cells against B cells in systemic lupus erythematosus, Nat. Med. 28, 2009-2010 (2022)). There are two main types of ANAs: anti-DNA antibodies and antibodies recognizing RNA-binding proteins (RBP) (Pisetsky et al., New insights into the role of antinuclear antibodies in systemic lupus erythematosus, Nat. Rev. Rheumatol. 16, 565-579 (2020)). In patients with SLE, the sources of autoantibodies include not only B cells but also a subset of plasma cells termed long-lived plasma cells (LLPCs). While the anti-DNA antibodies are produced by naïve B cells that transition to memory B cells and plasmablasts, which maintain high level expression of CD19 and CD20 on the cell surface, the anti-RBP antibodies are produced by LLPCs, which may lose surface expression of CD19 and CD20, but are positive for B-cell maturation antigen (BCMA), a cell surface protein expressed on all mature plasma cells (Dogan et al., B-cell maturation antigen expression across hematologic cancers: a systematic literature review. Blood Cancer J. 10, 73 (2020); Morgan et al., Unraveling B cell trajectories at single cell resolution, Trends Immunol. 43, 210-229 (2022)). Recent studies demonstrated that a CD11c^{hi}T-bet⁺ B cell subset is expanded in human SLE and serves as precursors of autoantibody producing plasma cells. This B cell subset displays high expression of CD19 and CD20 and corresponds to the autoreactive, murine age-associated B cells (autoreactive B cells or ABCs; the term may be used to represent human CD11c^{hi}T-bet⁺ B cells) (Jenks et al., Distinct Effector B Cells Induced by Unregulated Toll-like Receptor 7 Contribute to Pathogenic Responses in Systemic Lupus Erythematosus, Immunity 49, 725-739 e726 (2018); Wang et al., IL-21 drives expansion and plasma cell differentiation of autoreactive CD11c(hi)T-bet(+) B cells in SLE, Nat. Commun. 9, 1758 (2018)). In addition to autoantibody production, B cells also participate in the pathogenesis of SLE and other autoimmune diseases by secreting cytokines and acting as antigen-presenting cells. Therefore, depleting B cells in patients with SLE can be an effective therapy for this life-threatening disease.

B cell depletion could be achieved by administration of monoclonal antibodies against B cell surface markers. Although the anti-CD20 antibody rituximab was successful in early open-label trials in SLE, it failed to meet its primary end points in two randomized controlled trials (Lee et al., B cell depletion therapies in autoimmune disease: advances and mechanistic insights, Nat. Rev. Drug Discov. 20, 179-199 (2021)). Other antibodies targeting CD19 (obexelimab) were also tested in SLE. Although patients receiving obexelimab sustained their level of disease inactivity despite steroid withdrawal in initial studies, phase II clinical trials, failed to meet their primary end points (Lee et al., B cell depletion therapies in autoimmune disease: advances and mechanistic insights, Nat. Rev. Drug Discov. 20, 179-199 (2021)).

One promising approach to achieve B cell depletion is adoptive transfer of CAR-T cells. CAR-T cells are genetically engineered T lymphocytes that, in the absence of major histocompatibility complex (MHC), can recognize specific antigens on target cells, proliferate, and generate cytotoxic immune responses. In a recent study, compassionate-use of CD19 CAR-T therapy in 5 patients with refractory SLE led to deep depletion of B cells and drug-free remission, suggesting that CAR-T cell transfer is feasible, tolerable, and highly effective in SLE (Mackensen et al., Anti-CD19 CAR T cell therapy for refractory systemic lupus erythematosus, Nat. Med. 28, 2124-2132 (2022)).

There is still an urgent need to develop methods to effectively treat autoimmune diseases.

### SUMMARY

The present disclosure provides for a bispecific chimeric antigen receptor (CAR), comprising: (i) an anti-CD20 antigen-binding region which comprises a light chain variable region (V_{L}1) and a heavy chain variable region (V_{H}1), wherein V_{L}1 comprises three complementarity determining regions (CDRs), CDR1, CDR2 and CDR3, having amino acid sequences about 80% to about 100% identical to the amino acid sequences set forth in SEQ ID NO: 130, SEQ ID NO: 131, SEQ ID NO: 132, respectively, and wherein V_{H}1 comprises three CDRs, CDR1, CDR2 and CDR3, having amino acid sequences about 80% to about 100% identical to the amino acid sequences set forth in SEQ ID NO: 127, SEQ ID NO: 128, SEQ ID NO: 129, respectively; and (ii) an anti-BCMA antigen-binding region which comprises a light chain variable region (V_{L}2) and a heavy chain variable region (V_{H}2), wherein V_{L}2 comprises three complementarity determining regions (CDRs), CDR1, CDR2 and CDR3, having amino acid sequences about 80% to about 100% identical to the amino acid sequences set forth in SEQ ID NO: 134, SEQ ID NO: 136, SEQ ID NO: 138, respectively, and wherein V_{H}2 comprises three CDRs, CDR1, CDR2 and CDR3, having amino acid sequences about 80% to about 100% identical to the amino acid sequences set forth in SEQ ID NO: 141, SEQ ID NO: 143, SEQ ID NO: 145, respectively.

The present disclosure provides for a bispecific chimeric antigen receptor (CAR), comprising: (i) an anti-CD20 antigen-binding region which comprises a light chain variable region (V_{L}1) and a heavy chain variable region (V_{H}1); and (ii) an anti-BCMA antigen-binding region which comprises a light chain variable region (V_{L}2) and a heavy chain variable region (V_{H}2).

In one embodiment, V_{L}1 is located at the N-terminus of V_{H}1. In one embodiment, V_{H}1 is located at the N-terminus of V_{L}1. In one embodiment, V_{L}2 is located at the N-terminus of V_{H}2. In one embodiment, V_{H}2 is located at the N-terminus of V_{L}2.

In certain embodiments, V_{L}1 and V_{H}1 have amino acid sequences about 80% to about 100% identical to amino acid sequences set forth in SEQ ID NO: 4 and SEQ ID NO: 8, respectively.

In certain embodiments, V_{L}2 and V_{H}2 have amino acid sequences about 80% to about 100% identical to amino acid sequences set forth in SEQ ID NO: 12 and SEQ ID NO: 16, respectively.

The anti-CD20 antigen-binding region may be a single-chain variable fragment (scFv) that specifically binds CD20. The anti-BCMA antigen-binding region may be a scFv that specifically binds BCMA.

The bispecific CAR may further comprise one or more of the following: (a) a signal peptide, (b) a hinge region, (c) a transmembrane domain, (d) a co-stimulatory region, and (e) a cytoplasmic signaling domain.

The hinge region may comprise a hinge region of IgG4, CD8, CD28, CD137, or combinations thereof.

The transmembrane domain may comprise a transmembrane domain of CD8, CD28, CD3ε, CD45, CD4, CD5, CD9, CD16, CD22, CD33, CD37, CD64, CD80, CD86, CD134, CD137, CD154, or combinations thereof.

The co-stimulatory region may comprise a co-stimulatory region of 4-1BB (CD137), CD28, OX40, CD2, CD7, CD27, CD30, CD40, CD70, CD134, PD1, Dap10, CDS, ICAM-1, LFA-1 (CD11a/CD18), ICOS (CD278), NKG2D, GITR, TLR2, or combinations thereof.

The cytoplasmic signaling domain may comprise a cytoplasmic signaling domain of CD3ζ.

The present disclosure provides for a bispecific CAR comprising (or having) an amino acid sequence about 80% to about 100% identical to the amino acid sequence set forth in SEQ ID NO:26, SEQ ID NO:40, SEQ ID NO:54, SEQ ID NO:68, SEQ ID NO:84, SEQ ID NO:98, SEQ ID NO:112, or SEQ ID NO:126.

Also encompassed by the present disclosure is an immune cell expressing the bispecific CAR. The immune cell may be a T cell or a natural killer (NK) cell.

The present disclosure provides for a nucleic acid encoding the bispecific CAR.

The present disclosure provides for a vector comprising the present nucleic acid encoding the bispecific CAR.

The present disclosure provides for a pharmaceutical composition comprising the bispecific CAR, the immune cell, the nucleic acid, or the vector.

The present disclosure also provides for a method of treating an autoimmune disorder. The method may comprise administering the immune cell, or the pharmaceutical composition, to a subject in need thereof.

The autoimmune disorder may be systemic lupus erythematosus (SLE) (e.g., lupus nephritis), systemic vasculitis, systemic sclerosis, inflammatory myopathy (e.g., polymyositis, dermatomyositis, or inclusion-body myositis), systemic scleroderma, multiple sclerosis, myasthenia gravis, a myositis autoantibody-driven disease, or neuromyelitis optica.

The autoimmune disorder may be polymyositis, dermatomyositis, or inclusion-body myositis. The autoimmune disorder may be lupus nephritis.

The present disclosure also provides for a method of treating a cancer. The method may comprise administering the immune cell, or the pharmaceutical composition, to a subject in need thereof.

The cancer may be a hematologic cancer. The cancer may be a B-cell malignancy. The cancer may be Hodgkin's lymphoma, non-Hodgkin's lymphoma, leukemia, and/or multiple myeloma. The cancer may be acute myeloid leukemia (AML), multiple myeloma (MM), chronic lymphocytic leukemia (CLL), chronic myelogenous leukemia, acute lymphoblastic leukemia (ALL), diffuse large B cell lymphoma (DLBCL), or combinations thereof.

The immune cell may be allogeneic or autologous.

### BRIEF DESCRIPTION OF THE DRAWINGS

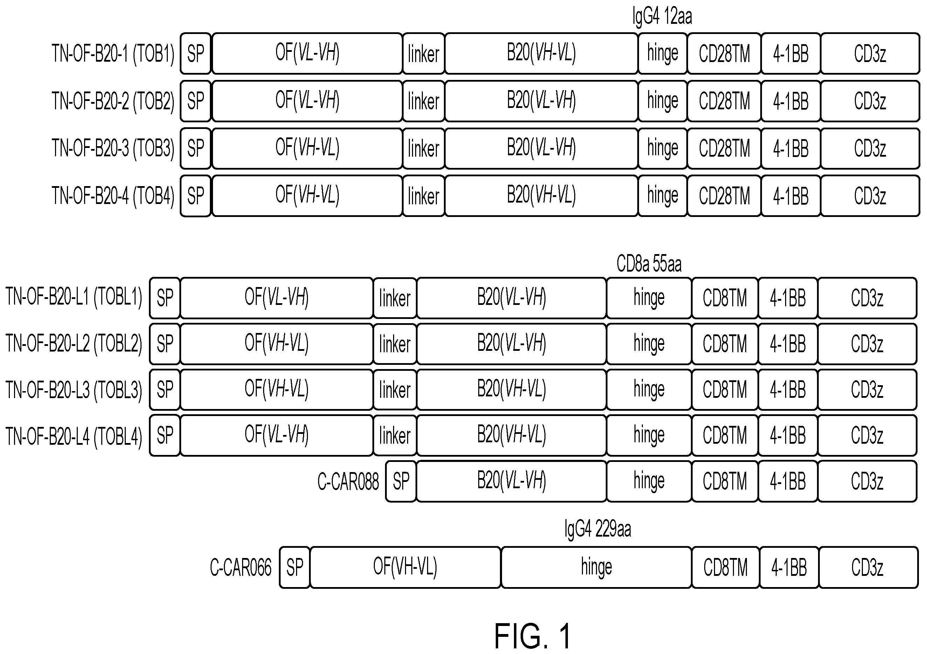

**Figure 1** shows the structures of the combined chimeric antigen receptors targeting CD20 and BCMA. The structures of the CARs include a signal peptide (SP), an anti-CD20 scFv (OF), a linker (linker-2), an anti-BCMA scFv (B20), a hinge region, a transmembrane domain, a co-stimulatory region, and a cytoplasmic signaling domain (CD3ζ). A short IgG4 hinge (12 aa) and a CD28 transmembrane domain are included in TOB1 to TOB4; a CD8a hinge and a CD8a transmembrane domain are included in TOBL1 to TOBL4. Four combinations of orientations of V_{H} and V_{L} in the two scFv sequences are included in the two groups of CARs (TOB1-4 and TOBL1-4). TOBL1 is also named C-CAR168.

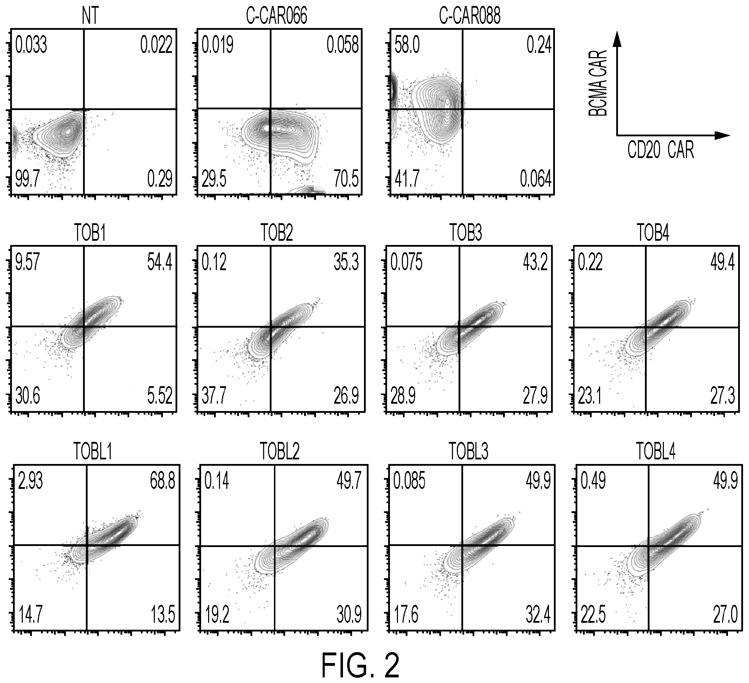

**Figure 2** shows the expression level of anti-CD20 and anti-BCMA CARs on the surface of the T cells.

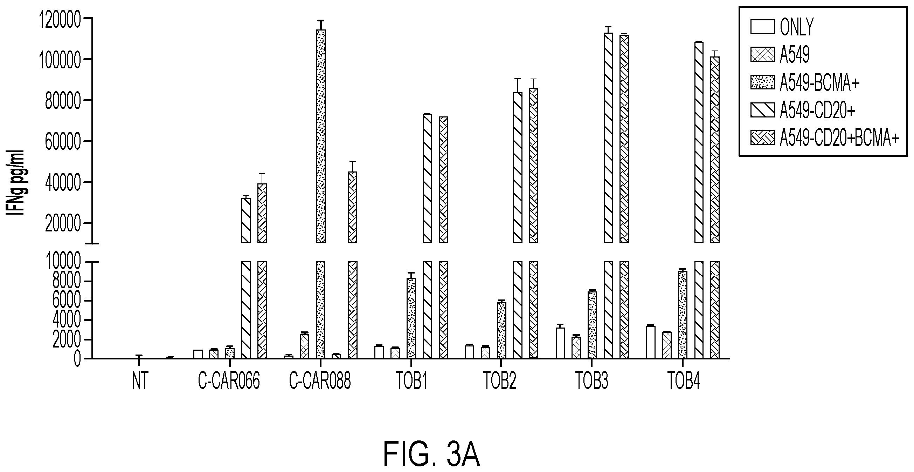

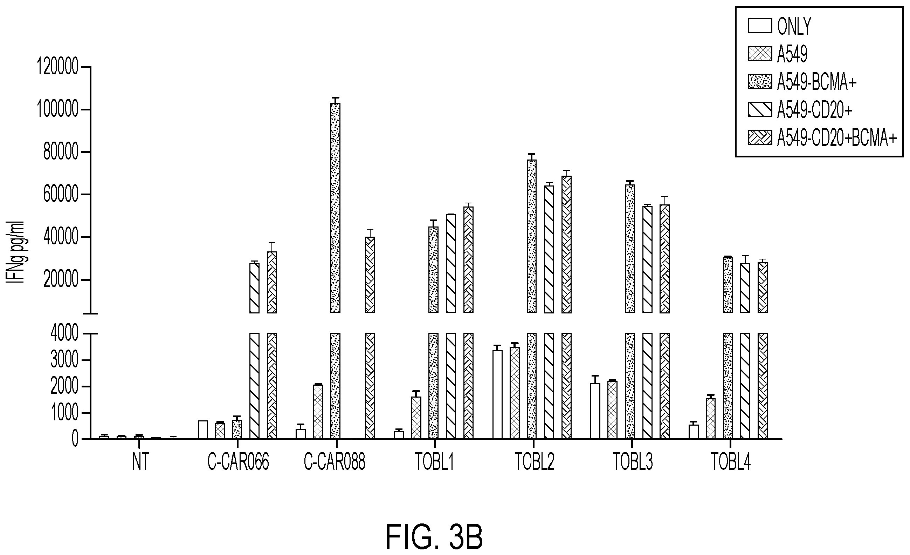

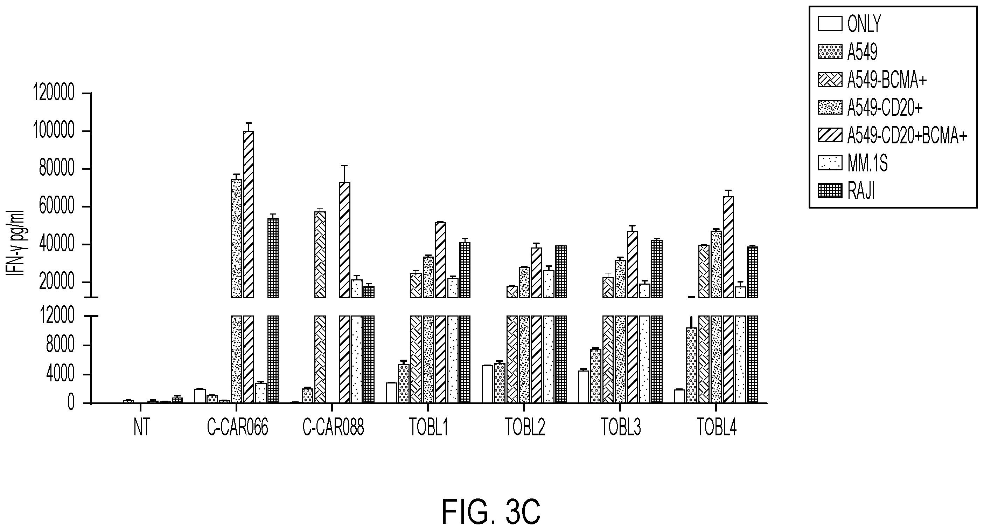

**Figures 3A-3C** show the levels of IFN-γ secreted by the activated CAR-T cells *in vitro* in the cell culture supernatant. Figure 3A shows the levels of IFN-γ secreted by the TOB1 to TOB4 CAR-T cells in the cell culture supernatant. Figure 3B shows the levels of IFN-γ secreted by the TOBL1 to TOBL4 CAR-T cells in the cell culture supernatant. Figure 3C: TOBL1 to TOBL4 showed high level IFN-γ release when co-cultured with target cells naturally expressing CD20 and BCMA. MM.1S is a BCMA-positive multiple myeloma (MM) cell line; RAJI is CD20 positive and BCMA positive.

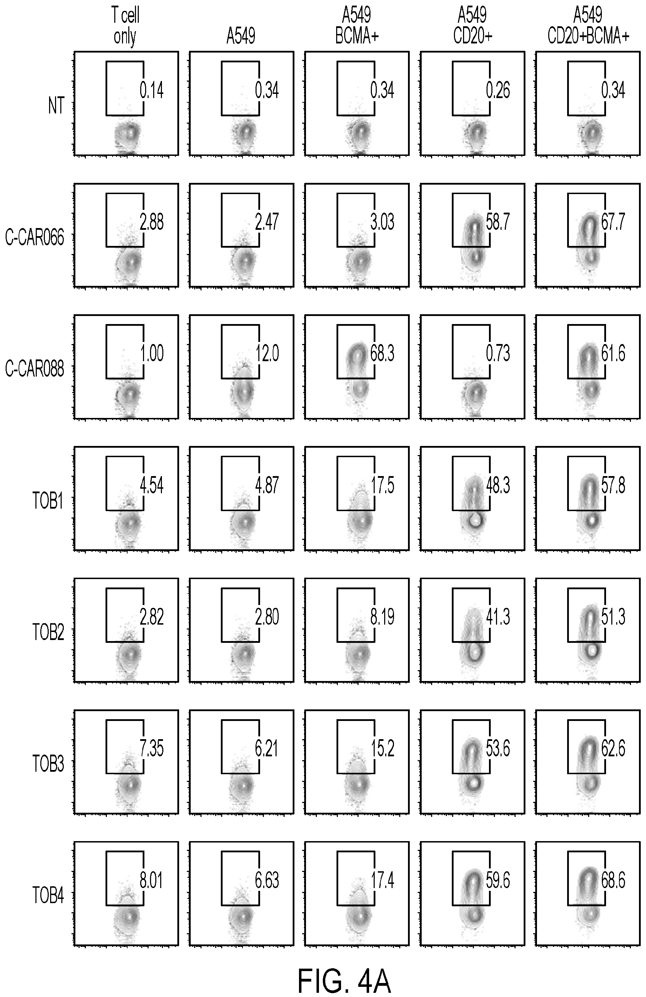

**Figures 4A-4B** show the expression levels of CD137 on the surface of the activated CAR-T cells.

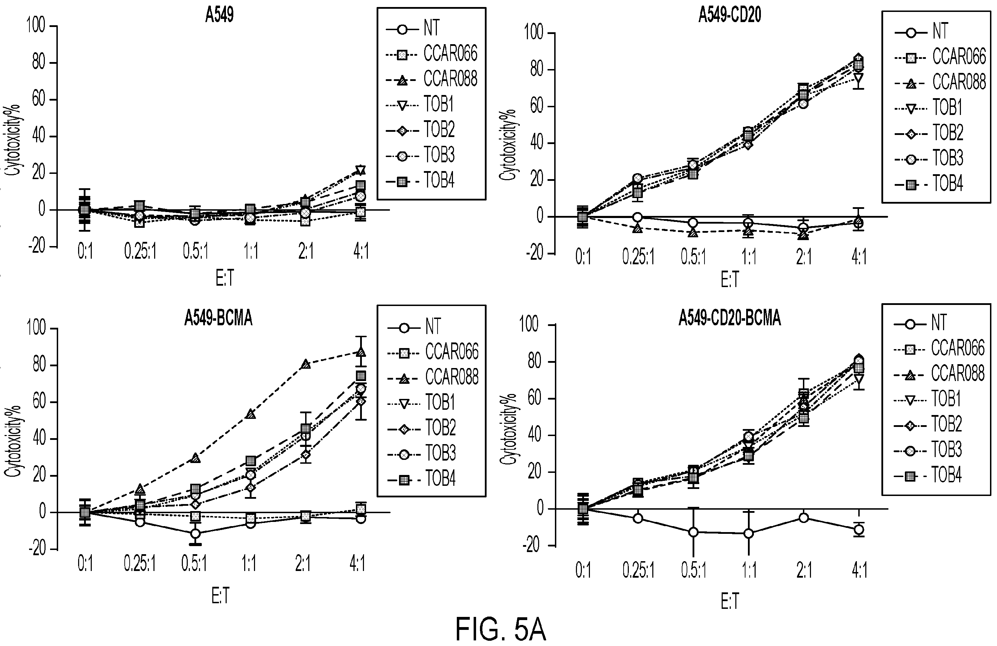

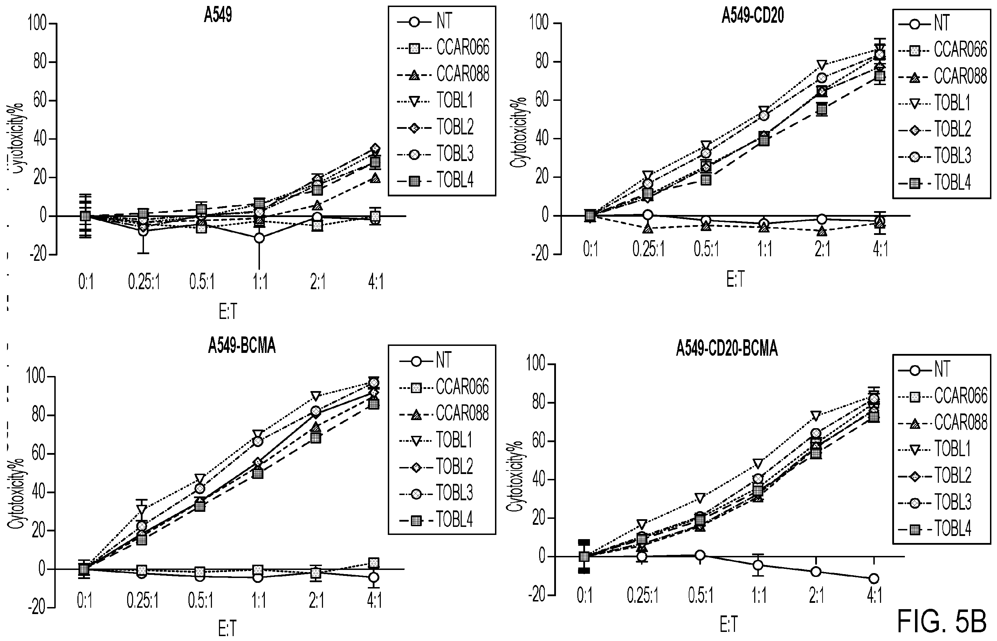

**Figures 5A-5B** show the *in vitro* cytotoxicity of CAR-Ts cells (Figure 5A: TOB1 to TOB4; Figure 5B: TOBL1 to TOBL4) by RTCA assays.

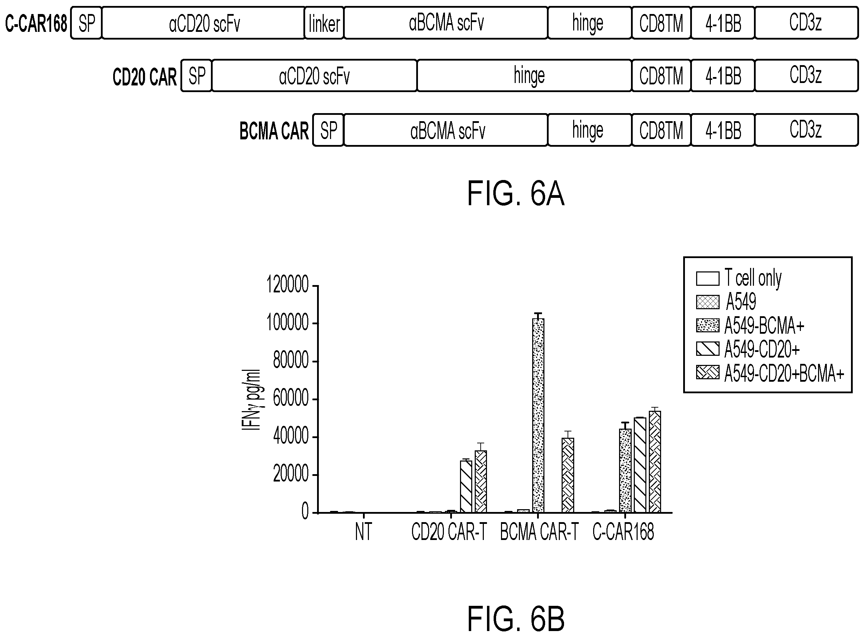

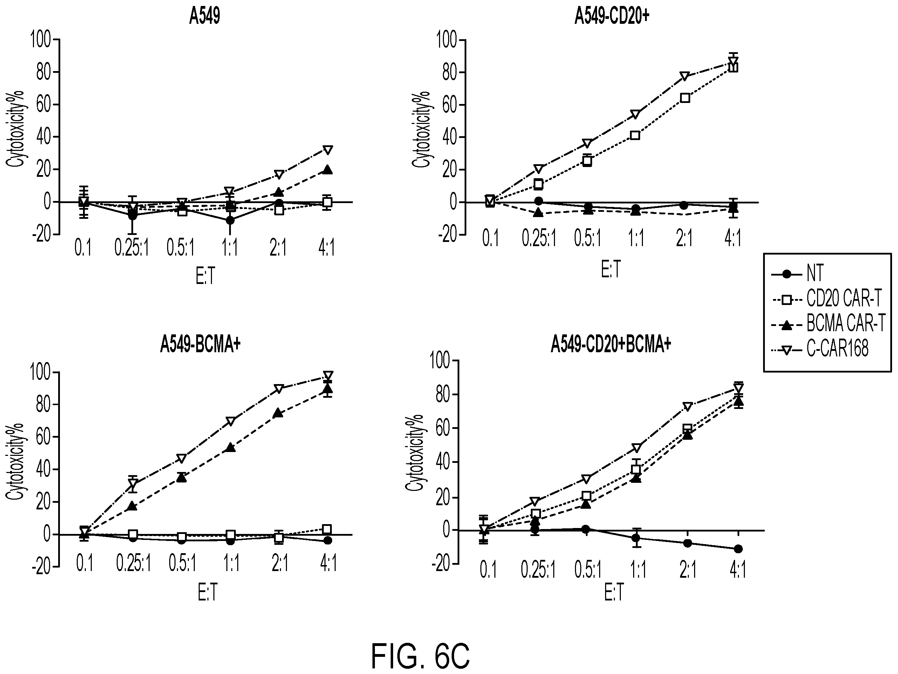

**Figures 6A-6C**: C-CAR168 shows robust potency against CD20+ and BCMA+ cells *in vitro*. Figure 6A shows the structures of C-CAR168 (TOBL1 which is an anti-CD20/BCMA CAR), anti-CD20 CAR (C-CAR066), and anti-BCMA CAR (C-CAR088). Figure 6B shows the release of IFN-γ after the CAR-T cells were co-cultured with CD20-positive and/or BCMA-positive target cells. Figure 6C shows the cytotoxicity of the CAR-T cells targeting CD20 and/or BCMA, at different E:T ratios.

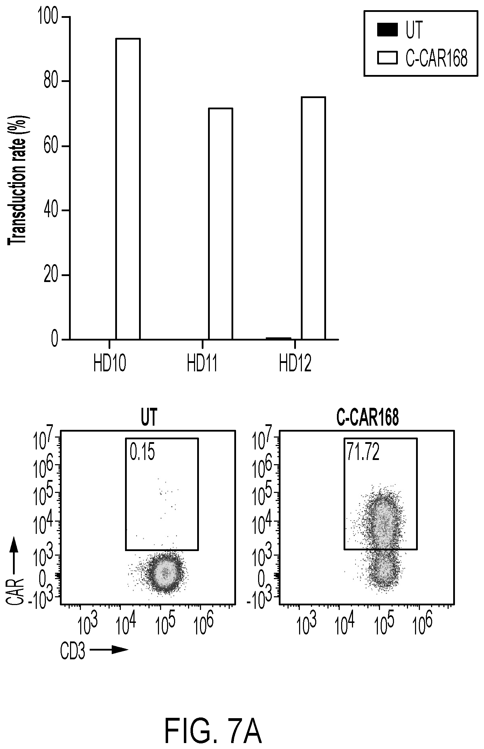

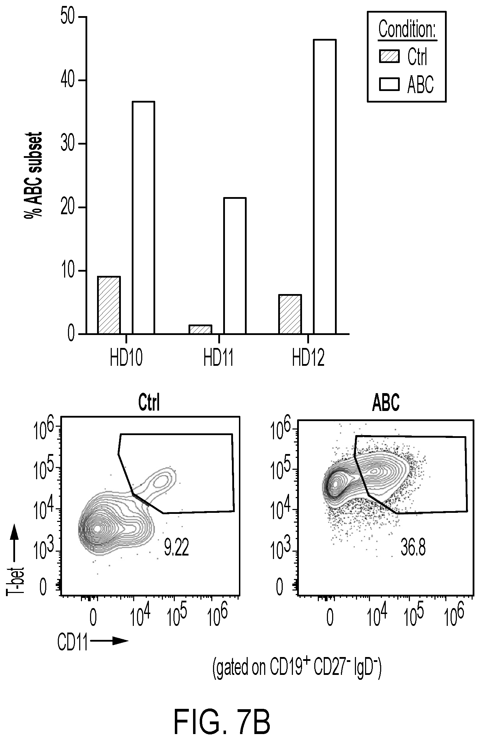

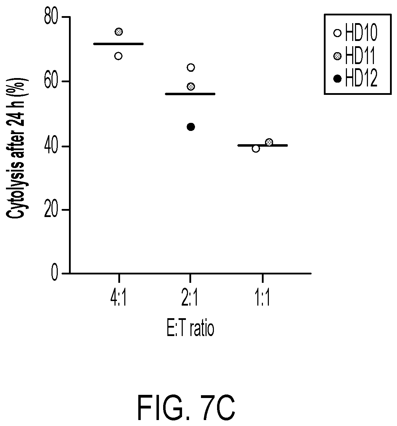

**Figures 7A-7C**: Cytotoxicity of C-CAR168 on ABC-enriched B cells *in vitro*. Figure 7A: Generation of C-CAR168 CAR-T cells. The lower panels show the CAR positive rate of C-CAR168 CAR-T cells prepared from the peripheral blood of 3 healthy donors. Figure 7B: Differentiation of ABCs. The lower panels show that the proportion of ABC subpopulations increased significantly after induction of differentiation of autologous B cells. Figure 7C: Cytolysis of ABC-enriched B cells by C-CAR168 at different E:T ratios.

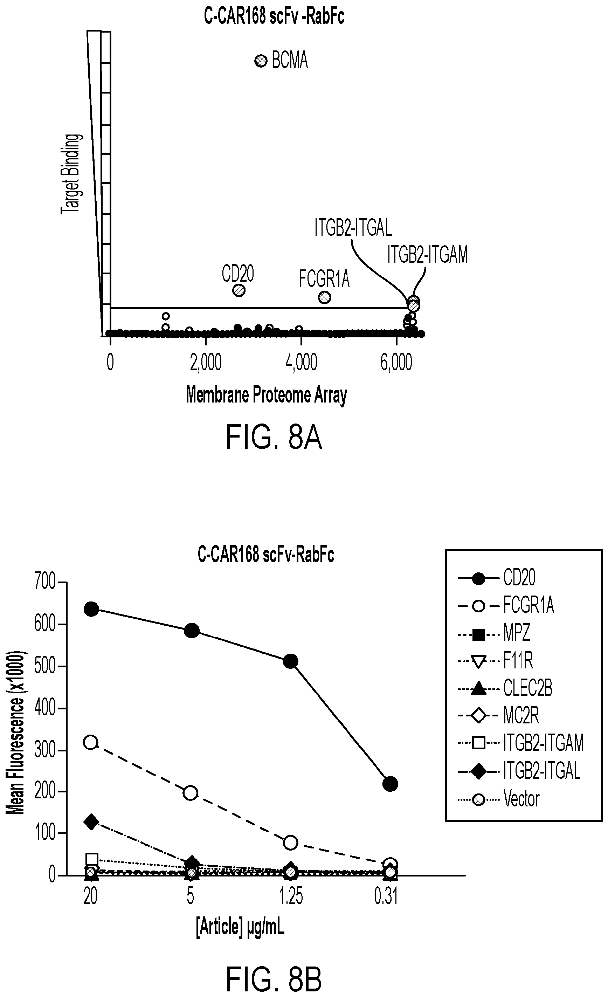

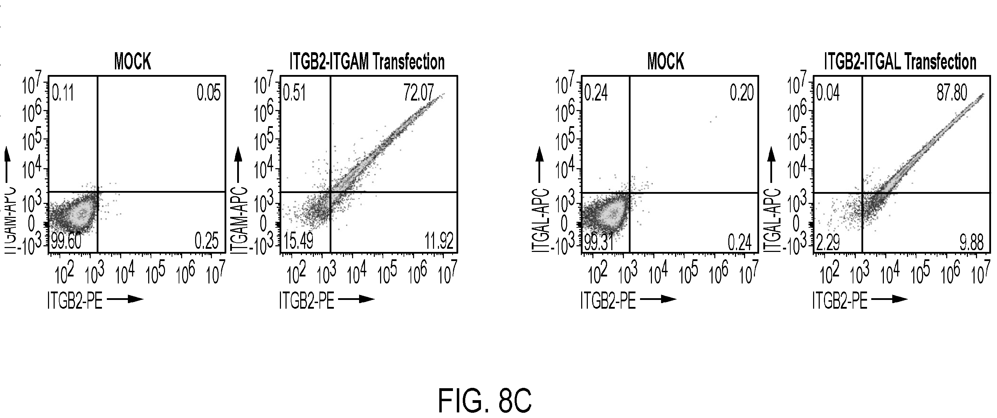

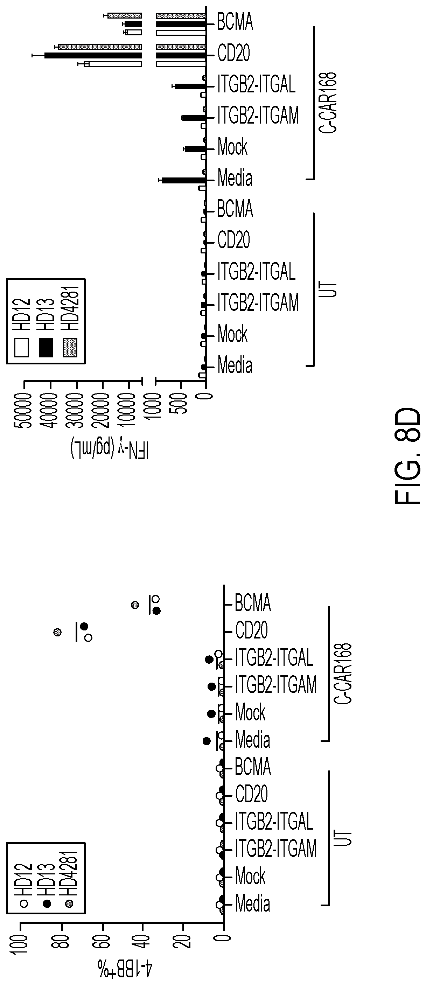

**Figures 8A-8D**: C-CAR168 bears no cross-reactivity against human membrane proteome. Figures 8A-8B: C-CAR168 scFv-RabFc binding specificity in the membrane protein array. Figure 8C: Flow cytometry detection of expression of ITGB2-ITGAM and ITGB2-ITGAL in 293T cells. Figure 8D (left panel): Flow cytometric detection of the proportion of 4-1BB-positive cells. Figure 8D (right panel): Flow cytometry detection of IFN-γ concentrations in the co-culture supernatants.

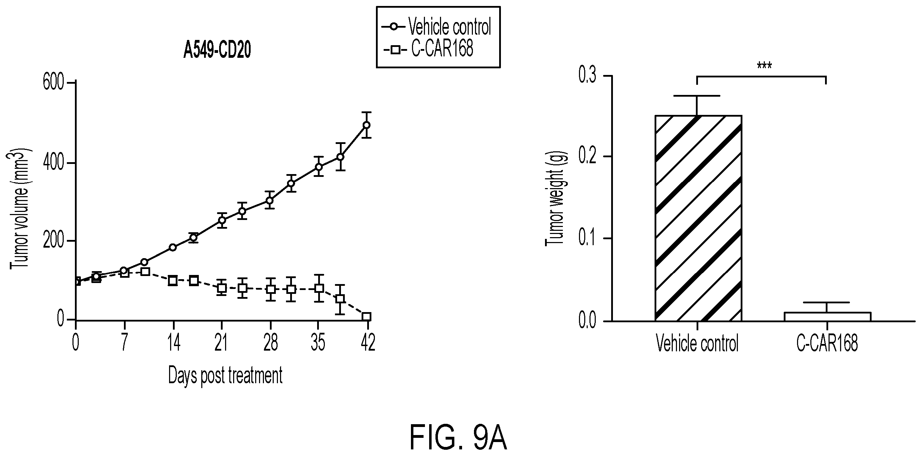

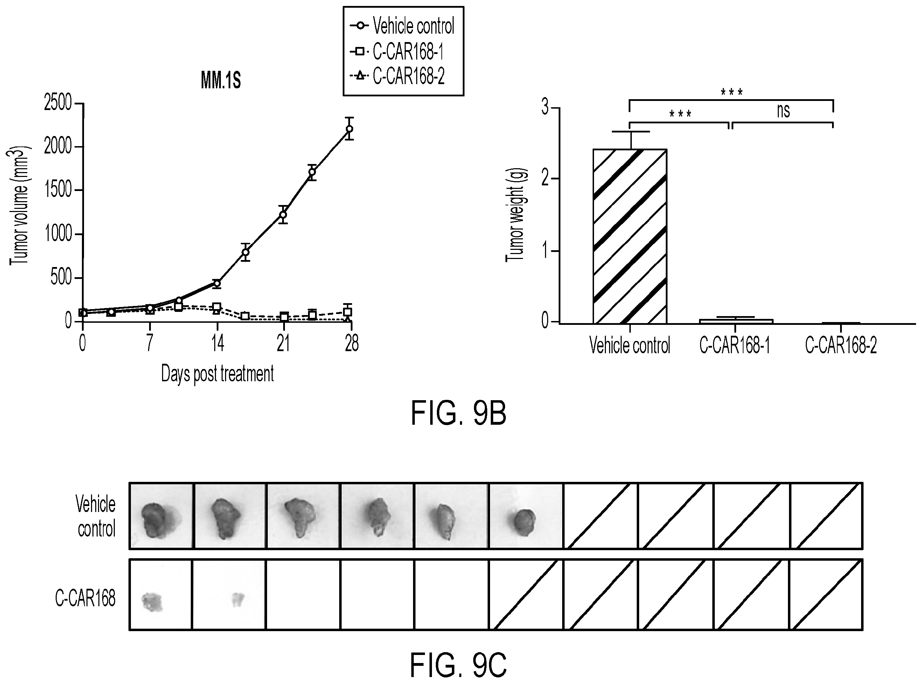

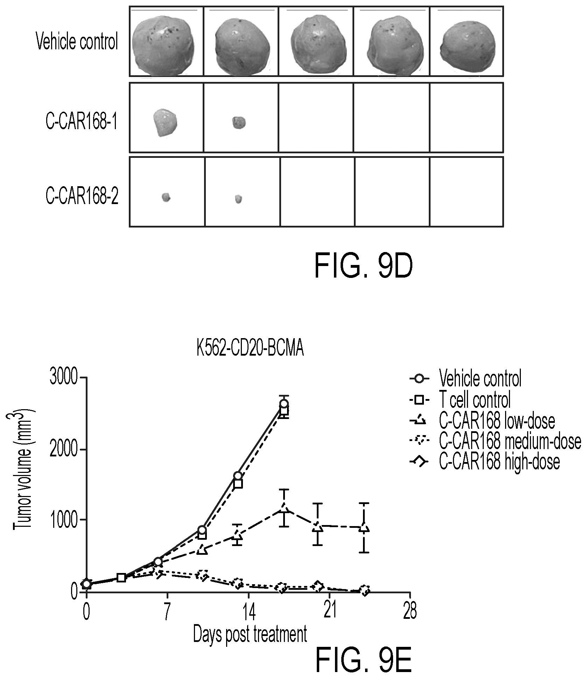

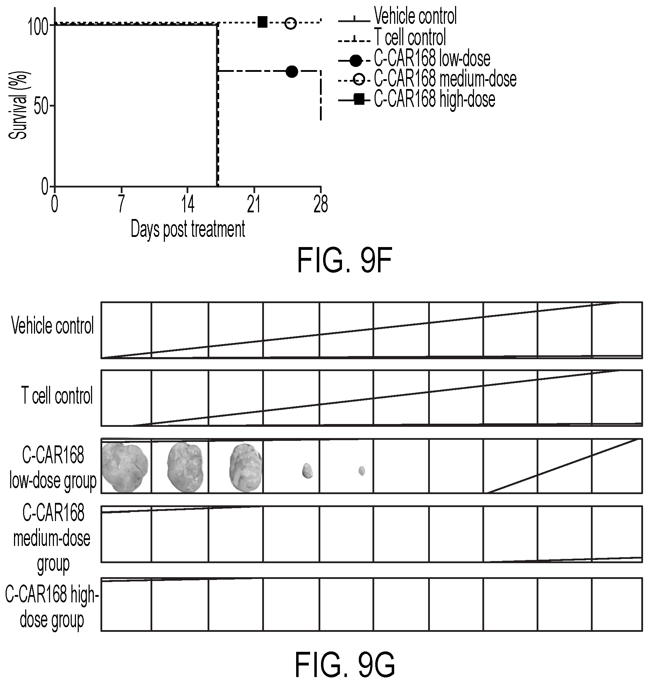

**Figures 9A-9G**: *In vivo* cytotoxicity of C-CAR168 in tumor-bearing mice. Figure 9A: C-CAR168 significantly inhibited the growth of A549-CD20 cells in B-NDG tumor bearing mice. Left panel: tumor growth curve of each group during the experiment; right panel: average tumor weight of animals in each group at Day 42. ***: P<0.001, compared to the vehicle control group. Figure 9B: C-CAR168 significantly inhibited the growth of human multiple myeloma MM.1S tumor cells in B-NDG tumor bearing mice. Left panel: tumor growth curve of each group during the experiment; right panel: average tumor weight of animals in each group at Day 28. ***: P<0.001, compared to the vehicle control group. Figure 9C: Images of the A549-CD20 tumors of the animals in each group at Day 42. "/" indicates that the animal was dead. The blank box indicates that no tumor tissue was collected. Figure 9D: Images of MM.1S tumors of the animals in each group at Day 28. The blank box indicates that no tumor tissue was collected. Figures 9E-9G: C-CAR168 significantly inhibited the growth of K562-CD20-BCMA tumor cells in B-NDG tumor bearing mice. Figure 9E: Tumor growth curve of each group during the experiment. Figure 9F: The survival rate curve of each group during the experimental period. Figure 9G: Images of tumors of animals in each group at Day 28. "/" indicates that the animal was dead. The blank box indicates that no tumor tissue was collected.

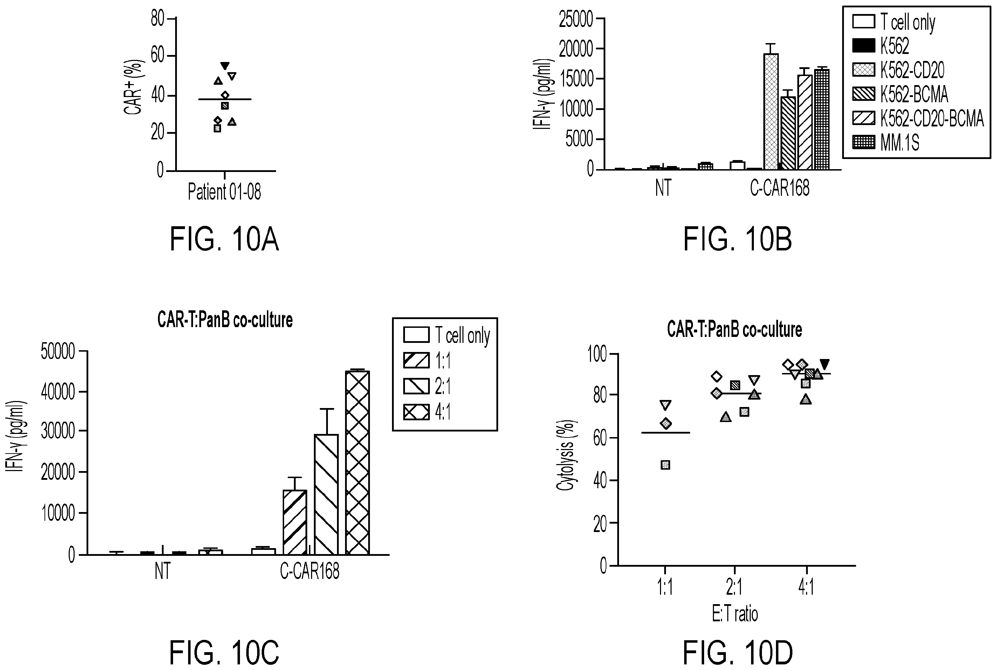

**Figures 10A-10D**: C-CAR168 shows robust potency *in vitro* against autologous B cells from SLE patients. Figure 10A: T cells from eight SLE patients were successfully transduced by lentiviral vectors encoding C-CAR168 and expressed the anti-CD20/BCMA CAR. Figure 10B: C-CAR168 CAR-T cells generated from the SLE patient samples showed robust activity (IFN-γ release) against target cells expressing CD20 and BCMA. K562 is negative for both CD20 and BCMA; MM.1S is a multiple myeloma cell line which is BCMA-positive. Figure 10C: C-CAR168 CAR-T cells generated from the SLE patient samples showed robust activity (e.g., IFN-γ release) against pan B cells isolated from the SLE patients. Figure 10D: Pan B cells isolated from the SLE patients were recognized and lysed by autologous C-CAR168 cells.

### DETAILED DESCRIPTION

The present disclosure provides a chimeric antigen receptor (CAR) that targets both CD20 and BCMA. The CAR may comprise a signal peptide, an anti-CD20 scFv, an anti-BCMA scFv, a hinge region, a transmembrane domain, a co-stimulatory region, and a cytoplasmic signaling domain. The present CARs can be used to treat autoimmune diseases or cancer.

B-cell maturation antigen (BCMA), also known as TNFRSF17 or CD269, is a member of the tumor necrosis factor receptor family. It serves as an important receptor for B-cell activating factor (BAFF), along with TACI and BAFF-R, and participates in the regulation of B lymphocyte differentiation and maturation. BCMA is a type III transmembrane protein, specifically expressed in B cells, especially in plasmablasts and differentiated mature plasma cells.

CD20, which is a B-cell membrane marker, also known as B1, is a transmembrane glycoprotein encoded by the MS4A gene. CD20 plays an important role in the development, proliferation, activation, differentiation, and malignant transformation of B cells through the regulation of transmembrane Ca²⁺ conductance.

The present disclosure provides for a bispecific chimeric antigen receptor (CAR). The bispecific CAR may comprise: (i) an anti-CD20 antigen-binding region which comprises a light chain variable region (V_{L}1) and a heavy chain variable region (V_{H}1); and (ii) an anti-BCMA antigen-binding region which comprises a light chain variable region (V_{L}2) and a heavy chain variable region (V_{H}2).

The present bispecific chimeric antigen receptor (CAR) may comprise: (i) an anti-CD20 antigen-binding region which comprises a light chain variable region (V_{L}1) and a heavy chain variable region (V_{H}1) having amino acid sequences about 80% to about 100%, about 85% to about 100%, about 90% to about 100%, or about 95% to about 100%, identical to the amino acid sequences set forth in SEQ ID NO: 4 and SEQ ID NO: 8, respectively; and (ii) an anti-BCMA antigen-binding region which comprises a light chain variable region (V_{L}2) and a heavy chain variable region (V_{H}2) having amino acid sequences about 80% to about 100%, about 85% to about 100%, about 90% to about 100%, or about 95% to about 100%, identical to the amino acid sequences set forth in SEQ ID NO: 12 and SEQ ID NO: 16, respectively.

The present disclosure provides for a bispecific chimeric antigen receptor (CAR). The bispecific CAR may comprise: (i) an anti-CD20 antigen-binding region which comprises a light chain variable region (V_{L}1) and a heavy chain variable region (V_{H}1), and (ii) an anti-BCMA antigen-binding region which comprises a light chain variable region (V_{L}2) and a heavy chain variable region (V_{H}2). V_{L}1 may comprise three complementarity determining regions (CDRs), CDR1, CDR2 and CDR3, having amino acid sequences about 80% to about 100%, about 85% to about 100%, about 90% to about 100%, or about 95% to about 100%, identical to the amino acid sequences set forth in SEQ ID NO: 130, SEQ ID NO: 131, SEQ ID NO: 132, respectively. V_{H}1 may comprise three CDRs, CDR1, CDR2 and CDR3, having amino acid sequences about 80% to about 100%, about 85% to about 100%, about 90% to about 100%, or about 95% to about 100%, identical to the amino acid sequences set forth in SEQ ID NO: 127, SEQ ID NO: 128, SEQ ID NO: 129, respectively. V_{L}2 may comprise three complementarity determining regions (CDRs), CDR1, CDR2 and CDR3, having amino acid sequences about 80% to about 100%, about 85% to about 100%, about 90% to about 100%, or about 95% to about 100%, identical to the amino acid sequences set forth in SEQ ID NO: 134, SEQ ID NO: 136, SEQ ID NO: 138, respectively. V_{H}2 may comprise three CDRs, CDR1, CDR2 and CDR3, having amino acid sequences about 80% to about 100%, about 85% to about 100%, about 90% to about 100%, or about 95% to about 100%, identical to the amino acid sequences set forth in SEQ ID NO: 141, SEQ ID NO: 143, SEQ ID NO: 145, respectively.

In certain embodiments, V_{L}1 is located at the N-terminus of V_{H}1. In certain embodiments, V_{H}1 is located at the N-terminus of V_{L}1. In certain embodiments, V_{H}2 is located at the N-terminus of V_{L}2. In certain embodiments, V_{L}2 is located at the N-terminus of V_{H}2. In one embodiment, V_{L}1 is located at the N-terminus of V_{H}1; V_{L}2 is located at the N-terminus of V_{H}2.

In certain embodiments, V_{L}1 and V_{H}1 have amino acid sequences about 80% to about 100% identical to amino acid sequences set forth in SEQ ID NO: 4 and SEQ ID NO: 8, respectively.

In certain embodiments, V_{L}2 and V_{H}2 have amino acid sequences about 80% to about 100% identical to amino acid sequences set forth in SEQ ID NO: 12 and SEQ ID NO: 16, respectively.

In certain embodiments, the antigen-binding region that specifically binds CD20 is located at the N-terminus of the antigen-binding region that specifically binds BCMA. In certain embodiments, the antigen-binding region that specifically binds BCMA is located at the N-terminus of the antigen-binding region that specifically binds CD20.

The anti-CD20 antigen-binding region may be a single-chain variable fragment (scFv) that specifically binds CD20. The anti-BCMA antigen-binding region may be a scFv that specifically binds BCMA. In certain embodiments, the scFv that specifically binds CD20 is located at the N-terminus of the scFv that specifically binds BCMA. In certain embodiments, the scFv that specifically binds BCMA is located at the N-terminus of the scFv that specifically binds CD20.

The bispecific CAR may further comprise one or more of the following: (a) a signal peptide or SP (or a leader sequence), (b) a hinge region, (c) a transmembrane domain, (d) a co-stimulatory region, and (e) a cytoplasmic signaling domain.

The present bispecific CARs may comprise, from N-terminus to C-terminus, a signal peptide, an anti-CD20 scFv, an anti-BCMA scFv, a hinge region, a transmembrane domain, and a co-stimulatory region, and a cytoplasmic signaling domain.

The signal peptide may comprise a signal peptide of (or may be derived from) CD8, CD28, GM-CSF, CD4, CD137, or combinations thereof. In one embodiment, the signal peptide is a signal peptide of (or is derived from) CD8.

In one embodiment, the signal peptide comprises an amino acid sequence about 80% to about 100%, about 85% to about 100%, about 90% to about 100%, or about 95% to about 100%, identical to the amino acid sequence set forth in SEQ ID NO: 2.

The hinge region may comprise a hinge region of (or may be derived from) IgG4, CD8, CD28, CD137, or combinations thereof, wildtype or mutants.

In one embodiment, the hinge region is a hinge region of (or is derived from) IgG4. In one embodiment, the hinge region comprises an amino acid sequence about 80% to about 100%, about 85% to about 100%, about 90% to about 100%, or about 95% to about 100%, identical to the amino acid sequence set forth in SEQ ID NO: 78.

In one embodiment, the hinge region is is a hinge region of (or is derived from) CD8a. In one embodiment, the hinge region comprises an amino acid sequence about 80% to about 100%, about 85% to about 100%, about 90% to about 100%, or about 95% to about 100%, identical to the amino acid sequence set forth in SEQ ID NO: 18.

The transmembrane domain may comprise a transmembrane domain of (or may be derived from) CD8, CD28, CD3ε, CD45, CD4, CD5, CD9, CD16, CD22, CD33, CD37, CD64, CD80, CD86, CD134, CD137, CD154, or combinations thereof.

In one embodiment, the transmembrane domain is a transmembrane domain of (or is derived from) CD8. In one embodiment, the transmembrane domain comprises an amino acid sequence about 80% to about 100%, about 85% to about 100%, about 90% to about 100%, or about 95% to about 100%, identical to the amino acid sequence set forth in SEQ ID NO: 20.

In one embodiment, the transmembrane domain is a transmembrane domain of (or is derived from) CD28. In one embodiment, the transmembrane domain comprises an amino acid sequence about 80% to about 100%, about 85% to about 100%, about 90% to about 100%, or about 95% to about 100%, identical to the amino acid sequence set forth in SEQ ID NO: 80.

The co-stimulatory region may comprise a co-stimulatory region of (or may be derived from) 4-1BB (CD137), CD28, OX40, CD2, CD7, CD27, CD30, CD40, CD70, CD134, PD1, Dap10, CDS, ICAM-1, LFA-1 (CD11a/CD18), ICOS (CD278), NKG2D, GITR, TLR2, or combinations thereof.

In one embodiment, the co-stimulatory region is a co-stimulatory region of (or is derived from) 4-1BB. In one embodiment, the co-stimulatory region comprises an amino acid sequence about 80% to about 100%, about 85% to about 100%, about 90% to about 100%, or about 95% to about 100%, identical to the amino acid sequence set forth in SEQ ID NO: 22.

The cytoplasmic signaling domain may comprise a cytoplasmic signaling domain of (or may be derived from) CD3ζ. In one embodiment, the cytoplasmic signaling domain comprises an amino acid sequence about 80% to about 100%, about 85% to about 100%, about 90% to about 100%, or about 95% to about 100%, identical to the amino acid sequence set forth in SEQ ID NO: 24.

The present CAR may comprise a linker (linker-1) between V_{L} and V_{H} of the anti-CD20 antigen-binding region. In one embodiment, the linker (linker-1) comprises an amino acid sequence about 80% to about 100%, about 85% to about 100%, about 90% to about 100%, or about 95% to about 100%, identical to the amino acid sequence set forth in SEQ ID NO:6.

The present CAR may comprise a linker (linker-2) between the anti-CD20 antigen-binding region and the anti-BCMA antigen-binding region. In one embodiment, the linker (linker-2) comprises an amino acid sequence about 80% to about 100%, about 85% to about 100%, about 90% to about 100%, or about 95% to about 100%, identical to the amino acid sequence set forth in SEQ ID NO:10.

The present CAR may comprise a linker (linker-3) between V_{L} and V_{H} of the anti-BCMA antigen-binding region. In one embodiment, the linker (linker-3) comprises an amino acid sequence about 80% to about 100%, about 85% to about 100%, about 90% to about 100%, or about 95% to about 100%, identical to the amino acid sequence set forth in SEQ ID NO:14.

In one embodiment, the bispecific CAR comprises, from N-terminus to C-terminus, (a) an anti-CD20 antigen-binding region with a light chain variable region (V_{L}1) and a heavy chain variable region (V_{H}1) of those of ofatumumab, (ii) an anti-BCMA antigen-binding region with a light chain variable region (V_{L}2) and a heavy chain variable region (V_{H}2) of those of the BCMA-20 antibody, (iii) a hinge region having an amino acid sequence set forth in SEQ ID NO:18, (iv) a transmembrane domain having an amino acid sequence set forth in SEQ ID NO:20, (v) a co-stimulatory region having an amino acid sequence set forth in SEQ ID NO:22, and (vi) a cytoplasmic signaling domain having an amino acid sequence set forth in SEQ ID NO:24.

In one embodiment, the bispecific CAR comprises, from N-terminus to C-terminus, (a) an anti-CD20 antigen-binding region with a light chain variable region (V_{L}1) and a heavy chain variable region (V_{H}1) having amino acid sequences set forth in SEQ ID NO:4 and SEQ ID NO:8, respectively, (ii) an anti-BCMA antigen-binding region with a light chain variable region (V_{L}2) and a heavy chain variable region (V_{H}2) having amino acid sequences set forth in SEQ ID NO:12 and SEQ ID NO:16, respectively, (iii) a hinge region having an amino acid sequence set forth in SEQ ID NO:18, (iv) a transmembrane domain having an amino acid sequence set forth in SEQ ID NO:20, (v) a co-stimulatory region having an amino acid sequence set forth in SEQ ID NO:22, and (vi) a cytoplasmic signaling domain having an amino acid sequence set forth in SEQ ID NO:24.

In one embodiment, the bispecific CAR comprises, from N-terminus to C-terminus, (a) an anti-CD20 antigen-binding region with a light chain variable region (V_{L}1) and a heavy chain variable region (V_{H}1) of those of ofatumumab, (ii) an anti-BCMA antigen-binding region with a light chain variable region (V_{L}2) and a heavy chain variable region (V_{H}2) of those of BCMA-20, (iii) a hinge region having an amino acid sequence set forth in SEQ ID NO:78, (iv) a transmembrane domain having an amino acid sequence set forth in SEQ ID NO:80, (v) a co-stimulatory region having an amino acid sequence set forth in SEQ ID NO:22, and (vi) a cytoplasmic signaling domain having an amino acid sequence set forth in SEQ ID NO:24.

In one embodiment, the bispecific CAR comprises, from N-terminus to C-terminus, (a) an anti-CD20 antigen-binding region with a light chain variable region (V_{L}1) and a heavy chain variable region (V_{H}1) having amino acid sequences set forth in SEQ ID NO:4 and SEQ ID NO:8, respectively, (ii) an anti-BCMA antigen-binding region with a light chain variable region (V_{L}2) and a heavy chain variable region (V_{H}2) having amino acid sequences set forth in SEQ ID NO:12 and SEQ ID NO:16, respectively, (iii) a hinge region having an amino acid sequence set forth in SEQ ID NO:78, (iv) a transmembrane domain having an amino acid sequence set forth in SEQ ID NO:80, (v) a co-stimulatory region having an amino acid sequence set forth in SEQ ID NO:22, and (vi) a cytoplasmic signaling domain having an amino acid sequence set forth in SEQ ID NO:24.

In certain embodiments, V_{L}1 is located at the N-terminus of V_{H}1. In certain embodiments, V_{H}1 is located at the N-terminus of V_{L}1. In certain embodiments, V_{H}2 is located at the N-terminus of V_{L}2. In certain embodiments, V_{L}2 is located at the N-terminus of V_{H}2. In one embodiment, V_{L}1 is located at the N-terminus of V_{H}1; V_{L}2 is located at the N-terminus of V_{H}2.

In certain embodiments, the bispecific CAR comprises an amino acid sequence about 80% to about 100%, about 85% to about 100%, about 90% to about 100%, or about 95% to about 100%, identical to the amino acid sequence set forth in SEQ ID NO:26, SEQ ID NO:40, SEQ ID NO:54, SEQ ID NO:68, SEQ ID NO:84, SEQ ID NO:98, SEQ ID NO:112, or SEQ ID NO:126.

In certain embodiments, the bispecific CAR may have an amino acid sequence set forth in SEQ ID NO:26, SEQ ID NO:40, SEQ ID NO:54, SEQ ID NO:68, SEQ ID NO:84, SEQ ID NO:98, SEQ ID NO:112, or SEQ ID NO:126.

The present bispecific CAR may be encoded by a nucleic acid having a nucleotide sequence about 80% to about 100%, about 85% to about 100%, about 90% to about 100%, or about 95% to about 100%, identical to the nucleotide sequence set forth in SEQ ID NO:25, SEQ ID NO:39, SEQ ID NO:53, SEQ ID NO:67, SEQ ID NO:83, SEQ ID NO:97, SEQ ID NO:111, or SEQ ID NO:125.

The present bispecific CAR may be encoded by a nucleic acid having a nucleotide sequence set forth in SEQ ID NO:25, SEQ ID NO:39, SEQ ID NO:53, SEQ ID NO:67, SEQ ID NO:83, SEQ ID NO:97, SEQ ID NO:111, or SEQ ID NO:125.

The present disclosure provides for an immune cell expressing or comprising the present bispecific CAR. The immune cell may be a T cell or a natural killer (NK) cell.

The present disclosure provides an immune cell, comprising the vector or the nucleic acid encoding the present CAR (e.g., integrated into its genome). The cell may be an isolated cell. The cell may be a genetically engineered cell. The cell may be a mammalian cell. In one embodiment, the cell is a CAR-T cell and/or a CAR-NK cell.

Also encompassed by the present disclosure is a nucleic acid encoding the present chimeric antigen receptor (e.g., the present bispecific CAR).

The present nucleic acid may comprise a nucleotide sequence about 80% to about 100%, about 85% to about 100%, about 90% to about 100%, or about 95% to about 100%, identical to the nucleotide sequence set forth in SEQ ID NO:25, SEQ ID NO:39, SEQ ID NO:53, SEQ ID NO:67, SEQ ID NO:83, SEQ ID NO:97, SEQ ID NO:111, or SEQ ID NO:125.

The present nucleic acid may comprise a nucleotide sequence set forth in SEQ ID NO:25, SEQ ID NO:39, SEQ ID NO:53, SEQ ID NO:67, SEQ ID NO:83, SEQ ID NO:97, SEQ ID NO:111, or SEQ ID NO:125.

The present disclosure provides for a vector comprising the present nucleic acid. The vector may comprise DNA or RNA. The vector may be a plasmid, virus vector, transposon, or combinations thereof. The vector may comprise a DNA virus or a retroviral vector. The vector may be a lentiviral vector, an adenoviral vector, an adeno-associated viral vector, or combinations thereof. In one embodiment, the vector is a lentiviral vector.

The present disclosure also provides for a pharmaceutical composition, comprising the present chimeric antigen receptor (e.g., the present bispecific CAR), the present immune cell, the present nucleic acid, or the present vector. The pharmaceutical composition may further comprise a pharmaceutically acceptable carrier, diluent or excipient. The pharmaceutical composition may be a liquid preparation.

The pharmaceutical composition may comprise the present immune cells at a concentration ranging from about 1×10³ cells/mL to about 1×10⁸ cells/mL, or from about 1×10⁴ cells/mL to about 1×10⁷ cells/mL.

The present disclosure also provides for a method of treating an autoimmune disease/disorder. The present disclosure provides for a method of treating cancer. The method may comprise administering the present immune cell or present pharmaceutical composition to a subject in need thereof.

The immune cell may be allogeneic or autologous.

The autoimmune disorder may be systemic lupus erythematosus (SLE) (e.g., lupus nephritis), systemic sclerosis (SSc), inflammatory myopathy (e.g., polymyositis, dermatomyositis, or inclusion-body myositis), systemic scleroderma, multiple sclerosis, or neuromyelitis optica (NMO).

The cancer may be a hematologic cancer. The cancer may be a B-cell malignancy. The cancer may be Hodgkin's lymphoma, non-Hodgkin's lymphoma, leukemia, and/or multiple myeloma. The cancer may be acute myeloid leukemia (AML), multiple myeloma (MM), chronic lymphocytic leukemia (CLL), chronic myelogenous leukemia, acute lymphoblastic leukemia (ALL), diffuse large B cell lymphoma (DLBCL), or combinations thereof.

The present disclosure provides a method for preparing an immune cell (e.g., a CAR-T cell) expressing the chimeric antigen receptor, where the method comprises: transducing the present nucleic acid molecule or the present vector into an immune cell (e.g., a T cell or NK cell), thereby obtaining the immune cell expressing the chimeric antigen receptor (e.g., the CAR-T cell).

The present disclosure provides a chimeric antigen receptor (CAR), wherein the structure of the chimeric antigen receptor may be shown in formula I:

SP-scFv1-linker 2-scFv2-H-TM-C-CSD (I),

where, each "-" is independently a linker peptide or a peptide bond; SP is an optional signal peptide; H is an optional hinge region; TM is a transmembrane domain; C is a co-stimulatory region; CSD is a cytoplasmic signaling domain; one of scFv1 and scFv2 is an anti-CD20 antigen binding region, and the other is an anti-BCMA antigen binding region.

In one embodiment, scFv1 is an anti-CD20 antigen binding region, and scFv2 is an anti-BCMA antigen binding region. In another embodiment, scFv1 is an anti-BCMA antigen binding region, and scFv2 is an anti-CD20 antigen binding region.

The structure of the anti-CD20 antigen binding region may be as shown in formula A or B as below:

V_{H1}-V_{L1} (A); V_{L1}-V_{H1} (B)

wherein V_{H1} is an anti-CD20 antibody heavy chain variable region; V_{L1} is an anti-CD20 antibody light chain variable region; and "-" is a linker peptide or a peptide bond.

In one embodiment, the present CAR has an anti-CD20 antigen binding region (or domain) with a structure as shown in formula B.

In certain embodiments, the amino acid sequence of V_{L1} is shown in SEQ ID NO: 4, and the amino acid sequence of V_{H1} is shown in SEQ ID NO: 8.

V_{L1} and V_{H1} may be linked with a linker peptide (linker 1 or linker-1). Linker-1 may have the sequence set forth in SEQ ID NO: 6.

The structure of the anti-BCMA antigen binding region may be as shown in formula C or D as below:

V_{L2}-V_{H2} (C); V_{H2}-V_{L2} (D)

where V_{L2} is an anti-BCMA antibody light chain variable region; V_{H2} is an anti-BCMA antibody heavy chain variable region; and "-" is a linker peptide or a peptide bond.

In one embodiment, the present CAR has an anti-BCMA antigen binding domain with a structure as shown in formula C.

In certain embodiments, the amino acid sequence of the V_{L2} is shown in SEQ ID NO: 12, and the amino acid sequence of the V_{H2} is shown in SEQ ID NO: 16.

V_{L2} and V_{H2} may be linked with a linker peptide (linker 3 or linker-3). Linker-3 may have the sequence set forth in SEQ ID NO: 14.

In another embodiment, the structure of the chimeric antigen receptor is shown in formula II as below:

SP-V_{L1}-V_{H1}- linker 2 -V_{L2}-V_{H2}-H-TM-C-CSD (II)

In one embodiment, linker 2 (or linker-2) has the sequence set forth in SEQ ID NO: 10.

In certain embodiments, the anti-CD20 antigen-binding region includes a light chain variable region (V_{L}) comprising an amino acid sequence at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100% identical to the amino acid sequence set forth in SEQ ID NO: 4.

In certain embodiments, the anti-CD20 antigen-binding region includes a heavy chain variable region (V_{H}) comprising an amino acid sequence at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100% identical to the amino acid sequence set forth in SEQ ID NO: 8.

In certain embodiments, the anti-BCMA antigen-binding region includes a light chain variable region (V_{L}) comprising an amino acid sequence at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100% identical to the amino acid sequence set forth in SEQ ID NO: 12.

In certain embodiments, the anti-BCMA antigen-binding region includes a heavy chain variable region (V_{H}) comprising an amino acid sequence at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100% identical to the amino acid sequence set forth in SEQ ID NO:16.

A light chain variable region (V_{L}) of the anti-CD20 antigen-binding region can comprise one, two, or three complementarity determining regions (CDRs), CDR1, CDR2 and CDR3, that are at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100%, identical to CDR1, CDR2 and CDR3 as set forth in position 24-34, position 50-56, position 89-97 of SEQ ID NO:4, respectively (the CDRs of a light chain variable region of the Ofatumumab antibody).

A light chain variable region (V_{L}) of the anti-CD20 antigen-binding region can comprise one, two, or three complementarity determining regions (CDRs), CDR1, CDR2 and CDR3, that are at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100%, identical to CDR1, CDR2 and CDR3 as set forth in SEQ ID NO: 130, SEQ ID NO: 131, SEQ ID NO: 132, respectively (the CDRs of a light chain variable region of the Ofatumumab antibody).

A heavy chain variable region (V_{H}) of the anti-CD20 antigen-binding region can comprise one, two, or three complementarity determining regions (CDRs), CDR1, CDR2 and CDR3, that are at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100%, identical to CDR1, CDR2 and CDR3 as set forth in position 30-35, position 50-66, position 99-111 of SEQ ID NO:8, respectively (the CDRs of a heavy chain variable region of the Ofatumumab antibody).

A heavy chain variable region (V_{H}) of the anti-CD20 antigen-binding region can comprise one, two, or three complementarity determining regions (CDRs), CDR1, CDR2 and CDR3, that are at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100%, identical to CDR1, CDR2 and CDR3 as set forth in SEQ ID NO: 127, SEQ ID NO: 128, SEQ ID NO: 129, respectively (the CDRs of a heavy chain variable region of the Ofatumumab antibody).

In certain embodiments, a light chain variable region (V_{L}) of the anti-CD20 antigen-binding region includes three CDRs, CDR1, CDR2 and CDR3, that are identical to CDR1, CDR2 and CDR3 as set forth in position 24-34, position 50-56, position 89-97 of SEQ ID NO: 4, respectively (CDRs of a light chain variable region of the Ofatumumab antibody), and a heavy chain variable region (V_{H}) of the anti-CD20 antigen-binding region includes three CDRs that are identical to CDR1, CDR2 and CDR3 as set forth in position 30-35, position 50-66, position 99-111 of SEQ ID NO: 8 (CDRs of a heavy chain variable region of the Ofatumumab antibody).

In certain embodiments, a light chain variable region (V_{L}) of the anti-CD20 antigen-binding region includes three CDRs, CDR1, CDR2 and CDR3, that are identical to CDR1, CDR2 and CDR3 as set forth in SEQ ID NO: 130, SEQ ID NO: 131, SEQ ID NO: 132, respectively (the CDRs of a light chain variable region of the Ofatumumab antibody), and a heavy chain variable region (V_{H}) of the anti-CD20 antigen-binding region includes three CDRs that are identical to CDR1, CDR2 and CDR3 as set forth in SEQ ID NO: 127, SEQ ID NO: 128, SEQ ID NO: 129, respectively (the CDRs of a heavy chain variable region of the Ofatumumab antibody).

A light chain variable region (V_{L}) of the anti-BCMA antigen-binding region can comprise one, two, or three complementarity determining regions (CDRs), CDR1, CDR2 and CDR3, that are at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100%, identical to CDR1, CDR2 and CDR3 as set forth position 24-34, position 50-56, position 89-97 of SEQ ID NO: 12, respectively (the CDRs of a light chain variable region of the BCMA-20 antibody).

A light chain variable region (V_{L}) of the anti-BCMA antigen-binding region can comprise one, two, or three complementarity determining regions (CDRs), CDR1, CDR2 and CDR3, that are at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100%, identical to CDR1, CDR2 and CDR3 as set forth SEQ ID NO: 134, SEQ ID NO: 136, SEQ ID NO: 138, respectively (the CDRs of a light chain variable region of the BCMA-20 antibody).

A heavy chain variable region (V_{H}) of the anti-BCMA antigen-binding region can comprise one, two, or three complementarity determining regions (CDRs), CDR1, CDR2 and CDR3, that are at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100%, identical to CDR1, CDR2 and CDR3 as set forth in position 31-35, position 50-66, position 99-110 of SEQ ID NO: 16, respectively (the CDRs of a heavy chain variable region of the BCMA-20 antibody).

A heavy chain variable region (V_{H}) of the anti-BCMA antigen-binding region can comprise one, two, or three complementarity determining regions (CDRs), CDR1, CDR2 and CDR3, that are at least or about 70%, at least or about 75%, at least or about 80%, at least or about 85%, at least or about 90%, at least or about 95%, at least or about 99%, at least or about 81%, at least or about 82%, at least or about 83%, at least or about 84%, at least or about 85%, at least or about 86%, at least or about 87%, at least or about 88%, at least or about 89%, at least or about 90%, at least or about 91%, at least or about 92%, at least or about 93%, at least or about 94%, at least or about 95%, at least or about 96%, at least or about 97%, at least or about 98%, at least or about 99%, or about 100%, identical to CDR1, CDR2 and CDR3 as set forth in SEQ ID NO: 141, SEQ ID NO: 143, SEQ ID NO: 145, respectively (the CDRs of a heavy chain variable region of the BCMA-20 antibody).

In certain embodiments, a light chain variable region (V_{L}) of the anti-BCMA antigen-binding region includes three CDRs, CDR1, CDR2 and CDR3, that are identical to CDR1, CDR2 and CDR3 as set forth position 24-34, position 50-56, position 89-97 of SEQ ID NO: 12, respectively (CDRs of a light chain variable region of the BCMA-20 antibody), and a heavy chain variable region (V_{H}) of the anti-BCMA antigen-binding region includes three CDRs, CDR1, CDR2 and CDR3, that are identical to CDR1, CDR2 and CDR3 as set forth in position 31-35, position 50-66, position 99-110 of SEQ ID NO: 16, respectively (CDRs of a heavy chain variable region of the BCMA-20 antibody).

In certain embodiments, a light chain variable region of the anti-BCMA antigen-binding region includes three CDRs, CDR1, CDR2 and CDR3, that are identical to CDR1, CDR2 and CDR3 as set forth SEQ ID NO: 134, SEQ ID NO: 136, SEQ ID NO: 138, respectively (CDRs of a light chain variable region (V_{L}) of the BCMA-20 antibody), and a heavy chain variable region (V_{H}) of the anti-BCMA antigen-binding region includes three CDRs, CDR1, CDR2 and CDR3, that are identical to CDR1, CDR2 and CDR3 as set forth in SEQ ID NO: 141, SEQ ID NO: 143, SEQ ID NO: 145, respectively (CDRs of a heavy chain variable region of the BCMA-20 antibody).

In certain embodiments, in the present CAR, the antigen binding domain targeting CD20 comprises a light chain variable domain V_{L} (SEQ ID NO: 4) and a heavy chain variable domain V_{H} (SEQ ID NO: 8) derived from the Ofatumumab antibody.

The light chain variable domain V_{L} derived from the Ofatumumab (OF) antibody may have the below sequence:

EIVLTQSPATLSLSPGERATLSCRASQSVSSYLAWYQQKPGQAPRLLIYDASNRATGIPA
RFSGSGSGTDFTLTISSLEPEDFAVYYCQQRSNWPITFGQGTRLEIK (SEQ ID NO: 4)

OF-VL-CDR1: SEQ ID NO: 4, position 24-34. The sequence of OF-VL-CDR1 is: RASQSVSSYLA (SEQ ID NO: 130).

OF-VL-CDR2: SEQ ID NO: 4, position 50-56. The sequence of OF-VL-CDR2 is: DASNRAT (SEQ ID NO: 131).

OF-VL-CDR3: SEQ ID NO: 4, position 89-97. The sequence of OF-VL-CDR3 is: QQRSNWPIT (SEQ ID NO: 132).

The heavy chain variable domain V_{H} derived from the Ofatumumab antibody may have the below sequence:

EVQLVESGGGLVQPGRSLRLSCAASGFTFNDYAMHWVRQAPGKGLEWVSTISWNSGS
IGYADSVKGRFTISRDNAKKSLYLQMNSLRAEDTALYYCAKDIQYGNYYYGMDVWG
QGTTVTVSS (SEQ ID NO: 8)

OF-VH-CDR1: SEQ ID NO: 8, position 30-35. The sequence of OF-VH-CDR1 is: NDYAMH (SEQ ID NO: 127).

OF-VH-CDR2: SEQ ID NO: 8, position 50-66. The sequence of OF-VH-CDR2 is: TISWNSGSIGYADSVKG (SEQ ID NO: 128).

OF-VH-CDR3: SEQ ID NO: 8, position 99-111. The sequence of OF-VH-CDR3 is: DIQYGNYYYGMDV (SEQ ID NO: 129).

In certain embodiments, the antigen-binding domain targeting BCMA in the present CAR comprises a light chain variable domain V_{L} (SEQ ID NO: 12) and a heavy chain variable domain V_{H} (SEQ ID NO: 16) derived from the BCMA-20 (B20) antibody.

The light chain variable domain V_{L} derived from the BCMA-20 antibody may have the below sequence:

DIQMTQSPSSLSASVGDRVTITCRASQGISNYLNWYQQKPGKAPKPLIYYTSNLQSGVPS
RFSGSGSGTDYTLTISSLQPEDFATYYCMGQTISSYTFGQGTKLEIK (SEQ ID NO:12)

B20-VL-CDR1: SEQ ID NO: 12, position 24-34. The sequence of B20-VL-CDR1 is: RASQGISNYLN (SEQ ID NO: 134).

B20-VL-CDR2: SEQ ID NO: 12, position 50-56. The sequence of B20-VL-CDR2 is: YTSNLQS (SEQ ID NO: 136).

B20-VL-CDR3: SEQ ID NO: 12, position 89-97. The sequence of B20-VL-CDR3 is: MGQTISSYT (SEQ ID NO: 138).

The heavy chain variable domain V_{H} derived from the BCMA-20 antibody may have the below sequence:

EVQLVESGGGLVQPGGSLRLSCAASGFTFSNFDMAWVRQAPGKGLVWVSSITTGADHA
IYADSVKGRFTISRDNAKNTLYLQMNSLRAEDTAVYYCVRHGYYDGYHLFDYWGQGT
LVTVSS (SEQ ID NO:16)

B20-VH-CDR1: SEQ ID NO: 16, position 31-35. The sequence of B20-VH-CDR1 is: NFDMA (SEQ ID NO: 141).

B20-VH-CDR2: SEQ ID NO: 16, position 50-66. The sequence of B20-VH-CDR2 is: SITTGADHAIYADSVKG (SEQ ID NO: 143).

B20-VH-CDR3: SEQ ID NO: 16, position 99-110. The sequence of B20-VH-CDR3 is: HGYYDGYHLFDY (SEQ ID NO: 145).

The signal peptide may be the signal peptide of CD8, having the following sequence: MALPVTALLLPLALLLHAARP (SEQ ID NO:2)

The linker between V_{L} and V_{H} (or V_{H} and V_{L}) of the anti-CD20 scFv (linker-1) may have the following sequence: GSTSGGGSGGGSGGGGSS (SEQ ID NO:6)

The linker between the anti-CD20 scFv and the anti-BCMA scFv (linker-2) may have the following sequence: GGGGS (SEQ ID NO:10)

The linker between V_{L} and V_{H} (or V_{H} and V_{L}) of the anti-BCMA scFv (linker-3) may have the following sequence: GGGGSGGGGSGGGGS (SEQ ID NO:14)

The hinge region between the extracellular region (antigen-binding domain) and the transmembrane domain may be derived from IgG4, CD8 (CD8a), CD28, CD137, or combinations thereof.

The hinge region may be derived from CD8a which has the following sequence: FVPVFLPAKPTTTPAPRPPTPAPTIASQPLSLRPEACRPAAGGAVHTRGLDFACD (SEQ ID NO:18)

The hinge region may be derived from IgG4 which has the following sequence:
ESKYGPPCPPCP (SEQ ID NO:78)

The transmembrane domain may be derived from CD8 (CD8TM) which has the following sequence: IYIWAPLAGTCGVLLLSLVITLYC (SEQ ID NO:20)

The transmembrane domain may be derived from CD28 (CD28TM) which has the following sequence: MFWVLVVVGGVLACYSLLVTVAFIIFWV (SEQ ID NO:80)

The co-stimulatory region may be derived from 4-1BB which has the following sequence: KRGRKKLLYIFKQPFMRPVQTTQEEDGCSCRFPEEEEGGCEL (SEQ ID NO:22)

The cytoplasmic signaling domain may be derived from CD3ζ which has the following sequence:

RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQALP
PR (SEQ ID NO:24)

### Chimeric antigen receptors (CARs)

The terms "chimeric antigen receptor," or alternatively "CAR", are used interchangeably throughout and refer to a recombinant polypeptide construct comprising, e.g., an extracellular antigen binding domain, a transmembrane domain and an intracellular domain. Lee et al., Clin. Cancer Res. (2012) 18(10):2780; Jensen et al., Immunol Rev. (2014) 257(1):127. In one embodiment, the stimulatory molecule is the zeta chain associated with the T cell receptor complex. In one aspect, the cytoplasmic signaling domain further comprises one or more functional signaling domains derived from at least one costimulatory molecule. The costimulatory molecule may also be 4-1BB (*i.e.*, CD137), CD27 and/or CD28 or fragments of those molecules. In another aspect, the CAR comprises a chimeric fusion protein comprising an extracellular antigen recognition domain, a transmembrane domain and an intracellular signaling domain comprising a functional signaling domain derived from a stimulatory molecule. The CAR may comprise a chimeric fusion protein comprising an extracellular antigen recognition domain, a transmembrane domain and an intracellular signaling domain comprising a functional signaling domain derived from a co-stimulatory molecule and a functional signaling domain derived from a stimulatory molecule. Alternatively, the CAR may comprise a chimeric fusion protein comprising an extracellular antigen recognition domain, a transmembrane domain and an intracellular signaling domain comprising two functional signaling domains derived from one or more co-stimulatory molecule(s) and a functional signaling domain derived from a stimulatory molecule. The CAR may also comprise a chimeric fusion protein comprising an extracellular antigen recognition domain, a transmembrane domain and an intracellular signaling domain comprising at least two functional signaling domains derived from one or more co-stimulatory molecule(s) and a functional signaling domain derived from a stimulatory molecule. The antigen-binding region of the CAR may contain any antigen-binding antibody fragment. The antibody fragment can comprise one or more CDRs, the variable region (or portions thereof), the constant region (or portions thereof), or combinations of any of the foregoing.

The term "zeta" or alternatively "zeta chain", "CD3-zeta" or "TCR-zeta" may be the protein provided as GenBank accession numbers NP_932170, NP_000725, or XP_011508447; or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" may be the amino acid residues from the cytoplasmic domain of the zeta chain that are sufficient to functionally transmit an initial signal necessary for T cell activation.

A chimeric receptor may refer to a non-naturally occurring molecule that can be expressed on the surface of a host cell and comprises an antigen-binding fragment that binds to an antigen. In addition to the antigen-binding fragment, the chimeric receptor may further comprise one or more of a hinge region, a transmembrane domain, at least one co-stimulatory region, and a cytoplasmic signaling domain. In some embodiments, the chimeric antigen receptor comprises from N terminus to C terminus, an antigen-binding region (or fragment), a hinge region, a transmembrane domain, and a cytoplasmic signaling domain. In some embodiments, the chimeric antigen receptor further comprises at least one co-stimulatory region. Thus, the chimeric antigen receptor may comprise from N terminus to C terminus, an antigen-binding region (or fragment), a hinge region, a transmembrane domain, a co-stimulatory region, and a cytoplasmic signaling domain.

In some embodiments, the chimeric antigen receptors comprise a hinge region, which may be located between the antigen-binding region and a transmembrane domain. The hinge region may contain about 10-200 amino acids, e.g., 15-150 amino acids, 20-100 amino acids, or 30-60 amino acids. In some embodiments, the hinge region may be of about 10, 11, 12, 13, 14,15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, or 200 amino acids in length. The hinge region may contain 0-300 amino acids, 2 amino acids to 100 amino acids, 5 amino acids to 80 amino acids, 10 amino acids to 60 amino acids, 10 amino acids to 15 amino acids, 20 amino acids to 80 amino acids, 30 amino acids to 70 amino acids, 40 amino acids to 60 amino acids, 50 amino acids to 60 amino acids, or 30 amino acids to 60 amino acids.

In some embodiments, the hinge region is a hinge domain of a naturally occurring protein. Hinge domains of any protein known in the art to comprise a hinge domain are compatible for use in the chimeric antigen receptors. In some embodiments, the hinge domain is of CD8α or CD28α. In some embodiments, the hinge domain is a portion of the hinge domain of CD8α, e.g., a fragment containing at least 15 (*e.g.,* 20, 25, 30, 35, or 40) consecutive amino acids of the hinge domain of CD8α or CD28α.

Hinge domains of antibodies, such as an IgG, IgA, IgM, IgE, or IgD antibody, are also compatible for use in the chimeric antigen receptors. In some embodiments, the hinge region is the hinge domain that joins the constant domains CH1 and CH2 of an antibody. In some embodiments, the hinge region is of an antibody and comprises the hinge domain of the antibody and one or more constant regions of the antibody. In some embodiments, the hinge region comprises the hinge domain of an antibody and the CH3 constant region of the antibody. In some embodiments, the hinge region comprises the hinge domain of an antibody and the CH2 and CH3 constant regions of the antibody. In some embodiments, the antibody is an IgG, IgA, IgM, IgE, or IgD antibody. In some embodiments, the antibody is an IgG antibody. In some embodiments, the antibody is an IgG1, IgG2, IgG3, or IgG4 antibody. In some embodiments, the hinge region comprises the hinge region and the CH2 and CH3 constant regions of an IgG4 antibody. In some embodiments, the hinge region comprises the hinge region and the CH3 constant region of an IgG4 antibody.

The hinge region may be a non-naturally occurring peptide. In some embodiments, the hinge region between the extracellular antigen-binding domain and the transmembrane domain is a peptide linker, such as a (GlyxSer)n (or (GxS)n) linker, wherein x and n, independently can be an integer between 3 and 12, including 3, 4, 5, 6, 7, 8, 9, 10, 11, 12, or more.

Additional peptide linkers that may be used in a hinge region of the chimeric receptors described herein are known in the art. See, *e.g.*, Wriggers et al. Current Trends in Peptide Science (2005) 80(6): 736-746 and PCT Publication WO 2012/088461.

In some embodiments, the chimeric antigen receptors may comprise a transmembrane domain. The transmembrane domain can be in any form known in the art. Transmembrane domains compatible for use in the chimeric antigen receptors may be obtained from a naturally occurring protein. Alternatively, the transmembrane domain may be a synthetic, non-naturally occurring protein segment, *e.g.*, a hydrophobic protein segment that is thermodynamically stable in a cell membrane.

In some embodiments, the transmembrane domain is that of CD8α. In some embodiments, the transmembrane domain is that of CD28. In some embodiments, the transmembrane domain is that of ICOS.

In some embodiments, the chimeric antigen receptors comprise one or more costimulatory regions. A co-stimulatory region may be at least a portion of a protein that mediates signal transduction within a cell to induce an immune response, such as an effector function. The co-stimulatory region of the chimeric antigen receptor can be from a protein which transduces a signal and modulates responses mediated by immune cells, such as T cells, natural killer (NK) cells, macrophages, neutrophils, or eosinophils.

In some embodiments, the chimeric antigen receptor comprises one or more than one (at least 2, 3, 4, or more) co-stimulatory region. In some embodiments, the chimeric antigen receptor comprises more than one co-stimulatory region obtained from different proteins. In some embodiments, the chimeric antigen receptor does not comprise a co-stimulatory region.

Examples of co-stimulatory regions for use in the chimeric antigen receptors can be a domain from co-stimulatory proteins, including, without limitation, CD27, CD28, 4-1BB, OX40, CD30, Cd40, PD-1, ICOS, lymphocyte function-associated antigen-1 (LFA-1), CD2, CD7, LIGHT, NKG2C, B7-H3. In some embodiments, the co-stimulatory region is derived from 4-1BB, CD28, or ICOS. In some embodiments, the co-stimulatory region is derived from CD28 and the chimeric antigen receptor comprises a second co-stimulatory region from 4-1BB or ICOS. In some embodiments, the co-stimulatory region is a fusion domain comprising more than one co-stimulatory region or portions of more than one co-stimulatory region. In some embodiments, the costimulatory region is a fusion of costimulatory regions from CD28 and ICOS.

In some embodiments, the chimeric antigen receptors comprise a cytoplasmic signaling domain. Any cytoplasmic signaling domain can be used in the chimeric antigen receptors described herein. A cytoplasmic signaling domain may relay a signal, such as interaction of an extracellular ligand-binding domain with its ligand, to stimulate a cellular response, such as inducing an effector function of the cell (*e.g.,* cytotoxicity).

The chimeric antigen receptors can be prepared by routine methods, such as recombinant technology. Methods for preparing the chimeric antigen receptors may involve generation of a nucleic acid that encodes a polypeptide comprising each of the domains of the chimeric antigen receptors, including the antigen-binding fragment and optionally, the hinge region, the transmembrane domain, at least one co-stimulatory region, and the cytoplasmic signaling domain. In some embodiments, nucleic acids encoding each of the components of the chimeric antigen receptor are joined together using recombinant technology. Sequences of each of the components (*e.g.,* domains) can be joined directly or indirectly (*e.g.,* using a nucleic acid sequence encoding a peptide linker) to form a nucleic acid sequence encoding the chimeric antigen receptor, using methods such as PCR amplification or ligation. Alternatively, the nucleic acid encoding the chimeric antigen receptor may be synthesized. In some embodiments, the nucleic acid is DNA. In other embodiments, the nucleic acid is RNA.

In one embodiment, the present CAR, from the N-terminus to C-terminus, comprises a signal peptide (also known as leader sequence), an antigen recognition sequence (antigen-binding domain), a hinge region, a transmembrane domain, a co-stimulatory region, and a cytoplasmic signaling domain (e.g., a CD3zeta signaling region (ζ chain portion)).

Bispecificity means that the CAR can specifically bind two different antigens. The bispecific CAR may generate an immune response by binding to one antigen or both antigens.

As used herein, the terms "CAR-T cell", "CAR-T", "CART", "CART cell" may refer to the T cell that expresses the present CAR targeting both CD20 and BCMA.

### Immune cells expressing chimeric antigen receptors

The present disclosure also provides immune cells expressing the present CAR. Recognition of a target cell having the antigen(s) on its cell surface by the antigen-binding fragment of the chimeric antigen receptor may transduce an activation signal to the signaling domain(s) (*e.g.,* co-stimulatory region and/or the cytoplasmic signaling domain) of the chimeric antigen receptor, which may activate an effector function in the immune cell expressing the chimeric antigen receptor.

The chimeric antigen receptor can be introduced into a suitable immune cell for expression via conventional technology. In some embodiments, the immune cells are T cells, such as primary T cells or T cell lines. Alternatively, the immune cells can be natural killer (NK) cells, such as established NK cell lines (*e.g.,* NK-92 cells). In some embodiments, the immune cells are T cells that express CD8 (CD8⁺) or CD8 and CD4 (CD8⁺/CD4⁺). In some embodiments, the T cells are T cells of an established T cell line, for example, Jurkat cells.

Primary T cells may be obtained from any source, such as peripheral blood mononuclear cells (PBMCs), bone marrow, tissues such as spleen, lymph node, thymus, or tumor tissue. In some embodiments, the population of immune cells is derived from a human patient having an autoimmune disorder or cancer (e.g., hematopoietic malignancy), such as from the bone marrow or from PBMCs obtained from the patient. In some embodiments, the population of immune cells is derived from a healthy donor. In some embodiments, the immune cells are obtained from the subject to whom the immune cells expressing the chimeric antigen receptors will be subsequently administered. Immune cells that are administered to the same subject from which the cells were obtained are referred to as autologous cells, whereas immune cells that are obtained from a subject who is not the subject to whom the cells will be administered may be referred to as allogeneic cells.

The type of immune cells desired may be expanded within the population of cells obtained by co-incubating the cells with stimulatory molecules, for example, anti-CD3 and anti-CD28 antibodies may be used for expansion of T cells.

To construct the immune cells that express the chimeric antigen receptors described herein, vectors for stable or transient expression of the chimeric antigen receptor may be constructed via conventional methods as described herein and introduced into immune cells. For example, nucleic acids encoding the chimeric antigen receptors may be cloned into a suitable vector, such as a viral vector.

In certain embodiments, immune cells (e.g., T cells) are transduced with lentiviral vectors (LVs) encoding the present CAR. The transduced immune cells (e.g., T cells) can target CD20 and BCMA, synergistically activate the T cells, and induce T cell-mediated immune responses.

In one embodiment, in the present method, T cells from an autologous patient (or an allogeneic donor) are isolated, activated and genetically modified to generate CAR-T cells expressing the present CAR, and then administered to the patient. CAR-T cells can replicate *in vivo* resulting in long-term persistence. In addition, the CAR-mediated immune response may be part of an adoptive immunotherapy approach in which the anti-CD20/BCMA CAR-T cells elicit an immune response against cells expressing CD20 and/or BCMA.

In certain embodiments, cells are isolated from a mammal (e.g., a human) and genetically modified (i.e., transduced or transfected *in vitro*) with a vector expressing a CAR disclosed herein. The CAR-modified cells can be administered to a mammalian recipient to provide a therapeutic benefit. The mammalian recipient may be a human. The CAR-modified cell can be autologous with respect to the recipient. Alternatively, the cells can be allogeneic, syngeneic or xenogeneic with respect to the recipient.

The methods of preparing immune cells expressing the present chimeric antigen receptors may comprise activating and/or expanding the immune cells *ex vivo*. Activating an immune cell means stimulating an immune cell into an activated state in which the cell may be able to perform effector functions (*e.g.,* cytotoxicity). Methods of activating an immune cell will depend on the type of the immune cell used for expression of the chimeric antigen receptors. Expanding immune cells may involve any method that results in an increase in the number of cells expressing chimeric antigen receptors, for example, allowing the cells to proliferate or stimulating the cells to proliferate. In some embodiments, the cells expressing the chimeric receptors described herein are activated and/or expanded *ex vivo* prior to administration to a subject.

The CAR-expressing immune cells may also serve as a vaccine for *ex vivo* immunization and/or *in vivo* therapy in a mammal. In addition to using a cell-based vaccine in terms of *ex vivo* immunization, the present disclosure also provides compositions and methods for *in vivo* immunization to elicit an immune response directed against an antigen in a patient. Preferably, the mammal is a human. With respect to *ex vivo* immunization, one or more of the following may occur *in vitro* prior to administering the cell into a mammal: i) expanding the cells, ii) introducing a nucleic acid encoding a CAR to the cells, and/or iii) cryopreservation of the cells.

### Vectors

The present disclosure provides a nucleic acid encoding the present CAR. The present disclosure also provides vectors comprising the present nucleic acid.

The vectors include, but are not limited to, a plasmid, a phagemid, a phage derivative, a virus, and a cosmid.

The vector may be a viral vector. Viruses, which are useful as vectors comprise, but are not limited to, retroviruses, adenoviruses, adeno-associated viruses, herpes viruses, and lentiviruses. In certain embodiments, the present vector is a retroviral vector such as a lentiviral vector. In some embodiments, the vectors for expression of the chimeric antigen receptors are retroviruses. In some embodiments, the vectors for expression of the chimeric antigen receptors are lentiviruses. In some embodiments, the vectors for expression of the chimeric antigen receptors are adeno-associated viruses.

A variety of promoters can be used for expression of the chimeric receptors, including, without limitation, cytomegalovirus (CMV) intermediate early promoter, a viral LTR such as the Rous sarcoma virus LTR, HIV-LTR, HTLV-1 LTR, Maloney murine leukemia virus (MMLV) LTR, myeoloproliferative sarcoma virus (MPSV) LTR, spleen focus-forming virus (SFFV) LTR, the simian virus 40 (SV40) early promoter, herpes simplex tk virus promoter, elongation factor 1-alpha (EF1-α) promoter with or without the EF1-α intron. Additional promoters for expression of the chimeric receptors include any constitutively active promoter in an immune cell. Alternatively, any regulatable promoter (e.g., inducible promoters) may be used, such that its expression can be modulated within an immune cell.

The vector can be introduced into a cell, e.g., mammalian, bacterial, yeast, or insect cell, by any method in the art. For example, the vector can be transferred into a cell by physical, chemical, or biological means.

Physical methods for introducing a polynucleotide into a cell comprise calcium phosphate precipitation, lipofection, particle bombardment, microinjection, electroporation, and the like. See, for example, Sambrook et al. (2001, Molecular Cloning: A Laboratory Manual, Cold Spring Harbor Laboratory, New York).

Biological methods for introducing a polynucleotide of interest into a cell comprise the use of DNA and RNA vectors. Viral vectors can be derived from retroviruses, lentiviruses, poxviruses, herpes simplex virus I, adenoviruses and adeno-associated viruses, and the like.

Chemical means for introducing a polynucleotide into a host cell comprise colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In some embodiments, the vector (nucleic acid) encoding the chimeric antigen receptor is a DNA vector and may be electroporated to immune cells (see, e.g., Till, et al. Blood (2012) 119(17): 3940-3950). In some embodiments, the vector (nucleic acid) encoding the chimeric antigen receptor is an RNA molecule, which may be electroporated to immune cells.

Any of the vectors comprising a nucleic acid that encodes a chimeric antigen receptor described herein is also within the scope of the present disclosure. Such a vector may be delivered into host cells such as immune cells by a suitable method. Methods of delivering vectors to immune cells are well known in the art and may include DNA, RNA, or transposon electroporation, transfection reagents such as liposomes or nanoparticles to delivery DNA, RNA, or transposons; delivery of DNA, RNA, or transposons or protein by mechanical deformation (see, *e.g.*, Sharei et al. PNAS (2013) 110(6): 2082-2087); or viral transduction. In some embodiments, the vectors for expression of the chimeric receptors are delivered to cells by viral transduction.

In examples in which the vectors encoding chimeric antigen receptors are introduced to the host cells using a viral vector, viral particles that are capable of infecting the immune cells and carry the vector may be produced by any method known in the art. The viral particles are harvested from the cell culture supernatant and may be isolated and/or purified prior to contacting the viral particles with the immune cells.

### Pharmaceutical compositions

The present disclosure provides a pharmaceutical composition comprising the present immune cells, the present CAR, the present nucleic acid, or the present vector. The present pharmaceutical composition may further comprise a pharmaceutically acceptable carrier, diluent or excipient. In one embodiment, the preparation is a liquid preparation. In one embodiment, the concentration of the immune cells (e.g., CAR-T cells) in the preparation is 1 × 10³-1×10⁸ cells/mL, or 1 × 10⁴-1×10⁷ cells/mL.

Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. In some embodiments, the effective amount alleviates, relieves, ameliorates, improves, reduces the symptoms, or delays the progression of a disease or disorder in the subject. In some embodiments, the subject is a mammal. In some embodiments, the subject is a human.

Pharmaceutically acceptable carriers, including buffers, are well known in the art, and may comprise phosphate, citrate, and other organic acids; antioxidants including ascorbic acid and methionine; preservatives; low molecular weight polypeptides; proteins, such as serum albumin, gelatin, or immunoglobulins; amino acids; hydrophobic polymers; monosaccharides; disaccharides; and other carbohydrates; metal complexes; and/or non-ionic surfactants. See, e.g. Remington: The Science and Practice of Pharmacy 20th Ed. (2000) Lippincott Williams and Wilkins, Ed. K. E. Hoover.

The present pharmaceutical composition may be delivered to a cell by contacting the cell with the present pharmaceutical composition.

The present pharmaceutical composition may be delivered/administered to a subject by any route, including, without limitation, intravenous, intracerebroventricular (ICV) injection, intracisternal injection or infusion, oral, transdermal, ocular, intraperitoneal, subcutaneous, implant, sublingual, subcutaneous, intramuscular, rectal, mucosal, ophthalmic, intrathecal, intra-articular, intra-arterial, sub-arachinoid, bronchial and lymphatic administration. The present pharmaceutical composition may be administered parenterally or systemically. The present composition may be administered locally. The pharmaceutical composition may be formulated for intravenous administration.

The administration of the present compositions may be carried out in any convenient manner, including by aerosol inhalation, injection, ingestion, transfusion, implantation or transplantation. The compositions may be administered to a patient subcutaneously, intradermaliy, intratumorally, intranodally, intramedullary, intramuscularly, by intravenous (*i.v.*) injection, or intraperitoneally. In one embodiment, the compositions are administered to a subject (e.g., a patient) by intradermal or subcutaneous injection. In another embodiment, the compositions are administered by *i.v.* injection. The compositions may be injected directly into a tumor, lymph node, or site of disorder.

The present immune cells or pharmaceutical composition may be delivered/administered to a subject via intravenous, intramuscular, subcutaneous, intraperitoneal, spinal or other parenteral administration, such as by injection or infusion.

The present pharmaceutical compositions may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an effective amount", "a therapeutically effective amount", or "a therapeutic amount" is indicated, the precise amount of the compositions to be administered can be determined by a physician with consideration of individual differences in age, weight, tumor size, extent of infection or metastasis, and condition of the patient (subject). A pharmaceutical composition comprising the immune cells may be administered at a dosage of 10⁴ to 10⁹ cells/kg body weight, or 10⁵ to 10⁶ cells/kg body weight, including all integer values within those ranges. The compositions may also be administered multiple times at these dosages. The cells can be administered by using infusion techniques that are commonly known in immunotherapy (see, e.g., Rosenberg et al, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The dosage of the above treatments to be administered to a patient may vary with the precise nature of the condition being treated and the recipient of the treatment. The scaling of dosages for patient administration can be performed according to art-accepted practices. In one embodiment, 1×10⁶ to 1×10¹⁰ of the immune cells (e.g., CAR-T cells) can be administered to a patient by means of, for example, intravenous infusion for each treatment or each course of treatment.

### Conditions to be treated

The present CAR, immune cells or pharmaceutical composition may be used to treat an autoimmune disease/disorder, or to treat cancer or tumor.

In certain embodiments, the present anti-CD20/BCMA bispecific CAR targets both B cells and plasma cells, which may reduce/eradicate autoimmune antibodies. In certain embodiments, the present anti-CD20/BCMA bispecific CAR may reduce/deplete B cells, plasmablasts, and/or long-lived plasma cells (LLPCs) to reduce/eradiate autoantibody production.

The present disclosure provides for a method of treating an autoimmune disease/disorder. The method may comprise administering the CAR, immune cells or pharmaceutical composition to a subject in need thereof.

The autoimmune disorder may be systemic lupus erythematosus (SLE), lupus nephritis (LN), systemic sclerosis (SSc), CREST syndrome (calcinosis, Raynaud's syndrome, esophageal dysmotility, sclerodactyl, and telangiectasia), opsoclonus, inflammatory myopathy (e.g., polymyositis, dermatomyositis, and inclusion-body myositis), myositis autoantibody-driven diseases, systemic scleroderma, primary biliary cirrhosis, celiac disease (e.g., gluten sensitive enteropathy), dermatitis herpetiformis, Miller-Fisher Syndrome, acute motor axonal neuropathy (AMAN), multifocal motor neuropathy with conduction block, autoimmune hepatitis, antiphospholipid syndrome, Wegener's granulomatosis, microscopic polyangiitis, Churg-Strauss syndrome, rheumatoid arthritis, chronic autoimmune hepatitis, scleromyositis, myasthenia gravis (MG), Lambert-Eaton myasthenic syndrome, Hashimoto's thyroiditis, Graves' disease, Paraneoplastic cerebellar degeneration, Stiff person syndrome, limbic encephalitis, Isaacs Syndrome, Sydenham's chorea, pediatric autoimmune neuropsychiatric disease associated with Streptococcus (PANDAS), encephalitis, diabetes mellitus type 1, neuromyelitis optica (NMO), chronic inflammatory bowel disease, Hashimoto's disease, organ transplant rejection, and/or neuromyelitis optica spectrum disorder (NMOSD).

The autoimmune disorder may be pernicious anemia, Addison's disease, psoriasis, inflammatory bowel disease (IBD), psoriatic arthritis, Sjögren's syndrome, lupus erythematosus (e.g., discoid lupus erythematosus, drug-induced lupus erythematosus, and neonatal lupus erythematosus), multiple sclerosis, and/or reactive arthritis.

The autoimmune disorder may be polymyositis, dermatomyositis, multiple endocrine failure, Schmidt's syndrome, autoimmune uveitis, adrenalitis, thyroiditis, autoimmune thyroid disease, gastric atrophy, chronic hepatitis, lupoid hepatitis, atherosclerosis, presenile dementia, demyelinating diseases, subacute cutaneous lupus erythematosus, hypoparathyroidism, Dressler's syndrome, autoimmune thrombocytopenia, idiopathic thrombocytopenic purpura, hemolytic anemia, pemphigus vulgaris, pemphigus, alopecia arcata, pemphigoid, scleroderma, progressive systemic sclerosis, adult onset diabetes mellitus (e.g., type II diabetes), male and female autoimmune infertility, ankylosing spondolytis, ulcerative colitis, Crohn's disease, sprue, mixed connective tissue disease, polyarteritis nedosa, systemic necrotizing vasculitis, juvenile onset rheumatoid arthritis, glomerulonephritis, atopic dermatitis, atopic rhinitis, Goodpasture's syndrome, Chagas' disease, sarcoidosis, rheumatic fever, asthma, recurrent abortion, antiphospholipid syndrome, farmer's lung, erythema multiforme, post cardiotomy syndrome, Cushing's syndrome, autoimmune chronic active hepatitis, bird-fancier's lung, allergic disease, allergic encephalomyelitis, toxic epidermal necrolysis, alopecia, Alport's syndrome, alveolitis, allergic alveolitis, fibrosing alveolitis, interstitial lung disease, erythema nodosum, pyoderma gangrenosum, transfusion reaction, leprosy, malaria, leishmaniasis, trypanosomiasis, Takayasu's arteritis, polymyalgia rheumatica, temporal arteritis, schistosomiasis, giant cell arteritis, ascariasis, aspergillosis, Sampter's syndrome, eczema, lymphomatoid granulomatosis, Behcet's disease, Caplan's syndrome, Kawasaki's disease, dengue, endocarditis, endomyocardial fibrosis, endophthalmitis, erythema elevatum et diutinum, erythroblastosis fetalis, eosinophilic faciitis, Shulman's syndrome, Felty's syndrome, filariasis, cyclitis, chronic cyclitis, heterochronic cyclitis, Fuch's cyclitis, IgA nephropathy, Henoch-Schonlein purpura, graft versus host disease, transplantation rejection, human immunodeficiency virus infection, echovirus infection, cardiomyopathy, Alzheimer's disease, parvovirus infection, rubella virus infection, post vaccination syndromes, congenital rubella infection, Hodgkin's and non-Hodgkin's lymphoma, renal cell carcinoma, multiple myeloma, Eaton-Lambert syndrome, relapsing polychondritis, malignant melanoma, cryoglobulinemia, Waldenstrom's macroglobulemia, Epstein-Barr virus infection, mumps, Evan's syndrome, and/or autoimmune gonadal failure.

The autoimmune diseases also include, e.g., acute disseminated encephalomyelitis, alopecia areata, antiphospholipid syndrome, autoimmune hepatitis, autoimmune myocarditis, autoimmune pancreatitis, autoimmune polyendocrine syndromesautoimmune uveitis, inflammatory bowel disease (Crohn's disease, ulcerative colitis), type I diabetes mellitus (e.g. , juvenile onset diabetes), multiple sclerosis, scleroderma, ankylosing spondylitis, sarcoid, pemphigus vulgaris, pemphigoid, psoriasis, myasthenia gravis, systemic lupus erythematosus, rheumatoid arthritis, juvenile arthritis, psoriatic arthritis, Behcet's syndrome, Reiter's disease, Berger's disease, dermatomyositis, polymyositis, antineutrophil cytoplasmic antibody-associated vasculitides (e.g., granulomatosis with polyangiitis (also known as Wegener's granulomatosis), microscopic polyangjitis, and Churg-Strauss syndrome), scleroderma, Sjogren's syndrome, anti-glomerular basement membrane disease (including Goodpasture's syndrome), dilated cardiomyopathy, primary biliary cirrhosis, thyroiditis (e.g., Hashimoto's thyroiditis, Graves' disease), transverse myelitis, allergies, arthritis, fibromyalgia, fibromytosis, lupus, vitiligo, and Guillane-Barre syndrome.

The autoimmune diseases include inflammatory bowel disease (IBD), ulcerative colitis, Crohn's disease, sprue, autoimmune arthritis, rheumatoid arthritis, Type I diabetes, multiple sclerosis, graft vs. host disease following bone marrow transplantation, osteoarthritis, juvenile chronic arthritis, Lyme arthritis, psoriatic arthritis, reactive arthritis, spondy loarthropathy, systemic lupus erythematosus, insulin dependent diabetes mellitus, thyroiditis, asthma, psoriasis, dermatitis scleroderma, atopic dermatitis, graft versus host disease, acute or chronic immune disease associated with organ transplantation, sarcoidosis, atherosclerosis, disseminated intravascular coagulation, Kawasaki's disease, Grave's disease, nephrotic syndrome, chronic fatigue syndrome, Wegener's granulomatosis, Henoch-Schoenlejn purpurea, microscopic vasculitis of the kidneys, chronic active hepatitis, uveitis, septic shock, toxic shock syndrome, sepsis syndrome, cachexia, acquired immunodeficiency syndrome, acute transverse myelitis, Huntington's chorea, Parkinson's disease, Alzheimer's disease, stroke, primary biliary cirrhosis, hemolytic anemia, polyglandular deficiency type I syndrome and polyglandular deficiency type II syndrome, Schmidt's syndrorme, adult (acute) respiratory distress syndrome, alopecia, alopecia areata, seronegative arthopathy, arthropathy, Reiter's disease, psoriatic arthropathy, chlamydia, yersinia and salmonella associated arthropathy spondyloarhopathy, atheromatous disease/arteriosclerosis, atopic allergy, food allergies, autoimmune bullous disease, pemphigus vulgaris, pemphigus foliaceus, pemphigoid, linear IgA disease, autoimmune haemolytic anaemia, Coombs positive haemolytic anaemia, acquired pernicious anaemia, juvenile pernicious anaemia, myalgic encephalitis/Royal Free Disease, chronic mucocutaneous candidiasis, giant cell arteritis, primary sclerosing hepatitis, cryptogenic autoimmune hepatitis, Acquired Immunodeficiency Disease Syndrome, Acquired Immunodeficiency Related Diseases, Hepatitis C, common varied immunodeficiency (common variable hypogammaglobulinaemia), dilated cardiomyopathy, fibrotic lung disease, cryptogenic fibrosing alveolitis, postinflammatory interstitial lung disease, interstitial pneumonitis, connective tissue disease associated interstitial lung disease, mixed connective tissue disease associated lung disease, systemic sclerosis associated interstitial lung disease, rheumatoid arthritis associated interstitial lung disease, systemic lupus erythematosus associated lung disease, dermatomyositis/polymyositis associated lung disease, Sjogren's disease associated lung disease, ankylosing spondy litis associated lung disease, vasculitic diffuse lung disease, haemosiderosis associated lung disease, drug-induced interstitial lung disease, radiation fibrosis, bronchiolitis obliterans, chronic eosinophilic pneumonia, lymphocytic infiltrative lung disease, postinfectious interstitial lung disease, gouty arthritis, autoimmune hepatitis, type-1 autoimmune hepatitis (classical autoimmune or lupoid hepatitis), type-2 autoimmune hepatitis (anti-LKM antibody hepatitis), autoimmune mediated hypoglycemia, type B insulin resistance with acanthosis nigricans, hypoparathyroidism, acute immune disease associated with organ transplantation, chronic immune disease associated with organ transplantation, osteoarthrosis, primary sclerosing cholangitis, idiopathic leucopenia, autoimmune neutropenia, renal disease NOS, glomerulonephritides, microscopic vasulitis of the kidneys, discoid lupus, erythematosus, male infertility idiopathic or NOS, sperm autoimmunity, multiple sclerosis (all subtypes), insulindependent diabetes mellitus, sympathetic ophthalmia, pulmonary hypertension secondary to connective tissue disease, Goodpasture's syndrome, pulmonary manifestation of polyarteritis nodosa, acute rheumatio fever, rheumatoid spondylitis, Still's disease, systemic sclerosis, Takayasu's disease/arteritis, autoimmune thrombocytopenia, idiopathic thrombocytopenia, autoimmune thyroid disease, hyperthyroidism, goitrous autoimmune hypothyroidism (Hashimoto's disease), atrophic autoimmune hypothyroidism, primary myxoedema, phacogenic uveitis, primary vasculitis, vitiligo, allergic rhinitis (pollen allergies), anaphylaxis, pet allergies, latex allergies, drug allergies, allergic rhinoconjuctivitis, eosinophilic esophagitis, hypereosinophilic syndrome, eosinophilic gastroenteritis cutaneous lupus erythematosus, eosinophilic esophagitis, hypereosinophilic syndrome, and eosinophilic gastroenteritis.

The autoimmune disorder may be an inflammatory muscle disease. Inflammatory myopathies are a group of diseases that involve chronic muscle inflammation, muscle weakness, and in some cases, muscle pain. The four main types of chronic, or long-term, inflammatory myopathy are: polymyositis, which affects skeletal muscles (the type involved in body movement) on both sides of the body; dermatomyositis, which causes progressive muscle weakness; inclusion body myositis, which is characterized by slow, progressive muscle weakness and muscle shrinking and loss of muscle; and necrotizing autoimmune myopathy, which involves muscle weakness in the upper and lower body.

In another embodiment, the autoimmune disease is an autoimmune disease caused by overexpression of B cells (such as lupus erythematosus).

Also encompassed by the present disclosure is a method of treating cancer. The method may comprise administering the CAR, immune cells or pharmaceutical composition to a subject in need thereof.

The present disclosure provides chimeric antigen receptors for treating CD20-positive diseases such as B cell lymphoma.

The cancer may be a BCMA-positive malignancy. The cancer may be multiple myeloma (MM), or plasma cell leukemia.

The cancer may be a hematologic cancer. The cancer may be a plasma-cell malignancy. The cancer may be a B-cell malignancy. The B-cell malignancy may be acute lymphocytic leukemia (ALL), chronic lymphocytic leukemia (CLL), B-cell acute lymphoblastic leukemia (BALL), B-cell leukemia, or B cell lymphoma.

The cancer may be Hodgkin's lymphoma, non-Hodgkin's lymphoma, leukemia, and/or multiple myeloma (MM).

The cancer may be acute myeloid leukemia (AML), multiple myeloma (MM), chronic lymphocytic leukemia (CLL), chronic myelogenous leukemia, acute lymphoblastic leukemia (ALL), diffuse large B cell lymphoma (DLBCL), or a combination thereof.

Diseases that may be treated using the present CAR, immune cells or pharmaceutical composition include CD20-positive tumors and diseases, e.g., caused by excessive B cells (such as autoimmune diseases, for example, lupus erythematosus, etc.). CD20-positive tumors may include CD20-positive non-solid tumors (such as hematological cancer, for example, leukemias and lymphomas) or solid tumors. Tumors or cancers to be treated with present CAR, immune cells or pharmaceutical composition include, but are not limited to, carcinoma, blastoma, and sarcoma, and leukemia or lymphoid malignancies, benign and malignant tumors, and malignancies e.g., sarcomas, carcinomas, gastric cancer, peritoneal metastasis of gastric cancer, liver cancer, renal cancer, lung cancer, small intestine cancer, bone cancer, prostate cancer, colorectal cancer, breast cancer, large intestine cancer, cervical cancer, ovarian cancer, lymphoma, nasopharyngeal carcinoma, adrenal tumor, bladder tumor, non-small cell lung cancer (NSCLC), glioma, endometrial cancer, and melanomas. Adult tumors/cancers and pediatric tumors/cancers are included.

Hematologic cancers are cancers of the blood or bone marrow. Examples of hematological (or hematogenous) cancers include leukemias, e.g., acute leukemias (such as acute lymphocytic leukemia, acute myelocytic leukemia, acute myelogenous leukemia and myeloblasts, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

The cancer may be a solid tumor. Solid tumors can be benign or malignant. Different types of solid tumors are named for the type of cells that form them (such as sarcomas, carcinomas, and lymphomas). Examples of solid tumors, such as sarcomas and carcinomas, include fibrosarcoma, myxosarcoma, liposarcoma, mesothelioma, malignant lymphoma, pancreatic cancer and ovarian cancer.

### Kits

Also within the scope of the present disclosure are kits for use of the present CARs, immune cells, nucleic acids, vectors or pharmaceutical compositions. Such kits may include one or more containers comprising the present CARs, immune cells, nucleic acids, vectors or pharmaceutical compositions.

In some embodiments, the kit can comprise instructions for use in any of the methods described herein. The included instructions can comprise a description of administration of the pharmaceutical composition to a subject to achieve the intended activity in a subject. The kit may further comprise a description of selecting a subject suitable for treatment based on identifying whether the subject is in need of the treatment. In some embodiments, the instructions comprise a description of administering the pharmaceutical compositions to a subject who is in need of the treatment.

The instructions relating to the use of the pharmaceutical compositions generally include information as to dosage, dosing schedule, and route of administration for the intended treatment. The containers may be unit doses, bulk packages (*e.g.,* multi-dose packages) or sub-unit doses.

The kits provided herein are in suitable packaging. Suitable packaging includes, but is not limited to, vials, bottles, jars, flexible packaging, and the like.

The following examples of specific aspects for carrying out the present disclosure are offered for illustrative purposes only, and are not intended to limit the scope of the present disclosure in any way.

### Example 1 Construction of anti-CD20/BCMA CARs

We prepared eight bispecific CARs having the anti-CD20 scFv and anti-BCMA scFv in the same order (i.e., anti-CD20 scFv ("OF") followed by anti-BCMA scFv ("B20")), but with different V_{H}/V_{L} orders and having different hinge regions and/or transmembrane domains: TOB1-4 and TOBL1-4, where TOBL1 is C-CAR168 (Figure 1).

The anti-CD20/BCMA CAR-T cells were prepared using apheresis from healthy donors. Specifically, PBMCs were isolated from the venous blood of healthy donors by density gradient centrifugation. On day 0, PBMCs were activated in a cell culture flask previously coated with CD3 monoclonal antibody (OKT3) and Retronectin (TAKARA). The medium was GT-551 cell culture medium containing 1% human albumin and 300 U/mL recombinant human interleukin 2 (IL-2). On day 3, activated PBMCs were transduced with lentiviral vectors encoding the anti-CD20/BCMA CARs.

Figure 2 shows the expression levels of the anti-CD20 and anti-BCMA CARs on the surface of the T cells. The expression levels of anti-BCMA CARs were detected by flow cytometry using BCMA-Fc fusion protein; the expression levels of anti-CD20 CARs were detected by flow cytometry using antibody specific to OF scFv.

### Example 2 Antigen-specific activation of anti-CD20/BCMA CAR-T cells in vitro

Antigen-specific activation of the anti-CD20/BCMA CAR-T was evaluated by assaying IFN-γ release and CD137 expression when the CAR-T cells were co-cultured with target cells. Target cells ("T") included CD20-positive A549-CD20+ tumor cells, BCMA-positive A549-BCMA+ tumor cells, CD20 and BCMA double positive A549-CD20+BCMA+ tumor cells, Raji cells, MM.1S cells, and double negative A549 tumor cells. Effector cells ("E") are the anti-CD20/BCMA CAR-T cells.

PBMCs were isolated from the venous blood of healthy donors by density gradient centrifugation. On day 0, PBMCs were activated in a cell culture flask previously coated with CD3 monoclonal antibody (OKT3) and Retronectin (TAKARA). The medium was GT-551 cell culture medium containing 1% human albumin and 300 U/mL recombinant human interleukin 2 (IL-2). On day 3, activated PBMCs were transduced with lentiviral vectors encoding the anti-CD20/BCMA CARs. Starting from day 6, the CAR-T cells can be taken for activity assays.

IFNγ release was assayed using the CAR-T cells cultured for 7 days. 1×10⁵ of CAR-T cells were cultured with CD20-positive A549-CD20+ tumor cells, BCMA-positive A549-BCMA+ tumor cells, CD20 and BCMA double positive A549-CD20+BCMA+ tumor cells, double negative A549 tumor cells, or without tumor cells (NT), in 200 µl of medium for 18h with an E:T ratio of 1:1. Then the levels of IFN-γ secreted in the cell culture supernatant were detected by ELISA.

Expression levels of CD137 were assayed using the CAR-T cells cultured for 7 days. 1×10⁵ of CAR-T cells were cultured with CD20-positive A549-CD20+ tumor cells, BCMA-positive A549-BCMA+ tumor cells, CD20 and BCMA double positive A549-CD20+BCMA+ tumor cells, double negative A549 tumor cells, or without tumor cells, in 200 µl of medium for 18h with an E:T ratio of 1:1. Then the expression levels of CD137 on the surface of the CAR-T cells were detected by flow cytometry.

The IFNγ release results are shown in Figures 3A-3C and Figure 6B. After co-culturing the CAR-T cells with A549 cells expressing CD20 and/or BCMA antigens, anti-CD20 CAR-T (C-CAR066) cells can specifically recognize CD20 single-positive or CD20/BCMA double-positive target cells and release IFN-γ. Similarly, anti-BCMA CAR-T (C-CAR088) cells can specifically recognize BCMA single-positive or CD20/BCMA double-positive target cells to release IFN-γ. Only anti-CD20/BCMA CAR-T (TOB1-4 and TOBL1-4, where TOBL1 is C-CAR168) cells can recognize CD20 single-positive, BCMA single-positive and CD20/BCMA double-positive target cells, as well as release high levels of IFN-γ. TOB1 to TOB4 CAR-T cells showed high IFN-γ release when co-cultured with CD20 positive targets cells, but lower reactivity to BCMA single positive target cells. TOBL1 to TOBL4 CAR-T cells showed high IFN-γ release when co-cultured with CD20 positive targets and BCMA positive target cells. TOBL1 to TOBL4 CAR-T cells showed high IFN-γ release when co-cultured with target cells naturally expressing CD20 and BCMA.

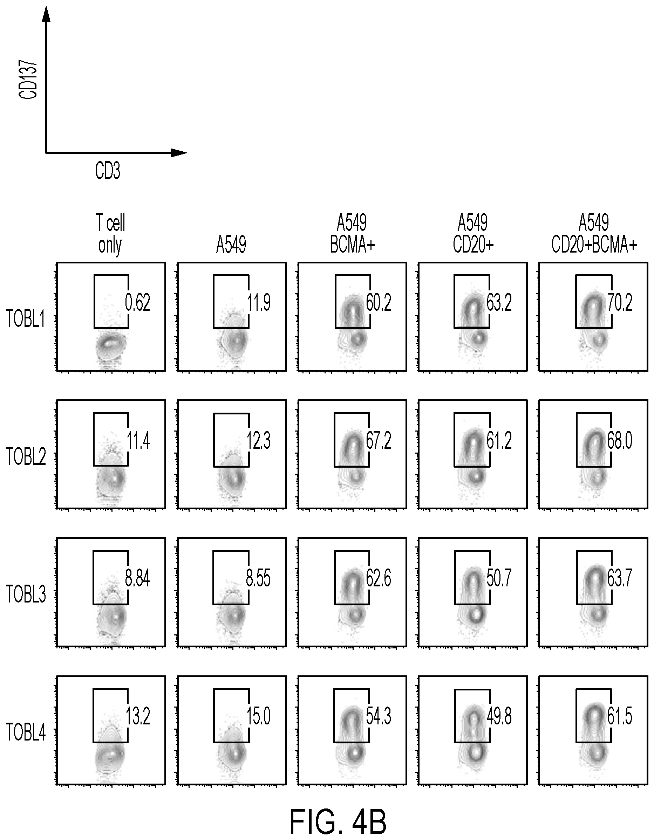

The flow cytometry results showed that the anti-CD20/BCMA CAR-T cells were activated by a variety of CD20/BCMA single-positive or double-positive cells and up-regulated the expression level of CD137 (Figures 4A and 4B).

### Example 3 Cytotoxicity of anti-CD20/BCMA CAR-T cells in vitro

The anti-CD20/BCMA CAR-T cells were co-cultured with target cells at E:T ratios of 0:1, 0.25:1, 0.5:1, 1:1, 2:1 and 4:1, respectively. Real-Time Cell Analysis (RTCA) label-free technology was used to evaluate the cytotoxicity of the CAR-T cells on target cells.

The results show that the anti-CD20/BCMA CAR-T cells effectively killed CD20/BCMA single-positive or double-positive tumor cells *in vitro* (A549-CD20+, A549-BCMA+, A549-BCMA+CD20+), while they had no effect on A549 cells which do not express CD20 or BCMA (Figures 5A-5B, Figure 6C). Their killing ability was comparable to the anti-CD20 and anti-BCMA monospecific CAR-T cells, with all being dose-dependent (Figures 5A-5B, Figure 6C). The TOBL1 to TOBL4 CAR-T cells (Figure 5B) showed high cytotoxicity to CD20-positivie and BCMA-positive target cells. The TOB1 to TOB4 CAR-T cells (Figure 5A) showed lower cytotoxicity to BCMA single positive target cells (compared to anti-BCMA CAR which is C-CAR088).

### Example 4 Cytotoxicity of anti-CD20/BCMA CAR-T cells on autoreactive B cells in vitro

Recent studies have shown that in patients with systemic lupus erythematosus (SLE), the proportion of CD11c^{hi}T-bet⁺ B cell subsets is significantly increased, and is closely related to the production of autoantibodies and the patient's clinical manifestations. Autoantibodies are characteristics of reactive B cells (see, Distinct Effector B Cells Induced by Unregulated Toll-like Receptor 7 Contribute to Pathogenic Responses in Systemic Lupus Erythematosus, Immunity, 2018, 16;49(4):725-739.e6. IL-21 drives expansion and plasma cell differentiation of autoreactive CD11chiT-bet+ B cells in SLE, Nat. Commun. 2018; 9(1):1758). This subset of cells is enriched with age in some animal models of autoimmune diseases and in the peripheral blood of patients with rheumatoid arthritis, so they are also called age-associated B cells (ABCs) (see, Toll-like receptor 7 (TLR7)-driven accumulation of a novel CD11c+ B-cell population is important for the development of autoimmunity. Blood, 2011;118(5):1305-15. A B-cell subset uniquely responsive to innate stimuli accumulates in aged mice, Blood, 2011; 118(5):1294-304).

TLR7 activation plays a role in the accumulation of autoreactive B cells and the production of autoantibodies in autoimmune diseases. One of the consequences of aberrant TLR7 activation is the accumulation of autoreactive B cells, or age-associated B cells (ABCs). ABCs are B cells that recognize self-antigens and have the potential to produce autoantibodies, which can target and damage the body's own tissues. Wang et al., Nature Communications, (2018) 9:1758.

In order to verify that the anti-CD20/BCMA CAR-T cells also have the ability to eliminate ABCs, we prepared C-CAR168 (TOBL1) CAR-T cells from the peripheral blood of three healthy human donors (HD10, HD11 and HD12). We also isolated autologous B cells from the PBMCs of heathy donors and induced their differentiation *in vitro* to obtain ABC-enriched autologous B cells which were then used as target cells to perform cytotoxicity experiments. After co-culture for 2 to 4 hours, C-CAR168 CAR-T cells derived from different donors showed apparent cytotoxicity effects on the ABC-enriched autologous B cells at different E:T ratios compared with control T cells without CAR transduction (Figures 7A-7C).

C-CAR168 can target both CD20+ B cells and BCMA+ plasma cells, which can provide superior duration of response in autoimmune diseases. The results show C-CAR168 CAR-T cells can eliminate ABC cells efficiently.

### In vitro ABC differentiation

PBMCs from healthy donors were isolated by gradient centrifugation using Ficoll and cryopreserved. On the day of ABC differentiation, pan B cells were first isolated from thawed PBMC by human B cell isolation kit (Miltenyi Biotec; negative selection, e.g., non-B cells were labeled and depleted) according to the manufacturer's instructions. B cells were then seeded in 96-well plates with 200µl RPMI complete medium and stimulated with TLR7 ligand R848, CD40L, BAFF, IL-2, Goat Anti-Human IgA + IgG + IgM (H+L), IL-21, and IFN-γ for 3 days. Cell medium was exchanged every day by replenishing with the complete medium and stimulation cocktail. The induction of ABCs was confirmed by FACS analysis. Antibodies for FACS staining included live/dead dye, anti-human CD19, CD38, CD27, IgD, CD11c, CD21, and T-bet.

### Cytotoxicity assay

After differentiation, the ABC-enriched B cells were cocultured with C-CAR168 or non-transduced (NT) T cells at the indicated E:T ratios. After 24 hours, cells were stained with the LIVE/DEAD Fixable Aqua Dead Cell Stain (Invitrogen) to determine their viability, along with anti-CD19 and anti-CD3 antibodies to distinguish B and T cells. Cytotoxicity was determined by the depletion of the percentage of viable CD19⁺ cells. The cytolysis of B cells was calculated by the following formula: Percentage of lysis (%) = (1 - (viable CD19⁺ cell fraction of the C-CAR168 coculture / viable CD19⁺ cell fraction of UT coculture)) × 100. See, Lin et al., Preclinical evaluation of CD8+ anti-BCMA mRNA CAR T-cells for treatment of multiple myeloma. Leukemia. 2021, 35(3): 752-763.

### Example 5 Inhibitory effect of anti-CD20/BCMA CAR-T cells on tumor cells in mice

### C-CAR168 effectively inhibited the growth of CD20 single positive and BCMA single positive tumor cells in tumor-bearing mice

The *in vivo* cytotoxicity effect of the anti-CD20/BCMA CAR-T cells on CD20 or BCMA single-positive cells was evaluated by mouse subcutaneous tumor model established using tumor cell lines expressing either CD20 (A549-CD20) or BCMA (MM.1S).

6-8 weeks female B-NDG mice were subcutaneously inoculated with A549-CD20 (CD20+) or MM.1S (BCMA+) cells. When the average tumor volume reached 100 mm³, C-CAR168 CAR-T cells were administered via the tail vein at the dosage of 3~5×10⁶ CAR-T cells/mouse. During the experiment, the tumor volume of the mice treated with the C-CAR168 CAR-T cells continued to decrease. At the end of the experiment, the tumor weight of the C-CAR168 groups was significantly lower than that of the vehicle control group. C-CAR168 cells showed strong cytotoxicity towards CD20-positive and BCMA-positive target cells *in vivo*.

Specifically, female B-NDG (NOD.Cg-Prkdc^{scid} Il2rg^{tm1Vst}/Vst) mice were subcutaneously inoculated with 5 ×10⁶ A549-CD20 cells/animal. When the average tumor volume reached about 100 mm³, 20 animals were selected and randomly divided into 2 groups (vehicle control group vs. C-CAR168 group), with 10 animals in each group. A single dose of a vehicle control or C-CAR168 (3×10⁶ CAR-T cells/animal) was administered to the mice by tail vein injection. After administration, the average tumor volume in the vehicle control group continued to increase, reaching 494.16 ± 31.5 mm³ on Day 42, with an average tumor weight of 0.254 ± 0.025 g. The average tumor volumes in the C-CAR168 group began to decrease from Day 10. By Day 42, the average tumor volumes were 10.02 ± 7.04 mm³ (Figure 9A, left panel), and the tumor weights were 0.013 ± 0.01 g, with significant differences compared to the vehicle control group (P<0.001) (Figure 9A, right panel). The tumor growth inhibition rates calculated based on tumor weight were 94.88%. The results show that C-CAR168 can significantly inhibit the growth of CD20-positive target cells *in vivo*.

To evaluate the *in vivo* effects of C-CAR168 on BCMA single positive target cells and compare *in vivo* efficacy of different batches of C-CAR168, 20 female B-NDG (NOD.CB17-Prkdc^{scid}Il2rg^{tm1}/Bcgen) mice were subcutaneously inoculated with 5 ×10⁶ MM.1S cells/animal. When the average tumor volume reached about 100 mm³, 15 animals were selected and randomly divided into 3 groups (a vehicle control group vs. two C-CAR168 groups), with 5 animals in each group. Each mouse was dosed once by tail vein injection. For C-CAR168, the dosage was 5×10⁶ CAR-T cells/animal. After administration, the average tumor volume in the vehicle control group continued to increase, reaching 2220.86 ± 117.35 mm³ on Day 28, with a tumor weight of 2.409 ± 0.216 g. The average tumor volumes in the C-CAR168-1 and C-CAR168-2 groups began to decrease from Day 10 (Figure 9B, left panel). By Day 28, the average tumor volumes were 109.2 ± 88.92 mm³ and 9.07 ± 5.58 mm³, respectively, and the tumor weights were 0.041 ± 0.034 g and 0.003 ± 0.002 g, respectively (Figure 9B, right panel), with significant differences compared to the vehicle control group, (P<0.001, P<0.001). The tumor growth inhibition rates calculated based on tumor weight were 98.30% and 99.88%, respectively. There was no significant difference between the two batches of C-CAR168. The results show that a single intravenous administration of 5×10⁶ C-CAR168 CAR-T cells/mouse was well tolerated in B-NDG tumor-bearing mice, and C-CAR168 can significantly inhibit the growth of BCMA-positive target cells *in vivo*.

### C-CAR168 effectively inhibited the growth of CD20 and BCMA double positive tumor cells in tumor-bearing mice

To evaluate the *in vivo* anti-tumor effects of C-CAR168, 65 female B-NDG (NOD.CB17-*Prkdc^{scid}Il2rg^{tml}* /Bcgen) mice were subcutaneous inoculated with 1×10⁶ K562-CD20-BCMA cells/animal. When the average tumor volume reached about 100mm³, 50 animals were selected and randomly divided into 5 groups: vehicle control group, T cell control group, C-CAR168 low-dose group (1×10⁶ CAR-T cells/mouse), medium-dose group (5×10⁶ CAR-T cells/mouse) and high-dose group (10×10⁶ CAR-T cells/mouse). The T cell control group were injected with non-transduced T cells from the same donor as C-CAR168, and the dose was consistent with the total T cell number in the C-CAR168 high-dose group. Each mouse was dosed once by tail vein injection.

During the experiment, the mean tumor volume of the animals in the vehicle control group and T cell control group continued to increase, and the mean tumor volume was 2628.78 ± 117.32 mm³ and 2536.23 ± 97.80 mm³, respectively at Day 17. The tumor volume in the C-CAR168 low-dose group continued to increase, although after 10 days of administration, the tumor volume was significantly lower than that in the vehicle control group and T cell control group. The average tumor volume of the C-CAR168 medium-dose group and high-dose group began to decline on Day 6. The C-CAR168 low-dose, medium-dose, and high-dose groups showed dose-dependent reductions in tumor, with tumor growth inhibition rates being 55.47%, 97.75%, and 98.01%, respectively on Day 17. No tumor tissues were observed in the C-CAR168 medium-dose and high-dose groups on Day 28 (Figure 9E). Figure 9F shows the survival rate curve of each group during the experimental period. Although all animals in the vehicle control group and T cell control group were dead around Day 17, all mice in the C-CAR168 medium-dose and high-dose groups were alive.

In summary, a single intravenous administration of 1×10⁶, 5×10⁶ or 10×10⁶ C-CAR168 CAR-T cells/mouse was well tolerated in B-NDG tumor bearing mice, and C-CAR168 significantly inhibited the growth of K562-CD20-BCMA tumor cells in a dose-dependent manner.

### Example 6 Antigen specificity of anti-CD20/BCMA CARs

In the membrane protein array, genetic engineering methods are used to construct the full-length cDNA sequences of human membrane proteins into expression vectors, which are then transiently transfected into HEK293T cells and arranged into an array by using microfluidic technology or chip printing technology. It is a high-throughput screening technology for studying the interaction between test substances and membrane proteins.

To examine the affinity and specificity of the anti-CD20/BCMA CARs, we used a membrane protein array assay to evaluate the risk of off-target binding between the antigen-binding domain of C-CAR168 and 5220 human cell membrane proteins.

A chimeric rabbit monoclonal antibody, C-CAR168 scFv-RabFc, was generated by linking the anti-CD20 scFv (e.g., derived from the Ofatumumab mAb) and the anti-BCMA scFv (e.g., derived from the BCMA-20 mAb) in frame with a rabbit IgG Fc region. The chimeric antibody was added at a concentration of 20 µg/mL to the HEK293T cell array transiently transfected with 5220 membrane proteins. Flow cytometry results show that C-CAR168 scFv-RabFc bound strongly to human CD20 and BCMA (Figure 8A). The average fluorescence intensity of its binding to CD20 and BCMA in flow cytometry was about 60-fold and 110-fold of that of the negative control group, respectively (Figure 8B). In addition to CD20 and BCMA, C-CAR168 scFv-RabFc showed specific binding to FCGR1A (Figure 8B), and the average fluorescence intensity was 2.5 times that of the negative control group. This is mainly due to the binding between FCGR1A and the rabbit-derived Fc of the recombinant protein; so there is no relevant risk in clinical applications. C-CAR168 scFv-RabFc showed weak binding to ITGB2-ITGAM and ITGB2-ITGAL heterodimers, and the average fluorescence intensity was 2 to 3 times that of the negative control group. For other proteins discovered in the preliminary screening (MPZ, F11R, CLEC2B and MC2R), the average fluorescence intensity binding to C-CAR168 scFv-RabFc did not change with concentration. At the concentrations of 20 µg/mL and 5 µg/mL, it did not exceed 2 times that of the negative control group, so the possibility of these proteins binding specifically to C-CAR168 scFv was low or minimal.

To test whether ITGB2-ITGAM and ITGB2-ITGAL heterodimers expressed on the cell membrane can be recognized by C-CAR168 CAR-T cells to activate downstream events, C-CAR168 was co-cultured with 293T cells transfected with ITGB2-ITGAM or ITGB2-ITGAL. Expression of CD137 on C-CAR168 CAR-T cells, as well the levels of IFN-γ, TNF-α, IL-2 and other cytokines in the cell culture supernatant, were essayed. 293T cells transfected with empty vector were used as negative control, and 293T cells transfected with CD20 and BCMA were used as positive control.

CD137 (4-1BB) is a cell surface marker for antigen-specific activation of T cells. The antigen-specific activation of CAR-T cells can be assessed by detecting the up-regulation of CD137 expression on the cell surface. The experiment found that after three batches of C-CAR168 cells were co-cultured with cells expressing CD20 and BCMA, the proportion of 4-1BB-positive cells increased compared with non-transduced T cells ("NT"). After co-culturing with those expressing ITGB2-ITGAM and ITGB2-ITGAL, the proportion of 4-1BB positive cells was not significantly different from that in the non-transduced T cell group ("NT") (Figure 8D, left panel), indicating that C-CAR168 does not bind specifically to ITGB2-ITGAM or ITGB2-ITGAL *in vitro*.

Cytokines in the cell culture supernatant were assayed, and the results showed that C-CAR168 CAR-T cells secreted high levels of IFN-γ when co-cultured with cells expressing CD20 or BCMA. When co-cultured with cells expressing ITGB2-ITGAM or ITGB2-ITGAL, compared with non-transduced T cells, the concentrations of IFN-γ in the supernatant did not increase significantly (Figure 8D, right panel). The results further showed that C-CAR168 did not specifically recognize ITGB2-ITGAM and ITGB2-ITGAL *in vitro*.

In summary, the membrane protein array and *in vitro* co-culture results show that the antigen-binding domain of C-CAR168 binds strongly to human CD20 and BCMA, and has no other non-specific binding sites. The membrane protein array study identified that C-CAR168 has no cross-reactivity against membrane proteome except weak binding to two heterocomplexes.

### Example 7 C-CAR168 shows robust potency against autologous B cells from SLE patients

To study CAR-T therapies for the treatment of autoimmune diseases, such as SLE, we evaluated the efficiency of the CAR-T cells to deplete autoreactive B cells. We will also study the efficacy of the CAR-T cells on remission and survival of a lupus model.

### Efficiency of C-CAR168 to eliminate pan B cells from lupus patients in vitro

10~15 mL of peripheral blood samples from eight patients with SLE were collected. The patients had different activity and autoantibody profile, displayed different organ damage (patients with lupus nephritis were preferable), and underwent different treatment, to represent the heterogenous nature of lupus patients. Patients who recently received B cell depleting antibodies were excluded.

For each sample, part of the blood was used to isolate T cells for CAR-T production, and the remaining blood was used to isolate pan B cells as target for a cytolytic assay. T cells isolated from eight SLE patients were transduced by lentiviral vectors encoding C-CAR168 and tested for CAR expression. T cell samples from 8 SLE patent samples were successfully transduced and expanded well for function assays (Figure 10A).

C-CAR168 CAR-T cells generated from 8 patient samples, or non-transduced (NT) T cells, were co-cultured with target cell lines expressing CD20 or/and BCMA. K562 is negative for both CD20 and BCMA; MM.1S is a multiple myeloma cell line which is BCMA-positive. After 24 hours, co-culture supernatants were collected for ELISA (enzyme-linked immunosorbent assay) to assess the IFN-γ levels. Result from one representative sample of 8 patients is shown in Figure 10B. Thus, C-CAR168 cells generated from SLE patient samples showed robust activity against target cells expressing CD20 and BCMA.

Isolated pan B cells isolated from 8 patient samples were co-cultured with autologous C-CAR168 CAR-T cells, or non-transduced (NT) T cells, at the indicated E:T (effector to target) ratios. After 24 hours, co-culture supernatants were collected for ELISA to assess the IFN-γ levels. Cytotoxicity was determined by fluorescence-activated cell sorting (FACS) and calculation of the depletion of the percentage of viable CD19+ pan B cells. The cytolysis of B cells was calculated by the following formula: Percentage of lysis (%) = (1 - (viable CD19+ cell fraction of the C-CAR168 coculture / viable CD19+ cell fraction of UT coculture)) × 100. Results from one representative sample of 8 patients are shown in Figures 10C and 10D. Pan B cells isolated from 8 SLE patient samples were recognized and lysed by autologous C-CAR168 cells. The results confirmed the efficiency of C-CAR168 CAR-T cells to deplete peripheral B cells from lupus patients in the *in vitro* setting.

### Efficiency of the CAR-T to eliminate ABCs from lupus patients in vitro

The efficiency of the CAR-T to eliminate ABCs, the essential subset of pathogenic B cells, from lupus patients *in vitro* will be studied.

Blood samples or PBMCs from lupus patients will be processed for ABCs differentiation and CAR-T production as well as functional analysis.

The study will confirm the efficiency of the CAR-T cells to deplete ABCs from lupus patients in the *in vitro* setting.

### Efficiency of the CAR-T to deplete B cells and the therapeutic efficacy in vivo

The efficiency of the CAR-T to deplete B cells and its therapeutic efficacy will be evaluated *in vivo* with a humanized mouse model of SLE. CD34⁺ stem cell humanized mice will be obtained. 2 or more mice will be sacrificed to collect spleens with aseptic technique. T cells will then be isolated from the spleens for CAR-T production. The remaining mice will be used to induce the onset of lupus disease, and upon successful induction, mice will be divided into groups to receive CAR-T or control treatment (for example, non-transduced T cells). Blood samples will be obtained from the mice periodically to monitor the persistence of CAR-T cells, as well as efficiency of B cell depletion (including ABCs) by FACS. The sera samples will be used to measure the titers of various autoantibodies. Urine samples will also be routinely collected to measure the levels of proteinuria. At the end of the study, or in case an animal dies early (presumably in control group), tissues will be collected for histology, for example, to examine the deposition of immune complex in the kidney and the severity of nephritis. The presence of B cells or plasma cells in diseased tissue will also be examined. Survival curves will be generated to compare the effect of CAR-T versus control treatment.

### References

Bhoj, V.G. et al. Persistence of long-lived plasma cells and humoral immunity in individuals responding to CD19-directed CAR T-cell therapy. Blood 128, 360-370 (2016).
Taubmann et al. Long term safety and efficacy of CAR-T cell treatment in refractory systemic lupus erythematosus - data from the first seven patients. Annals of the Rheumatic Diseases, OP0141, page 93 (2023).
Qin, C., Tian, DS., Zhou, LQ. et al. Anti-BCMA CAR T-cell therapy CT103A in relapsed or refractory AQP4-IgG seropositive neuromyelitis optica spectrum disorders: phase 1 trial interim results. Sig Transduct Target Ther 8, 5 (2023).
Tai et al. Role of B-cell-activating factor in adhesion and growth of human multiple myeloma cells in the bone marrow microenvironment. Cancer research. 2006;66(13):6675-82.
Pavlasova et al. (2020). The regulation and function of CD20: an "enigma" of B-cell biology and targeted therapy. Haematologica, 105(6), 1494-1506.
Krumbholz et al. B cells and antibodies in multiple sclerosis pathogenesis and therapy [J]. Nature reviews Neurology, 2012, 8(11): 613-23.
Parker et al., Single-Cell Analyses Identify Brain Mural Cells Expressing CD19 as Potential Off-Tumor Targets for CAR-T Immunotherapies. Cell. 2020 Oct 1;183(1):126-142.e17.
Qu et al. Phase 1 study of C-CAR088, a novel humanized anti-BCMA CAR T-cell therapy in relapsed/ refractory multiple myeloma. J Immunother Cancer. 2022 Sep;10(9):e005145.

The structures of the anti-CD20/BCMA CARs, TOB1-4 and TOBL1-4, are shown in Table 1.

**Table 1**

| **Anti-CD20/BCMA CAR** | **scFv V_{H}/V_{L} order** | **CAR structure** |
|---|---|---|
| TN-OF-B20-L1 (TOBL1, or C-CAR168) | OF(V_{L}-V_{H}) - B20(V_{L}-V_{H}) | SP - OF V_{L} - linker 1 - OF V_{H} - linker 2 - B20 V_{L} - linker 3 - B20V_{H} - CD8 hinge - CD8 TM - 41BB - CD3z |
| TN-OF-B20-L2 (TOBL2) | OF(V_{H}-V_{L}) - B20(V_{L}-V_{H}) | SP - OF V_{H} - linker 1 - OF V_{L} - linker 2 - B20 V_{L} - linker 3 - B20V_{H} - CD8 hinge - CD8 TM - 41BB - CD3z |
| TN-OF-B20-L3 (TOBL3) | OF(V_{H}-V_{L}) - B20(V_{H}-V_{L}) | SP - OF V_{H} - linker 1 - OF V_{L} - linker 2 - B20 V_{H} - linker 3 - B20V_{L} - CD8 hinge - CD8 TM - 41BB - CD3z |
| TN-OF-B20-L4 (TOBL4) | OF(V_{L}-V_{H}) - B20(V_{H}-V_{L}) | SP - OF V_{L} - linker 1 - OF V_{H} - linker 2 - B20 V_{H} - linker 3 - B20V_{L} - CD8 hinge - CD8 TM - 41BB - CD3z |
| TN-OF-B20-1 (TOB1) | OF(V_{L}-V_{H}) - B20(V_{H}-V_{L}) | SP - OF V_{L} - linker 1 - OF V_{H} - linker 2 - B20 V_{H} - linker 3 - B20V_{L} - IgG4 hinge - CD28 TM - 41BB - CD3z |
| TN-OF-B20-2 (TOB2) | OF(V_{L}-V_{H}) - B20(V_{L}-V_{H}) | SP - OF V_{L} - linker 1 - OF V_{H} - linker 2 - B20 V_{L} - linker 3 - B20V_{H} - IgG4 hinge - CD28 TM - 41BB - CD3z |
| TN-OF-B20-3 (TOB3) | OF(V_{H}-V_{L}) - B20(V_{L}-V_{H}) | SP - OF V_{H} - linker 1 - OF V_{L} - linker 2 - B20 V_{L} - linker 3 - B20V_{H} - IgG4 hinge - CD28 TM - 41BB - CD3z |
| TN-OF-B20-4 (TOB4) | OF(V_{H}-V_{L}) - B20(V_{H}-V_{L}) | SP - OF V_{H} - linker 1 - OF V_{L} - linker 2 - B20 V_{H} - linker 3 - B20V_{L} - IgG4 hinge - CD28 TM - 41BB - CD3z |

### Sequences:

**TN-OF-B20-L1 (TOBL1, or C-CAR168)**
   CD8a SP nucleic acid sequence (63nt)
   atggccttaccagtgaccgccttgctcctgccgctggccttgctgctccacgccgccaggccg (SEQ ID NO:1)
CD8a SP amino acid sequence:
   **MALPVTALLLPLALLLHAARP** (SEQ ID NO:2)
OF V_{L} nucleic acid sequence (321nt) 
GAAATTGTGTTGACACAGTCTCCAGCCACCCTGTCTTTGTCTCCAGGGGAAAGAGCC
ACCCTCTCCTGCAGGGCCAGTCAGAGTGTTAGCAGCTACTTAGCCTGGTACCAACAG
AAACCTGGCCAGGCTCCCAGGCTCCTCATCTATGATGCATCCAACAGGGCCACTGGC
ATCCCAGCCAGGTTCAGTGGCAGTGGGTCTGGGACAGACTTCACTCTCACCATCAGC
AGCCTAGAGCCTGAAGATTTTGCAGTTTATTACTGTCAGCAGCGTAGCAACTGGCCG
ATCACCTTCGGCCAAGGGACACGACTGGAGATTAAA (SEQ ID NO:3)
OF V_{L} amino acid sequence: 
EIVLTQSPATLSLSPGERATLSCRASQSVSSYLAWYQQKPGQAPRLLIYDASNRATGIPA
RFSGSGSGTDFTLTISSLEPEDFAVYYCQQRSNWPITFGQGTRLEIK (SEQ ID NO:4)
Linker-1 nucleic acid sequence (54nt)
   GGCAGTACTAGCGGTGGTGGCTCCGGGGGCGGTTCCGGTGGGGGCGGCAGCAGC (SEQ ID NO:5)
Linker-1 amino acid sequence:
   **GSTSGGGSGGGSGGGGSS** (SEQ ID NO:6)
OF V_{H} nucleic acid sequence (366nt) 
GAAGTGCAGCTGGTGGAGTCTGGGGGAGGCTTGGTACAGCCTGGCAGGTCCCTGAG
ACTCTCCTGTGCAGCCTCTGGATTCACCTTTAATGATTATGCCATGCACTGGGTCCGG
CAAGCTCCAGGGAAGGGCCTGGAGTGGGTCTCAACTATTAGTTGGAATAGTGGTTCC
ATAGGCTATGCGGACTCTGTGAAGGGCCGATTCACCATCTCCAGAGACAACGCCAA
GAAGTCCCTGTATCTGCAAATGAACAGTCTGAGAGCTGAGGACACGGCCTTGTATTA
CTGTGCAAAAGATATACAGTACGGCAACTACTACTACGGTATGGACGTCTGGGGCC
AAGGGACCACGGTCACCGTCTCCTCA (SEQ ID NO:7)
OF V_{H} amino acid sequence: 
EVQLVESGGGLVQPGRSLRLSCAASGFTFNDYAMHWVRQAPGKGLEWVSTISWNSGS
IGYADSVKGRFTISRDNAKKSLYLQMNSLRAEDTALYYCAKDIQYGNYYYGMDVWG
QGTTVTVSS (SEQ ID NO:8)
Linker-2 nucleic acid sequence (15nt)
   GGAGGTGGTGGATCC (SEQ ID NO:9)
Linker-2 amino acid sequence:
   **GGGGS** (SEQ ID NO:10)
B20 V_{L} nucleic acid sequence (321nt) 
Gacatccagatgacccagtcccctcctccctgtccgcctccgtgggcgaccgggtgaccatcacctgccgggcctcccagggcatctcc
aactacctgaactggtaccagcagaagcccggcaaggcccccaagcccctgatctactacacctccaacctgcagtccggcgtgccctcc
cggttctccggctccggctccggcaccgactacaccctgaccatctcctccctgcagcccgaggacttcgccacctactactgcatgggcc
agaccatctcctcctacaccttcggccagggcaccaagctggagatcaag (SEQ ID NO:11)
B20 V_{L} amino acid sequence: 
DIQMTQSPSSLSASVGDRVTITCRASQGISNYLNWYQQKPGKAPKPLIYYTSNLQSG
VPSRFSGSGSGTDYTLTISSLQPEDFATYYCMGQTISSYTFGQGTKLEIK (SEQ ID
NO:12)
Linker-3 nucleic acid sequence (45 nt)
   Ggtggcggtggctcgggcggtggtgggtcgggtggcggcggatct (SEQ ID NO:13)
Linker-3 amino acid sequence:
   **GGGGSGGGGSGGGGS** (SEQ ID NO:14)
B20 V_{H} nucleic acid sequence (363nt) 
Gaggtgcagctggtggagtccggcggcggcctggtgcagcccggcggctccctgcggctgtcctgcgccgcctccggcttcaccttctc
caacttcgacatggcctgggtgcggcaggcccccggcaagggcctggtgtgggtgtcctccatcaccaccggcgccgaccacgccatct
 
acgccgactccgtgaagggccggttcaccatctcccgggacaacgccaagaacaccctgtacctgcagatgaactccctgcgggccgag
gacaccgccgtgtactactgcgtgcggcacggctactacgacggctaccacctgttcgactactggggccagggcaccctggtgaccgtg
tcctcc (SEQ ID NO:15)
B20 V_{H} amino acid sequence: 
EVQLVESGGGLVQPGGSLRLSCAASGFTFSNFDMAWVRQAPGKGLVWVSSITTGA
DHAIYADSVKGRFTISRDNAKNTLYLQMNSLRAEDTAVYYCVRHGYYDGYHLFDY
WGQGTLVTVSS (SEQ ID NO:16)
CD8a hinge nucleic acid sequence (165nt) 
Ttcgtgccggtcttcctgccagcgaagcccaccacgacgccagcgccgcgaccaccaacaccggcgcccaccatcgcgtcgcagccc
ctgtccctgcgcccagaggcgtgccggccagcggcggggggcgcagtgcacacgagggggctggacttcgcctgtgat (SEQ ID
NO:17)
CD8a hinge amino acid sequence:
   **FVPVFLPAKPTTTPAPRPPTPAPTIASQPLSLRPEACRPAAGGAVHTRGLDFACD** (SEQ ID NO:18)
CD8a TM nucleic acid sequence (72nt)
   Atctacatctgggcgcccttggccgggacttgtggggtccttctcctgtcactggttatcaccctttactgc (SEQ ID NO:19)
CD8a TM amino acid sequence:
   **IYIWAPLAGTCGVLLLSLVITLYC** (SEQ ID NO:20)
4-1BB nucleic acid sequence (126nt) 
Aaacggggcagaaagaaactcctgtatatattcaaacaaccatttatgagaccagtacaaactactcaagaggaagatggctgtagctgcc
gatttccagaagaagaagaaggaggatgtgaactg (SEQ ID NO:21)
4-1BB amino acid sequence:
   **KRGRKKLLYIFKQPFMRPVQTTQEEDGCSCRFPEEEEGGCEL** (SEQ ID NO:22)
CD3z nucleic acid sequence (336nt) 
Agagtgaagttcagcaggagcgcagacgcccccgcgtaccagcagggccagaaccagctctataacgagctcaatctaggacgaaga
gaggagtacgatgttttggacaagagacgtggccgggaccctgagatggggggaaagccgagaaggaagaaccctcaggaaggcctg
tacaatgaactgcagaaagataagatggcggaggcctacagtgagattgggatgaaaggcgagcgccggaggggcaaggggcacgat
ggcctttaccagggtctcagtacagccaccaaggacacctacgacgcccttcacatgcaggccctgccccctcgctaa (SEQ ID
NO:23)
CD3z amino acid sequence: 
RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQ
ALPPR (SEQ ID NO:24)
**TOBL1** nucleic acid sequence (2247nt) 
atggccttaccagtgaccgccttgctcctgccgctggccttgctgctccacgccgccaggccgGAAATTGTGTTGACACA
GTCTCCAGCCACCCTGTCTTTGTCTCCAGGGGAAAGAGCCACCCTCTCCTGCAGGGC
CAGTCAGAGTGTTAGCAGCTACTTAGCCTGGTACCAACAGAAACCTGGCCAGGCTC
CCAGGCTCCTCATCTATGATGCATCCAACAGGGCCACTGGCATCCCAGCCAGGTTCA
GTGGCAGTGGGTCTGGGACAGACTTCACTCTCACCATCAGCAGCCTAGAGCCTGAA
GATTTTGCAGTTTATTACTGTCAGCAGCGTAGCAACTGGCCGATCACCTTCGGCCAA
GGGACACGACTGGAGATTAAAGGCAGTACTAGCGGTGGTGGCTCCGGGGGCGGTTC
CGGTGGGGGCGGCAGCAGCGAAGTGCAGCTGGTGGAGTCTGGGGGAGGCTTGGTAC
AGCCTGGCAGGTCCCTGAGACTCTCCTGTGCAGCCTCTGGATTCACCTTTAATGATT
ATGCCATGCACTGGGTCCGGCAAGCTCCAGGGAAGGGCCTGGAGTGGGTCTCAACT
ATTAGTTGGAATAGTGGTTCCATAGGCTATGCGGACTCTGTGAAGGGCCGATTCACC
ATCTCCAGAGACAACGCCAAGAAGTCCCTGTATCTGCAAATGAACAGTCTGAGAGC
TGAGGACACGGCCTTGTATTACTGTGCAAAAGATATACAGTACGGCAACTACTACTA
CGGTATGGACGTCTGGGGCCAAGGGACCACGGTCACCGTCTCCTCAGGAGGTGGTG
GATCCGacatccagatgacccagtcccctcctccctgtccgcctccgtgggcgaccgggtgaccatcacctgccgggcctcccag
ggcatctccaactacctgaactggtaccagcagaagcccggcaaggcccccaagcccctgatctactacacctccaacctgcagtccggc
gtgccctcccggttctccggctccggctccggcaccgactacaccctgaccatctcctccctgcagcccgaggacttcgccacctactactg
catgggccagaccatctcctcctacaccttcggccagggcaccaagctggagatcaagGgtggcggtggctcgggcggtggtgggtcg
 
ggtggcggcggatctgaggtgcagctggtggagtccggcggcggcctggtgcagcccggcggctccctgcggctgtcctgcgccgcct
ccggcttcaccttctccaacttcgacatggcctgggtgcggcaggcccccggcaagggcctggtgtgggtgtcctccatcaccaccggcg
ccgaccacgccatctacgccgactccgtgaagggccggttcaccatctcccgggacaacgccaagaacaccctgtacctgcagatgaact
ccctgcgggccgaggacaccgccgtgtactactgcgtgcggcacggctactacgacggctaccacctgttcgactactggggccagggc
accctggtgaccgtgtcctccttcgtgccggtcttcctgccagcgaagcccaccacgacgccagcgccgcgaccaccaacaccggcgcc
caccatcgcgtcgcagcccctgtccctgcgcccagaggcgtgccggccagcggcgggggcgcagtgcacacgagggggctggactt
cgcctgtgatatctacatctgggcgcccttggccgggacttgtggggtccttctcctgtcactggttatcaccctttactgcaaacggggcaga
aagaaactcctgtatatattcaaacaaccatttatgagaccagtacaaactactcaagaggaagatggctgtagctgccgatttccagaagaa
gaagaaggaggatgtgaactgagagtgaagttcagcaggagcgcagacgcccccgcgtaccagcagggccagaaccagctctataac
gagctcaatctaggacgaagagaggagtacgatgttttggacaagagacgtggccgggaccctgagatggggggaaagccgagaagg
aagaaccctcaggaaggcctgtacaatgaactgcagaaagataagatggcggaggcctacagtgagattgggatgaaaggcgagcgcc
ggaggggcaaggggcacgatggcctttaccagggtctcagtacagccaccaaggacacctacgacgcccttcacatgcaggccctgccc
cctcgctaa (SEQ ID NO:25)
**TOBL1** amino acid sequence: 
MALPVTALLLPLALLLHAARPEIVLTQSPATLSLSPGERATLSCRASQSVSSYLAWY
QQKPGQAPRLLIYDASNRATGIPARFSGSGSGTDFTLTISSLEPEDFAVYYCQQRSN
WPITFGQGTRLEIKGSTSGGGSGGGSGGGGSSEVQLVESGGGLVQPGRSLRLSCAA
SGFTFNDYAMHWVRQAPGKGLEWVSTISWNSGSIGYADSVKGRFTISRDNAKKSL
YLQMNSLRAEDTALYYCAKDIQYGNYYYGMDVWGQGTTVTVSSGGGGSDIQMTQ
SPSSLSASVGDRVTITCRASQGISNYLNWYQQKPGKAPKPLIYYTSNLQSGVPSRFSG
SGSGTDYTLTISSLQPEDFATYYCMGQTISSYTFGQGTKLEIKGGGGSGGGGSGGG
GSEVQLVESGGGLVQPGGSLRLSCAASGFTFSNFDMAWVRQAPGKGLVWVSSITT
GADHAIYADSVKGRFTISRDNAKNTLYLQMNSLRAEDTAVYYCVRHGYYDGYHLF
DYWGQGTLVTVSSFVPVFLPAKPTTTPAPRPPTPAPTIASQPLSLRPEACRPAAGGA
VHTRGLDFACDIYIWAPLAGTCGVLLLSLVITLYCKRGRKKLLYIFKQPFMRPVQT
TQEEDGCSCRFPEEEEGGCELRVKFSRSADAPAYQQGQNQLYNELNLGRREEYDV
LDKRRGRDPEMGGKPRRKNPQEGLYNELQKDKMAEAYSEIGMKGERRRGKGHD
GLYQGLSTATKDTYDALHMQALPPR (SEQ ID NO:26)
***TN-OF-B20-L2 (TOBL2)***
CD8a SP nucleic acid sequence (63nt)
   Atggccttaccagtgaccgccttgctcctgccgctggccttgctgctccacgccgccaggccg (SEQ ID NO:27)
OF V_{H} nucleic acid sequence 
GAAGTGCAGCTGGTGGAGTCTGGGGGAGGCTTGGTACAGCCTGGCAGGTCCCTGAG
ACTCTCCTGTGCAGCCTCTGGATTCACCTTTAATGATTATGCCATGCACTGGGTCCGG
CAAGCTCCAGGGAAGGGCCTGGAGTGGGTCTCAACTATTAGTTGGAATAGTGGTTCC
ATAGGCTATGCGGACTCTGTGAAGGGCCGATTCACCATCTCCAGAGACAACGCCAA
GAAGTCCCTGTATCTGCAAATGAACAGTCTGAGAGCTGAGGACACGGCCTTGTATTA
CTGTGCAAAAGATATACAGTACGGCAACTACTACTACGGTATGGACGTCTGGGGCC
AAGGGACCACGGTCACCGTCTCCTCA (SEQ ID NO:28)
Linker-1 nucleic acid sequence
   GGCAGTACTAGCGGTGGTGGCTCCGGGGGCGGTTCCGGTGGGGGCGGCAGCAGC (SEQ ID NO:29)
OF V_{L} nucleic acid sequence 
GAAATTGTGTTGACACAGTCTCCAGCCACCCTGTCTTTGTCTCCAGGGGAAAGAGCC
ACCCTCTCCTGCAGGGCCAGTCAGAGTGTTAGCAGCTACTTAGCCTGGTACCAACAG
AAACCTGGCCAGGCTCCCAGGCTCCTCATCTATGATGCATCCAACAGGGCCACTGGC
ATCCCAGCCAGGTTCAGTGGCAGTGGGTCTGGGACAGACTTCACTCTCACCATCAGC
AGCCTAGAGCCTGAAGATTTTGCAGTTTATTACTGTCAGCAGCGTAGCAACTGGCCG
ATCACCTTCGGCCAAGGGACACGACTGGAGATTAAA (SEQ ID NO:30)
Linker-2 nucleic acid sequence
   GGAGGTGGTGGATCC (SEQ ID NO:31)
BCMA-20 scFv (729nt):
B20 V_{L} nucleic acid sequence (321nt) 
gacatccagatgacccagtcccccctcctccctgtccgcctccgtgggcgaccgggtgaccatcacctgccgggcctcccagggcatctcc
aactacctgaactggtaccagcagaagcccggcaaggcccccaagcccctgatctactacacctccaacctgcagtccggcgtgccctcc
 
cggttctccggctccggctccggcaccgactacaccctgaccatctcctccctgcagcccgaggacttcgccacctactactgcatgggcc
agaccatctcctcctacaccttcggccagggcaccaagctggagatcaag (SEQ ID NO:32)
Linker-3 nucleic acid sequence (45 nt)
   ggtggcggtggctcgggcggtggtgggtcgggtggcggcggatct (SEQ ID NO:33)
B20 V_{H} nucleic acid sequence (363nt) 
gaggtgcagctggtggagtccggcggcggcctggtgcagcccggcggctccctgcggctgtcctgcgccgcctccggcttcaccttctcc
aacttcgacatggcctgggtgcggcaggcccccggcaagggcctggtgtgggtgtcctccatcaccaccggcgccgaccacgccatcta
cgccgactccgtgaagggccggttcaccatctcccgggacaacgccaagaacaccctgtacctgcagatgaactccctgcgggccgagg
acaccgccgtgtactactgcgtgcggcacggctactacgacggctaccacctgttcgactactggggccagggcaccctggtgaccgtgt
cctcc (SEQ ID NO:34)
CD8a hinge nucleic acid sequence (165nt) 
Ttcgtgccggtcttcctgccagcgaagcccaccacgacgccagcgccgcgaccaccaacaccggcgcccaccatcgcgtcgcagccc
ctgtccctgcgcccagaggcgtgccggccagcggcgggggcgcagtgcacacgagggggctggacttcgcctgtgat (SEQ ID
NO:35)
CD8a TM nucleic acid sequence (72nt)
   Atctacatctgggcgcccttggccgggacttgtggggtccttctcctgtcactggttatcaccctttactgc (SEQ ID NO:36)
4-1BB nucleic acid sequence (126nt) 
Aaacggggcagaaagaaactcctgtatatattcaaacaaccatttatgagaccagtacaaactactcaagaggaagatggctgtagctgcc
gatttccagaagaagaagaaggaggatgtgaactg (SEQ ID NO:37)
CD3z nucleic acid sequence (336nt) 
Agagtgaagttcagcaggagcgcagacgcccccgcgtaccagcagggccagaaccagctctataacgagctcaatctaggacgaaga
gaggagtacgatgttttggacaagagacgtggccgggaccctgagatggggggaaagccgagaaggaagaaccctcaggaaggcctg
tacaatgaactgcagaaagataagatggcggaggcctacagtgagattgggatgaaaggcgagcgccggaggggcaaggggcacgat
ggcctttaccagggtctcagtacagccaccaaggacacctacgacgcccttcacatgcaggccctgccccctcgctaa (SEQ ID
NO:38)
***TOBL2*** nucleic acid sequence (2247nt) 
atggccttaccagtgaccgccttgctcctgccgctggccttgctgctccacgccgccaggccgGAAGTGCAGCTGGTGGA
GTCTGGGGGAGGCTTGGTACAGCCTGGCAGGTCCCTGAGACTCTCCTGTGCAGCCTC
TGGATTCACCTTTAATGATTATGCCATGCACTGGGTCCGGCAAGCTCCAGGGAAGGG
CCTGGAGTGGGTCTCAACTATTAGTTGGAATAGTGGTTCCATAGGCTATGCGGACTC
TGTGAAGGGCCGATTCACCATCTCCAGAGACAACGCCAAGAAGTCCCTGTATCTGC
AAATGAACAGTCTGAGAGCTGAGGACACGGCCTTGTATTACTGTGCAAAAGATATA
CAGTACGGCAACTACTACTACGGTATGGACGTCTGGGGCCAAGGGACCACGGTCAC
CGTCTCCTCAGGCAGTACTAGCGGTGGTGGCTCCGGGGGCGGTTCCGGTGGGGGCG
GCAGCAGCGAAATTGTGTTGACACAGTCTCCAGCCACCCTGTCTTTGTCTCCAGGGG
AAAGAGCCACCCTCTCCTGCAGGGCCAGTCAGAGTGTTAGCAGCTACTTAGCCTGGT
ACCAACAGAAACCTGGCCAGGCTCCCAGGCTCCTCATCTATGATGCATCCAACAGG
GCCACTGGCATCCCAGCCAGGTTCAGTGGCAGTGGGTCTGGGACAGACTTCACTCTC
ACCATCAGCAGCCTAGAGCCTGAAGATTTTGCAGTTTATTACTGTCAGCAGCGTAGC
AACTGGCCGATCACCTTCGGCCAAGGGACACGACTGGAGATTAAAGGAGGTGGTGG
ATCCGacatccagatgacccagtccccctcctccctgtccgcctccgtgggcgaccgggtgaccatcacctgccgggcctcccaggg
catctccaactacctgaactggtaccagcagaagcccggcaaggcccccaagcccctgatctactacacctccaacctgcagtccggcgt
gccctcccggttctccggctccggctccggcaccgactacaccctgaccatctcctccctgcagcccgaggacttcgccacctactactgc
atgggccagaccatctcctcctacaccttcggccagggcaccaagctggagatcaagGgtggcggtggctcgggcggtggtgggtcgg
gtggcggcggatctgaggtgcagctggtggagtccggcggcggcctggtgcagcccggcggctccctgcggctgtcctgcgccgcctc
cggcttcaccttctccaacttcgacatggcctgggtgcggcaggcccccggcaagggcctggtgtgggtgtcctccatcaccaccggcgc
cgaccacgccatctacgccgactccgtgaagggccggttcaccatctcccgggacaacgccaagaacaccctgtacctgcagatgaactc
cctgcgggccgaggacaccgccgtgtactactgcgtgcggcacggctactacgacggctaccacctgttcgactactggggccagggca
ccctggtgaccgtgtcctccttcgtgccggtcttcctgccagcgaagcccaccacgacgccagcgccgcgaccaccaacaccggcgccc
accatcgcgtcgcagcccctgtccctgcgcccagaggcgtgccggccagcggcgggggggcgcagtgcacacgagggggctggacttc
gcctgtgatatctacatctgggcgcccttggccgggacttgtggggtccttctcctgtcactggttatcaccctttactgcaaacggggcagaa
agaaactcctgtatatattcaaacaaccatttatgagaccagtacaaactactcaagaggaagatggctgtagctgccgatttccagaagaag
aagaaggaggatgtgaactgAgagtgaagttcagcaggagcgcagacgcccccgcgtaccagcagggccagaaccagctctataacg
agctcaatctaggacgaagagaggagtacgatgttttggacaagagacgtggccgggaccctgagatggggggaaagccgagaaggaa
gaaccctcaggaaggcctgtacaatgaactgcagaaagataagatggcggaggcctacagtgagattgggatgaaaggcgagcgccgg
 
agggggcaaggggcacgatggcctttaccagggtctcagtacagccaccaaggacacctacgacgcccttcacatgcaggccctgccccc
tcgctaa (SEQ ID NO:39)
***TOBL2*** amino acid sequence: 
MALPVTALLLPLALLLHAARPEVQLVESGGGLVQPGRSLRLSCAASGFTFNDYAM
HWVRQAPGKGLEWVSTISWNSGSIGYADSVKGRFTISRDNAKKSLYLQMNSLRAE
DTALYYCAKDIQYGNYYYGMDVWGQGTTVTVSSGSTSGGGSGGGSGGGGSSEIVL
TQSPATLSLSPGERATLSCRASQSVSSYLAWYQQKPGQAPRLLIYDASNRATGIPAR
FSGSGSGTDFTLTISSLEPEDFAVYYCQQRSNWPITFGQGTRLEIKGGGGSDIQMTQ
SPSSLSASVGDRVTITCRASQGISNYLNWYQQKPGKAPKPLIYYTSNLQSGVPSRFSG
SGSGTDYTLTISSLQPEDFATYYCMGQTISSYTFGQGTKLEIKGGGGSGGGGSGGG
GSEVQLVESGGGLVQPGGSLRLSCAASGFTFSNFDMAWVRQAPGKGLVWVSSITT
GADHAIYADSVKGRFTISRDNAKNTLYLQMNSLRAEDTAVYYCVRHGYYDGYHLF
DYWGQGTLVTVSSFVPVFLPAKPTTTPAPRPPTPAPTIASQPLSLRPEACRPAAGGA
VHTRGLDFACDIYIWAPLAGTCGVLLLSLVITLYCKRGRKKLLYIFKQPFMRPVQT
TQEEDGCSCRFPEEEEGGCELRVKFSRSADAPAYQQGQNQLYNELNLGRREEYDV
LDKRRGRDPEMGGKPRRKNPQEGLYNELQKDKMAEAYSEIGMKGERRRGKGHD
GLYQGLSTATKDTYDALHMQALPPR (SEQ ID NO:40)
***TN-OF-B20-L3 (TOBL3)***
CD8a SP (63nt) nucleic acid sequence
   Atggccttaccagtgaccgccttgctcctgccgctggccttgctgctccacgccgccaggccg (SEQ ID NO:41)
OF V_{H} nucleic acid sequence 
GAAGTGCAGCTGGTGGAGTCTGGGGGAGGCTTGGTACAGCCTGGCAGGTCCCTGAG
ACTCTCCTGTGCAGCCTCTGGATTCACCTTTAATGATTATGCCATGCACTGGGTCCGG
CAAGCTCCAGGGAAGGGCCTGGAGTGGGTCTCAACTATTAGTTGGAATAGTGGTTCC
ATAGGCTATGCGGACTCTGTGAAGGGCCGATTCACCATCTCCAGAGACAACGCCAA
GAAGTCCCTGTATCTGCAAATGAACAGTCTGAGAGCTGAGGACACGGCCTTGTATTA
CTGTGCAAAAGATATACAGTACGGCAACTACTACTACGGTATGGACGTCTGGGGCC
AAGGGACCACGGTCACCGTCTCCTCA (SEQ ID NO:42)
Linker-1 nucleic acid sequence
   GGCAGTACTAGCGGTGGTGGCTCCGGGGGCGGTTCCGGTGGGGGCGGCAGCAGC (SEQ ID NO:43)
OF V_{L} nucleic acid sequence

GAAATTGTGTTGACACAGTCTCCAGCCACCCTGTCTTTGTCTCCAGGGGAAAGAGCC
ACCCTCTCCTGCAGGGCCAGTCAGAGTGTTAGCAGCTACTTAGCCTGGTACCAACAG
AAACCTGGCCAGGCTCCCAGGCTCCTCATCTATGATGCATCCAACAGGGCCACTGGC
ATCCCAGCCAGGTTCAGTGGCAGTGGGTCTGGGACAGACTTCACTCTCACCATCAGC
AGCCTAGAGCCTGAAGATTTTGCAGTTTATTACTGTCAGCAGCGTAGCAACTGGCCG
ATCACCTTCGGCCAAGGGACACGACTGGAGATTAAA (SEQ ID NO:44)

Linker-2 nucleic acid sequence
   GGAGGTGGTGGATCC (SEQ ID NO:45)
BCMA-20 scFv (729nt):
B20 V_{H} nucleic acid sequence (363nt) 
Gaggtgcagctggtggagtccggcggcggcctggtgcagcccggcggctccctgcggctgtcctgcgccgcctccggcttcaccttctc
caacttcgacatggcctgggtgcggcaggcccccggcaagggcctggtgtgggtgtcctccatcaccaccggcgccgaccacgccatct
acgccgactccgtgaagggccggttcaccatctcccgggacaacgccaagaacaccctgtacctgcagatgaactccctgcgggccgag
gacaccgccgtgtactactgcgtgcggcacggctactacgacggctaccacctgttcgactactggggccagggcaccctggtgaccgtg
tcctcc (SEQ ID NO:46)
Linker-3 nucleic acid sequence (45 nt)
   Ggtggcggtggctcgggcggtggtgggtcgggtggcggcggatct (SEQ ID NO:47)
B20 V_{L} nucleic acid sequence (321nt) 
Gacatccagatgacccagtccccctcctccctgtccgcctccgtgggcgaccgggtgaccatcacctgccgggcctcccagggcatctcc
aactacctgaactggtaccagcagaagcccggcaaggcccccaagcccctgatctactacacctccaacctgcagtccggcgtgccctcc
cggttctccggctccggctccggcaccgactacaccctgaccatctcctccctgcagcccgaggacttcgccacctactactgcatgggcc
 agaccatctcctcctacaccttcggccagggcaccaagctggagatcaag (SEQ ID NO:48)
CD8a hinge nucleic acid sequence (165nt) 
Ttcgtgccggtcttcctgccagcgaagcccaccacgacgccagcgccgcgaccaccaacaccggcgcccaccatcgcgtcgcagccc
ctgtccctgcgcccagaggcgtgccggccagcggcgggggcgcagtgcacacgagggggctggacttcgcctgtgat (SEQ ID
NO:49)
CD8a TM nucleic acid sequence (72nt)
   Atctacatctgggcgcccttggccgggacttgtggggtccttctcctgtcactggttatcaccctttactgc (SEQ ID NO:50)
4-1BB nucleic acid sequence (126nt) 
Aaacggggcagaaagaaactcctgtatatattcaaacaaccatttatgagaccagtacaaactactcaagaggaagatggctgtagctgcc
gatttccagaagaagaagaaggaggatgtgaactg (SEQ ID NO:51)
CD3z nucleic acid sequence (336nt) 
Agagtgaagttcagcaggagcgcagacgcccccgcgtaccagcagggccagaaccagctctataacgagctcaatctaggacgaaga
gaggagtacgatgttttggacaagagacgtggccgggaccctgagatggggggaaagccgagaaggaagaaccctcaggaaggcctg
tacaatgaactgcagaaagataagatggcggaggcctacagtgagattgggatgaaaggcgagcgccggaggggcaaggggcacgat
ggcctttaccagggtctcagtacagccaccaaggacacctacgacgcccttcacatgcaggccctgccccctcgctaa (SEQ ID
NO:52)
***TOBL3*** nucleic acid sequence 
atggccttaccagtgaccgccttgctcctgccgctggccttgctgctccacgccgccaggccgGAAGTGCAGCTGGTGGA
GTCTGGGGGAGGCTTGGTACAGCCTGGCAGGTCCCTGAGACTCTCCTGTGCAGCCTC
TGGATTCACCTTTAATGATTATGCCATGCACTGGGTCCGGCAAGCTCCAGGGAAGGG
CCTGGAGTGGGTCTCAACTATTAGTTGGAATAGTGGTTCCATAGGCTATGCGGACTC
TGTGAAGGGCCGATTCACCATCTCCAGAGACAACGCCAAGAAGTCCCTGTATCTGC
AAATGAACAGTCTGAGAGCTGAGGACACGGCCTTGTATTACTGTGCAAAAGATATA
CAGTACGGCAACTACTACTACGGTATGGACGTCTGGGGCCAAGGGACCACGGTCAC
CGTCTCCTCAGGCAGTACTAGCGGTGGTGGCTCCGGGGGCGGTTCCGGTGGGGGCG
GCAGCAGCGAAATTGTGTTGACACAGTCTCCAGCCACCCTGTCTTTGTCTCCAGGGG
 
AAAGAGCCACCCTCTCCTGCAGGGCCAGTCAGAGTGTTAGCAGCTACTTAGCCTGGT
ACCAACAGAAACCTGGCCAGGCTCCCAGGCTCCTCATCTATGATGCATCCAACAGG
GCCACTGGCATCCCAGCCAGGTTCAGTGGCAGTGGGTCTGGGACAGACTTCACTCTC
ACCATCAGCAGCCTAGAGCCTGAAGATTTTGCAGTTTATTACTGTCAGCAGCGTAGC
AACTGGCCGATCACCTTCGGCCAAGGGACACGACTGGAGATTAAAGGAGGTGGTGG
ATCCgaggtgcagctggtggagtccggcggcggcctggtgcagcccggcggctccctgcggctgtcctgcgccgcctccggcttca
ccttctccaacttcgacatggcctgggtgcggcaggcccccggcaagggcctggtgtgggtgtcctccatcaccaccggcgccgaccac
gccatctacgccgactccgtgaagggccggttcaccatctcccgggacaacgccaagaacaccctgtacctgcagatgaactccctgcgg
gccgaggacaccgccgtgtactactgcgtgcggcacggctactacgacggctaccacctgttcgactactggggccagggcaccctggt
gaccgtgtcctccGgtggcggtggctcgggcggtggtgggtcgggtggcggcggatctGacatccagatgacccagtccccctcctcc
ctgtccgcctccgtgggcgaccgggtgaccatcacctgccgggcctcccagggcatctccaactacctgaactggtaccagcagaagccc
ggcaaggcccccaagcccctgatctactacacctccaacctgcagtccggcgtgccctcccggttctccggctccggctccggcaccgac
tacaccctgaccatctcctccctgcagcccgaggacttcgccacctactactgcatgggccagaccatctcctcctacaccttcggccaggg
caccaagctggagatcaagttcgtgccggtcttcctgccagcgaagcccaccacgacgccagcgccgcgaccaccaacaccggcgccc
accatcgcgtcgcagcccctgtccctgcgcccagaggcgtgccggccagcggcggggggcgcagtgcacacgagggggctggacttc
gcctgtgatatctacatctgggcgcccttggccgggacttgtggggtccttctcctgtcactggttatcaccctttactgcaaacggggcagaa
agaaactcctgtatatattcaaacaaccatttatgagaccagtacaaactactcaagaggaagatggctgtagctgccgatttccagaagaag
aagaaggaggatgtgaactgAgagtgaagttcagcaggagcgcagacgcccccgcgtaccagcagggccagaaccagctctataacg
agctcaatctaggacgaagagaggagtacgatgttttggacaagagacgtggccgggaccctgagatggggggaaagccgagaaggaa
gaaccctcaggaaggcctgtacaatgaactgcagaaagataagatggcggaggcctacagtgagattgggatgaaaggcgagcgccgg
aggggcaaggggcacgatggcctttaccagggtctcagtacagccaccaaggacacctacgacgcccttcacatgcaggccctgccccc
tcgctaa (SEQ ID NO:53)
***TOBL3*** amino acid sequence: 
MALPVTALLLPLALLLHAARPEVQLVESGGGLVQPGRSLRLSCAASGFTFNDYAM
HWVRQAPGKGLEWVSTISWNSGSIGYADSVKGRFTISRDNAKKSLYLQMNSLRAE
DTALYYCAKDIQYGNYYYGMDVWGQGTTVTVSSGSTSGGGSGGGSGGGGSSEIVL
TQSPATLSLSPGERATLSCRASQSVSSYLAWYQQKPGQAPRLLIYDASNRATGIPAR
FSGSGSGTDFTLTISSLEPEDFAVYYCQQRSNWPITFGQGTRLEIKGGGGSEVQLVE
SGGGLVQPGGSLRLSCAASGFTFSNFDMAWVRQAPGKGLVWVSSITTGADHAIYA
DSVKGRFTISRDNAKNTLYLQMNSLRAEDTAVYYCVRHGYYDGYHLFDYWGQGT
 
LVTVSSGGGGSGGGGSGGGGSDIQMTQSPSSLSASVGDRVTITCRASQGISNYLNW
YQQKPGKAPKPLIYYTSNLQSGVPSRFSGSGSGTDYTLTISSLQPEDFATYYCMGQT
ISSYTFGQGTKLEIKFVPVFLPAKPTTTPAPRPPTPAPTIASQPLSLRPEACRPAAGG
AVHTRGLDFACDIYIWAPLAGTCGVLLLSLVITLYCKRGRKKLLYIFKQPFMRPVQ
TTQEEDGCSCRFPEEEEGGCELRVKFSRSADAPAYQQGQNQLYNELNLGRREEYD
VLDKRRGRDPEMGGKPRRKNPQEGLYNELQKDKMAEAYSEIGMKGERRRGKGH
DGLYQGLSTATKDTYDALHMQALPPR (SEQ ID NO:54)
***TN-OF-B20-L4 (TOBL4)***
CD8a SP nucleic acid sequence (63nt)
   Atggccttaccagtgaccgccttgctcctgccgctggccttgctgctccacgccgccaggccg (SEQ ID NO:55)
OF V_{L} nucleic acid sequence 
GAAATTGTGTTGACACAGTCTCCAGCCACCCTGTCTTTGTCTCCAGGGGAAAGAGCC
ACCCTCTCCTGCAGGGCCAGTCAGAGTGTTAGCAGCTACTTAGCCTGGTACCAACAG
AAACCTGGCCAGGCTCCCAGGCTCCTCATCTATGATGCATCCAACAGGGCCACTGGC
ATCCCAGCCAGGTTCAGTGGCAGTGGGTCTGGGACAGACTTCACTCTCACCATCAGC
AGCCTAGAGCCTGAAGATTTTGCAGTTTATTACTGTCAGCAGCGTAGCAACTGGCCG
ATCACCTTCGGCCAAGGGACACGACTGGAGATTAAA (SEQ ID NO:56)
Linker-1 nucleic acid sequence
   GGCAGTACTAGCGGTGGTGGCTCCGGGGGCGGTTCCGGTGGGGGCGGCAGCAGC (SEQ ID NO:57)
OF V_{H} nucleic acid sequence 
GAAGTGCAGCTGGTGGAGTCTGGGGGAGGCTTGGTACAGCCTGGCAGGTCCCTGAG
ACTCTCCTGTGCAGCCTCTGGATTCACCTTTAATGATTATGCCATGCACTGGGTCCGG
CAAGCTCCAGGGAAGGGCCTGGAGTGGGTCTCAACTATTAGTTGGAATAGTGGTTCC
ATAGGCTATGCGGACTCTGTGAAGGGCCGATTCACCATCTCCAGAGACAACGCCAA
GAAGTCCCTGTATCTGCAAATGAACAGTCTGAGAGCTGAGGACACGGCCTTGTATTA
CTGTGCAAAAGATATACAGTACGGCAACTACTACTACGGTATGGACGTCTGGGGCC
 AAGGGACCACGGTCACCGTCTCCTCA (SEQ ID NO:58)
Linker-2 nucleic acid sequence
   GGAGGTGGTGGATCC (SEQ ID NO:59)
BCMA-20 scFv (729nt):
B20 V_{H} nucleic acid sequence (363nt) 
Gaggtgcagctggtggagtccggcggcggcctggtgcagcccggcggctccctgcggctgtcctgcgccgcctccggcttcaccttctc
caacttcgacatggcctgggtgcggcaggcccccggcaagggcctggtgtgggtgtcctccatcaccaccggcgccgaccacgccatct
acgccgactccgtgaagggccggttcaccatctcccgggacaacgccaagaacaccctgtacctgcagatgaactccctgcgggccgag
gacaccgccgtgtactactgcgtgcggcacggctactacgacggctaccacctgttcgactactggggccagggcaccctggtgaccgtg
tcctcc (SEQ ID NO:60)
Linker-3 nucleic acid sequence (45 nt)
   Ggtggcggtggctcgggcggtggtgggtcgggtggcggcggatct (SEQ ID NO:61)
B20 V_{L} nucleic acid sequence (321nt) 
Gacatccagatgacccagtcccctcctccctgtccgcctccgtgggcgaccgggtgaccatcacctgccgggcctcccagggcatctcc
aactacctgaactggtaccagcagaagcccggcaaggcccccaagcccctgatctactacacctccaacctgcagtccggcgtgccctcc
cggttctccggctccggctccggcaccgactacaccctgaccatctcctccctgcagcccgaggacttcgccacctactactgcatgggcc
agaccatctcctcctacaccttcggccagggcaccaagctggagatcaag (SEQ ID NO:62)
CD8a hinge nucleic acid sequence (165nt) 
Ttcgtgccggtcttcctgccagcgaagcccaccacgacgccagcgccgcgaccaccaacaccggcgcccaccatcgcgtcgcagccc
ctgtccctgcgcccagaggcgtgccggccagcggcgggggcgcagtgcacacgagggggctggacttcgcctgtgat (SEQ ID
NO:63)
CD8a TM nucleic acid sequence (72nt)
   Atctacatctgggcgcccttggccgggacttgtggggtccttctcctgtcactggttatcaccctttactgc (SEQ ID NO:64)
4-1BB nucleic acid sequence (126nt) 
Aaacggggcagaaagaaactcctgtatatattcaaacaaccatttatgagaccagtacaaactactcaagaggaagatggctgtagctgcc
gatttccagaagaagaagaaggaggatgtgaactg (SEQ ID NO:65)
CD3z nucleic acid sequence (336nt) 
Agagtgaagttcagcaggagcgcagacgcccccgcgtaccagcagggccagaaccagctctataacgagctcaatctaggacgaaga
gaggagtacgatgttttggacaagagacgtggccgggaccctgagatggggggaaagccgagaaggaagaaccctcaggaaggcctg
tacaatgaactgcagaaagataagatggcggaggcctacagtgagattgggatgaaaggcgagcgccggaggggcaaggggcacgat
ggcctttaccagggtctcagtacagccaccaaggacacctacgacgcccttcacatgcaggccctgccccctcgctaa (SEQ ID
NO:66)
**TOBL4** nucleic acid sequence (2247nt) 
atggccttaccagtgaccgccttgctcctgccgctggccttgctgctccacgccgccaggccgGAAATTGTGTTGACACA
GTCTCCAGCCACCCTGTCTTTGTCTCCAGGGGAAAGAGCCACCCTCTCCTGCAGGGC
CAGTCAGAGTGTTAGCAGCTACTTAGCCTGGTACCAACAGAAACCTGGCCAGGCTC
CCAGGCTCCTCATCTATGATGCATCCAACAGGGCCACTGGCATCCCAGCCAGGTTCA
GTGGCAGTGGGTCTGGGACAGACTTCACTCTCACCATCAGCAGCCTAGAGCCTGAA
GATTTTGCAGTTTATTACTGTCAGCAGCGTAGCAACTGGCCGATCACCTTCGGCCAA
GGGACACGACTGGAGATTAAAGGCAGTACTAGCGGTGGTGGCTCCGGGGGCGGTTC
CGGTGGGGGCGGCAGCAGCGAAGTGCAGCTGGTGGAGTCTGGGGGAGGCTTGGTAC
AGCCTGGCAGGTCCCTGAGACTCTCCTGTGCAGCCTCTGGATTCACCTTTAATGATT
ATGCCATGCACTGGGTCCGGCAAGCTCCAGGGAAGGGCCTGGAGTGGGTCTCAACT
ATTAGTTGGAATAGTGGTTCCATAGGCTATGCGGACTCTGTGAAGGGCCGATTCACC
ATCTCCAGAGACAACGCCAAGAAGTCCCTGTATCTGCAAATGAACAGTCTGAGAGC
TGAGGACACGGCCTTGTATTACTGTGCAAAAGATATACAGTACGGCAACTACTACTA
CGGTATGGACGTCTGGGGCCAAGGGACCACGGTCACCGTCTCCTCAGGAGGTGGTG
GATCCgaggtgcagctggtggagtccggcggcggcctggtgcagcccggcggctccctgcggctgtcctgcgccgcctccggcttc
accttctccaacttcgacatggcctgggtgcggcaggcccccggcaagggcctggtgtgggtgtcctccatcaccaccggcgccgaccac
gccatctacgccgactccgtgaagggccggttcaccatctcccgggacaacgccaagaacaccctgtacctgcagatgaactccctgcgg
gccgaggacaccgccgtgtactactgcgtgcggcacggctactacgacggctaccacctgttcgactactggggccagggcaccctggt
gaccgtgtcctccGgtggcggtggctcgggcggtggtgggtcgggtggcggcggatctGacatccagatgacccagtcccccctcctcc
ctgtccgcctccgtgggcgaccgggtgaccatcacctgccgggcctcccagggcatctccaactacctgaactggtaccagcagaagccc
 
ggcaaggcccccaagcccctgatctactacacctccaacctgcagtccggcgtgccctcccggttctccggctccggctccggcaccgac
tacaccctgaccatctcctccctgcagcccgaggacttcgccacctactactgcatgggccagaccatctcctcctacaccttcggccaggg
caccaagctggagatcaagttcgtgccggtcttcctgccagcgaagcccaccacgacgccagcgccgcgaccaccaacaccggcgccc
accatcgcgtcgcagcccctgtccctgcgcccagaggcgtgccggccagcggcggggggcgcagtgcacacgagggggctggacttc
gcctgtgatatctacatctgggcgcccttggccgggacttgtggggtccttctcctgtcactggttatcaccctttactgcaaacggggcagaa
agaaactcctgtatatattcaaacaaccatttatgagaccagtacaaactactcaagaggaagatggctgtagctgccgatttccagaagaag
aagaaggaggatgtgaactgAgagtgaagttcagcaggagcgcagacgcccccgcgtaccagcagggccagaaccagctctataacg
agctcaatctaggacgaagagaggagtacgatgttttggacaagagacgtggccgggaccctgagatggggggaaagccgagaaggaa
gaaccctcaggaaggcctgtacaatgaactgcagaaagataagatggcggaggcctacagtgagattgggatgaaaggcgagcgccgg
aggggcaaggggcacgatggcctttaccagggtctcagtacagccaccaaggacacctacgacgcccttcacatgcaggccctgccccc
tcgctaa (SEQ ID NO:67)
**TOBL4** amino acid sequence: 
MALPVTALLLPLALLLHAARPEIVLTQSPATLSLSPGERATLSCRASQSVSSYLAWY
QQKPGQAPRLLIYDASNRATGIPARFSGSGSGTDFTLTISSLEPEDFAVYYCQQRSN
WPITFGQGTRLEIKGSTSGGGSGGGSGGGGSSEVQLVESGGGLVQPGRSLRLSCAA
SGFTFNDYAMHWVRQAPGKGLEWVSTISWNSGSIGYADSVKGRFTISRDNAKKSL
YLQMNSLRAEDTALYYCAKDIQYGNYYYGMDVWGQGTTVTVSSGGGGSEVQLVE
SGGGLVQPGGSLRLSCAASGFTFSNFDMAWVRQAPGKGLVWVSSITTGADHAIYA
DSVKGRFTISRDNAKNTLYLQMNSLRAEDTAVYYCVRHGYYDGYHLFDYWGQGT
LVTVSSGGGGSGGGGSGGGGSDIQMTQSPSSLSASVGDRVTITCRASQGISNYLNW
YQQKPGKAPKPLIYYTSNLQSGVPSRFSGSGSGTDYTLTISSLQPEDFATYYCMGQT
ISSYTFGQGTKLEIKFVPVFLPAKPTTTPAPRPPTPAPTIASQPLSLRPEACRPAAGG
AVHTRGLDFACDIYIWAPLAGTCGVLLLSLVITLYCKRGRKKLLYIFKQPFMRPVQ
TTQEEDGCSCRFPEEEEGGCELRVKFSRSADAPAYQQGQNQLYNELNLGRREEYD
VLDKRRGRDPEMGGKPRRKNPQEGLYNELQKDKMAEAYSEIGMKGERRRGKGH
DGLYQGLSTATKDTYDALHMQALPPR (SEQ ID NO:68)
***TN-OF-B20-1(TOB1)***
CD8a SP nucleic acid sequence ATGGCCTTACCAGTGACCGCCTTGCTCCTGCCGCTGGCCTTGCTGCTCCACGCCGCC AGGCCG (SEQ ID NO:69)
OF V_{L} nucleic acid sequence 
GAAATTGTGTTGACACAGTCTCCAGCCACCCTGTCTTTGTCTCCAGGGGAAAGAGCC
ACCCTCTCCTGCAGGGCCAGTCAGAGTGTTAGCAGCTACTTAGCCTGGTACCAACAG
AAACCTGGCCAGGCTCCCAGGCTCCTCATCTATGATGCATCCAACAGGGCCACTGGC
ATCCCAGCCAGGTTCAGTGGCAGTGGGTCTGGGACAGACTTCACTCTCACCATCAGC
AGCCTAGAGCCTGAAGATTTTGCAGTTTATTACTGTCAGCAGCGTAGCAACTGGCCG
ATCACCTTCGGCCAAGGGACACGACTGGAGATTAAA (SEQ ID NO:70)
Linker-1 nucleic acid sequence
   GGCAGTACTAGCGGTGGTGGCTCCGGGGGCGGTTCCGGTGGGGGCGGCAGCAGC (SEQ ID NO:71)
OF V_{H} nucleic acid sequence 
GAAGTGCAGCTGGTGGAGTCTGGGGGAGGCTTGGTACAGCCTGGCAGGTCCCTGAG
ACTCTCCTGTGCAGCCTCTGGATTCACCTTTAATGATTATGCCATGCACTGGGTCCGG
CAAGCTCCAGGGAAGGGCCTGGAGTGGGTCTCAACTATTAGTTGGAATAGTGGTTCC
ATAGGCTATGCGGACTCTGTGAAGGGCCGATTCACCATCTCCAGAGACAACGCCAA
GAAGTCCCTGTATCTGCAAATGAACAGTCTGAGAGCTGAGGACACGGCCTTGTATTA
CTGTGCAAAAGATATACAGTACGGCAACTACTACTACGGTATGGACGTCTGGGGCC
AAGGGACCACGGTCACCGTCTCCTCA (SEQ ID NO:72)
Linker-2 nucleic acid sequence
   GGAGGTGGTGGATCC (SEQ ID NO:73)
B20 V_{H} nucleic acid sequence 
Gaggtgcagctggtggagtccggcggcggcctggtgcagcccggcggctccctgcggctgtcctgcgccgcctccggcttcaccttctc
caacttcgacatggcctgggtgcggcaggcccccggcaagggcctggtgtgggtgtcctccatcaccaccggcgccgaccacgccatct
acgccgactccgtgaagggccggttcaccatctcccgggacaacgccaagaacaccctgtacctgcagatgaactccctgcgggccgag
gacaccgccgtgtactactgcgtgcggcacggctactacgacggctaccacctgttcgactactggggccagggcaccctggtgaccgtg
 tcctcc (SEQ ID NO:74)
Linker-3 nucleic acid sequence
   ggtggcggtggctcgggcggtggtgggtcgggtggcggcggatct (SEQ ID NO:75)
B20 V_{L} nucleic acid sequence 
Gacatccagatgacccagtcccctcctccctgtccgcctccgtgggcgaccgggtgaccatcacctgccgggcctcccagggcatctcc
aactacctgaactggtaccagcagaagcccggcaaggcccccaagcccctgatctactacacctccaacctgcagtccggcgtgccctcc
cggttctccggctccggctccggcaccgactacaccctgaccatctcctccctgcagcccgaggacttcgccacctactactgcatgggcc
agaccatctcctcctacaccttcggccagggcaccaagctggagatcaag (SEQ ID NO:76)
IgG4 hinge nucleic acid sequence (36nt)
   GAGAGCAAGTACGGACCGCCCTGCCCCCCTTGCCCT (SEQ ID NO:77)
IgG4 hinge amino acid sequence:
   **ESKYGPPCPPCP** (SEQ ID NO:78)
CD28 TM nucleic acid sequence (84nt) 
ATGTTCTGGGTGCTGGTGGTGGTCGGAGGCGTGCTGGCCTGCTACAGCCTGCTGGTC
ACCGTGGCCTTCATCATCTTTTGGGTG (SEQ ID NO:79)
CD28 TM amino acid sequence:
   **MFWVLVVVGGVLACYSLLVTVAFIIFWV** (SEQ ID NO:80)
4-1BB nucleic acid sequence 
AAACGGGGCAGAAAGAAACTCCTGTATATATTCAAACAACCATTTATGAGACCAGT
ACAAACTACTCAAGAGGAAGATGGCTGTAGCTGCCGATTTCCAGAAGAAGAAGAAG
GAGGATGTGAACTG (SEQ ID NO:81)
CD3z nucleic acid sequence CGGGTGAAGTTCAGCAGAAGCGCCGACGCCCCTGCCTACCAGCAGGGCCAGAATCA 
GCTGTACAACGAGCTGAACCTGGGCAGAAGGGAAGAGTACGACGTCCTGGATAAGC
GGAGAGGCCGGGACCCTGAGATGGGCGGCAAGCCTCGGCGGAAGAACCCCCAGGA
AGGCCTGTATAACGAACTGCAGAAAGACAAGATGGCCGAGGCCTACAGCGAGATCG
GCATGAAGGGCGAGCGGAGGCGGGGCAAGGGCCACGACGGCCTGTATCAGGGCCT
GTCCACCGCCACCAAGGATACCTACGACGCCCTGCACATGCAGGCCCTGCCCCCAA
GG (SEQ ID NO:82)
***TOB1*** nucleic acid sequence (2130nt) 
ATGGCCTTACCAGTGACCGCCTTGCTCCTGCCGCTGGCCTTGCTGCTCCACGCCGCC
AGGCCGGAAATTGTGTTGACACAGTCTCCAGCCACCCTGTCTTTGTCTCCAGGGGAA
AGAGCCACCCTCTCCTGCAGGGCCAGTCAGAGTGTTAGCAGCTACTTAGCCTGGTAC
CAACAGAAACCTGGCCAGGCTCCCAGGCTCCTCATCTATGATGCATCCAACAGGGC
CACTGGCATCCCAGCCAGGTTCAGTGGCAGTGGGTCTGGGACAGACTTCACTCTCAC
CATCAGCAGCCTAGAGCCTGAAGATTTTGCAGTTTATTACTGTCAGCAGCGTAGCAA
CTGGCCGATCACCTTCGGCCAAGGGACACGACTGGAGATTAAAGGCAGTACTAGCG
GTGGTGGCTCCGGGGGCGGTTCCGGTGGGGGCGGCAGCAGCGAAGTGCAGCTGGTG
GAGTCTGGGGGAGGCTTGGTACAGCCTGGCAGGTCCCTGAGACTCTCCTGTGCAGCC
TCTGGATTCACCTTTAATGATTATGCCATGCACTGGGTCCGGCAAGCTCCAGGGAAG
GGCCTGGAGTGGGTCTCAACTATTAGTTGGAATAGTGGTTCCATAGGCTATGCGGAC
TCTGTGAAGGGCCGATTCACCATCTCCAGAGACAACGCCAAGAAGTCCCTGTATCTG
CAAATGAACAGTCTGAGAGCTGAGGACACGGCCTTGTATTACTGTGCAAAAGATAT
ACAGTACGGCAACTACTACTACGGTATGGACGTCTGGGGCCAAGGGACCACGGTCA
CCGTCTCCTCAGGAGGTGGTGGATCCgaggtgcagctggtggagtccggcggcggcctggtgcagcccggcgg
ctccctgcggctgtcctgcgccgcctccggcttcaccttctccaacttcgacatggcctgggtgcggcaggcccccggcaagggcctggtg
tgggtgtcctccatcaccaccggcgccgaccacgccatctacgccgactccgtgaagggccggttcaccatctcccgggacaacgccaa
gaacaccctgtacctgcagatgaactccctgcgggccgaggacaccgccgtgtactactgcgtgcggcacggctactacgacggctacc
acctgttcgactactggggccagggcaccctggtgaccgtgtcctccggtggcggtggctcgggcggtggtgggtcgggtggcggcgga
tctgacatccagatgacccagtccccctcctccctgtccgcctccgtgggcgaccgggtgaccatcacctgccgggcctcccagggcatct
ccaactacctgaactggtaccagcagaagcccggcaaggcccccaagcccctgatctactacacctccaacctgcagtccggcgtgccct
cccggttctccggctccggctccggcaccgactacaccctgaccatctcctccctgcagcccgaggacttcgccacctactactgcatggg
ccagaccatctcctcctacaccttcggccagggcaccaagctggagatcaagGAGAGCAAGTACGGACCGCCCTG
 
CCCCCCTTGCCCTATGTTCTGGGTGCTGGTGGTGGTCGGAGGCGTGCTGGCCTGCTA
CAGCCTGCTGGTCACCGTGGCCTTCATCATCTTTTGGGTGAAACGGGGCAGAAAGAA
ACTCCTGTATATATTCAAACAACCATTTATGAGACCAGTACAAACTACTCAAGAGGA
AGATGGCTGTAGCTGCCGATTTCCAGAAGAAGAAGGAGGATGTGAACTGCGGG
TGAAGTTCAGCAGAAGCGCCGACGCCCCTGCCTACCAGCAGGGCCAGAATCAGCTG
TACAACGAGCTGAACCTGGGCAGAAGGGAAGAGTACGACGTCCTGGATAAGCGGA
GAGGCCGGGACCCTGAGATGGGCGGCAAGCCTCGGCGGAAGAACCCCCAGGAAGG
CCTGTATAACGAACTGCAGAAAGACAAGATGGCCGAGGCCTACAGCGAGATCGGCA
TGAAGGGCGAGCGGAGGCGGGGCAAGGGCCACGACGGCCTGTATCAGGGCCTGTCC
ACCGCCACCAAGGATACCTACGACGCCCTGCACATGCAGGCCCTGCCCCCAAGGTA
A (SEQ ID NO:83)
***TOB1*** amino acid sequence: 
MALPVTALLLPLALLLHAARPEIVLTQSPATLSLSPGERATLSCRASQSVSSYLAWY
QQKPGQAPRLLIYDASNRATGIPARFSGSGSGTDFTLTISSLEPEDFAVYYCQQRSN
WPITFGQGTRLEIKGSTSGGGSGGGSGGGGSSEVQLVESGGGLVQPGRSLRLSCAA
SGFTFNDYAMHWVRQAPGKGLEWVSTISWNSGSIGYADSVKGRFTISRDNAKKSL
YLQMNSLRAEDTALYYCAKDIQYGNYYYGMDVWGQGTTVTVSSGGGGSEVQLVE
SGGGLVQPGGSLRLSCAASGFTFSNFDMAWVRQAPGKGLVWVSSITTGADHAIYA
DSVKGRFTISRDNAKNTLYLQMNSLRAEDTAVYYCVRHGYYDGYHLFDYWGQGT
LVTVSSGGGGSGGGGSGGGGSDIQMTQSPSSLSASVGDRVTITCRASQGISNYLNW
YQQKPGKAPKPLIYYTSNLQSGVPSRFSGSGSGTDYTLTISSLQPEDFATYYCMGQT
ISSYTFGQGTKLEIKESKYGPPCPPCPMFWVLVVVGGVLACYSLLVTVAFIIFWVKR
GRKKLLYIFKQPFMRPVQTTQEEDGCSCRFPEEEEGGCELRVKFSRSADAPAYQQ
GQNQLYNELNLGRREEYDVLDKRRGRDPEMGGKPRRKNPQEGLYNELQKDKMA
EAYSEIGMKGERRRGKGHDGLYQGLSTATKDTYDALHMQALPPR (SEQ ID NO:84)
***TN-OF-B20-2 (TOB2)***
CD8a SP nucleic acid sequence 
ATGGCCTTACCAGTGACCGCCTTGCTCCTGCCGCTGGCCTTGCTGCTCCACGCCGCC
AGGCCG (SEQ ID NO:85)
OF V_{L} nucleic acid sequence 
GAAATTGTGTTGACACAGTCTCCAGCCACCCTGTCTTTGTCTCCAGGGGAAAGAGCC
ACCCTCTCCTGCAGGGCCAGTCAGAGTGTTAGCAGCTACTTAGCCTGGTACCAACAG
AAACCTGGCCAGGCTCCCAGGCTCCTCATCTATGATGCATCCAACAGGGCCACTGGC
ATCCCAGCCAGGTTCAGTGGCAGTGGGTCTGGGACAGACTTCACTCTCACCATCAGC
AGCCTAGAGCCTGAAGATTTTGCAGTTTATTACTGTCAGCAGCGTAGCAACTGGCCG
ATCACCTTCGGCCAAGGGACACGACTGGAGATTAAA (SEQ ID NO:86)
Linker-1 nucleic acid sequence
   GGCAGTACTAGCGGTGGTGGCTCCGGGGGCGGTTCCGGTGGGGGCGGCAGCAGC (SEQ ID NO:87)
OF V_{H} nucleic acid sequence 
GAAGTGCAGCTGGTGGAGTCTGGGGGAGGCTTGGTACAGCCTGGCAGGTCCCTGAG
ACTCTCCTGTGCAGCCTCTGGATTCACCTTTAATGATTATGCCATGCACTGGGTCCGG
CAAGCTCCAGGGAAGGGCCTGGAGTGGGTCTCAACTATTAGTTGGAATAGTGGTTCC
ATAGGCTATGCGGACTCTGTGAAGGGCCGATTCACCATCTCCAGAGACAACGCCAA
GAAGTCCCTGTATCTGCAAATGAACAGTCTGAGAGCTGAGGACACGGCCTTGTATTA
CTGTGCAAAAGATATACAGTACGGCAACTACTACTACGGTATGGACGTCTGGGGCC
AAGGGACCACGGTCACCGTCTCCTCA (SEQ ID NO:88)
Linker-2 nucleic acid sequence
   GGAGGTGGTGGATCC (SEQ ID NO:89)
B20 V_{L} nucleic acid sequence 
Gacatccagatgacccagtcccccctcctccctgtccgcctccgtgggcgaccgggtgaccatcacctgccgggcctcccagggcatctcc
aactacctgaactggtaccagcagaagcccggcaaggcccccaagcccctgatctactacacctccaacctgcagtccggcgtgccctcc
cggttctccggctccggctccggcaccgactacaccctgaccatctcctccctgcagcccgaggacttcgccacctactactgcatgggcc
agaccatctcctcctacaccttcggccagggcaccaagctggagatcaag (SEQ ID NO:90)
Linker-3 nucleic acid sequence
   ggtggcggtggctcgggcggtggtgggtcgggtggcggcggatct (SEQ ID NO:91)
B20 V_{H} nucleic acid sequence 
Gaggtgcagctggtggagtccggcggcggcctggtgcagcccggcggctccctgcggctgtcctgcgccgcctccggcttcaccttctc
caacttcgacatggcctgggtgcggcaggcccccggcaagggcctggtgtgggtgtcctccatcaccaccggcgccgaccacgccatct
acgccgactccgtgaagggccggttcaccatctcccgggacaacgccaagaacaccctgtacctgcagatgaactccctgcgggccgag
gacaccgccgtgtactactgcgtgcggcacggctactacgacggctaccacctgttcgactactggggccagggcaccctggtgaccgtg
tcctcc (SEQ ID NO:92)
hinge nucleic acid sequence
   GAGAGCAAGTACGGACCGCCCTGCCCCCCTTGCCCT (SEQ ID NO:93)
CD28 TM nucleic acid sequence 
ATGTTCTGGGTGCTGGTGGTGGTCGGAGGCGTGCTGGCCTGCTACAGCCTGCTGGTC
ACCGTGGCCTTCATCATCTTTTGGGTG (SEQ ID NO:94)
4-1BB nucleic acid sequence 
AAACGGGGCAGAAAGAAACTCCTGTATATATTCAAACAACCATTTATGAGACCAGT
ACAAACTACTCAAGAGGAAGATGGCTGTAGCTGCCGATTTCCAGAAGAAGAAG
GAGGATGTGAACTG (SEQ ID NO:95)
CD3z nucleic acid sequence 
CGGGTGAAGTTCAGCAGAAGCGCCGACGCCCCTGCCTACCAGCAGGGCCAGAATCA
GCTGTACAACGAGCTGAACCTGGGCAGAAGGGAAGAGTACGACGTCCTGGATAAGC
GGAGAGGCCGGGACCCTGAGATGGGCGGCAAGCCTCGGCGGAAGAACCCCCAGGA
AGGCCTGTATAACGAACTGCAGAAAGACAAGATGGCCGAGGCCTACAGCGAGATCG
GCATGAAGGGCGAGCGGAGGCGGGGCAAGGGCCACGACGGCCTGTATCAGGGCCT
GTCCACCGCCACCAAGGATACCTACGACGCCCTGCACATGCAGGCCCTGCCCCCAA
GG (SEQ ID NO:96)
**TOB2** nucleic acid sequence (2130nt) 
ATGGCCTTACCAGTGACCGCCTTGCTCCTGCCGCTGGCCTTGCTGCTCCACGCCGCC
AGGCCGGAAATTGTGTTGACACAGTCTCCAGCCACCCTGTCTTTGTCTCCAGGGGAA
AGAGCCACCCTCTCCTGCAGGGCCAGTCAGAGTGTTAGCAGCTACTTAGCCTGGTAC
CAACAGAAACCTGGCCAGGCTCCCAGGCTCCTCATCTATGATGCATCCAACAGGGC
CACTGGCATCCCAGCCAGGTTCAGTGGCAGTGGGTCTGGGACAGACTTCACTCTCAC
CATCAGCAGCCTAGAGCCTGAAGATTTTGCAGTTTATTACTGTCAGCAGCGTAGCAA
CTGGCCGATCACCTTCGGCCAAGGGACACGACTGGAGATTAAAGGCAGTACTAGCG
GTGGTGGCTCCGGGGGCGGTTCCGGTGGGGGCGGCAGCAGCGAAGTGCAGCTGGTG
GAGTCTGGGGGAGGCTTGGTACAGCCTGGCAGGTCCCTGAGACTCTCCTGTGCAGCC
TCTGGATTCACCTTTAATGATTATGCCATGCACTGGGTCCGGCAAGCTCCAGGGAAG
GGCCTGGAGTGGGTCTCAACTATTAGTTGGAATAGTGGTTCCATAGGCTATGCGGAC
TCTGTGAAGGGCCGATTCACCATCTCCAGAGACAACGCCAAGAAGTCCCTGTATCTG
CAAATGAACAGTCTGAGAGCTGAGGACACGGCCTTGTATTACTGTGCAAAAGATAT
ACAGTACGGCAACTACTACTACGGTATGGACGTCTGGGGCCAAGGGACCACGGTCA
CCGTCTCCTCAGGAGGTGGTGGATCCGacatccagatgacccagtcccccctccctgtccgcctccgtgggcg
accgggtgaccatcacctgccgggcctcccagggcatctccaactacctgaactggtaccagcagaagcccggcaaggcccccaagcc
cctgatctactacacctccaacctgcagtccggcgtgccctcccggttctccggctccggctccggcaccgactacaccctgaccatctcct
ccctgcagcccgaggacttcgccacctactactgcatgggccagaccatctcctcctacaccttcggccagggcaccaagctggagatca
agggtggcggtggctcgggcggtggtgggtcgggtggcggcggatctgaggtgcagctggtggagtccggcggcggcctggtgcagc
ccggcggctccctgcggctgtcctgcgccgcctccggcttcaccttctccaacttcgacatggcctgggtgcggcaggcccccggcaagg
gcctggtgtgggtgtcctccatcaccaccggcgccgaccacgccatctacgccgactccgtgaagggccggttcaccatctcccgggaca
acgccaagaacaccctgtacctgcagatgaactccctgcgggccgaggacaccgccgtgtactactgcgtgcggcacggctactacgac
ggctaccacctgttcgactactggggccagggcaccctggtgaccgtgtcctccGAGAGCAAGTACGGACCGCCCT
GCCCCCCTTGCCCTATGTTCTGGGTGCTGGTGGTGGTCGGAGGCGTGCTGGCCTGCT
ACAGCCTGCTGGTCACCGTGGCCTTCATCATCTTTTGGGTGAAACGGGGCAGAAAGA
AACTCCTGTATATATTCAAACAACCATTTATGAGACCAGTACAAACTACTCAAGAGG
AAGATGGCTGTAGCTGCCGATTTCCAGAAGAAGAAGGAGGATGTGAACTGCGG
GTGAAGTTCAGCAGAAGCGCCGACGCCCCTGCCTACCAGCAGGGCCAGAATCAGCT
GTACAACGAGCTGAACCTGGGCAGAAGGGAAGAGTACGACGTCCTGGATAAGCGG
AGAGGCCGGGACCCTGAGATGGGCGGCAAGCCTCGGCGGAAGAACCCCCAGGAAG
 
GCCTGTATAACGAACTGCAGAAAGACAAGATGGCCGAGGCCTACAGCGAGATCGGC
ATGAAGGGCGAGCGGAGGCGGGGCAAGGGCCACGACGGCCTGTATCAGGGCCTGT
CCACCGCCACCAAGGATACCTACGACGCCCTGCACATGCAGGCCCTGCCCCCAAGG
TAA (SEQ ID NO:97)
**TOB2** amino acid sequence: 
MALPVTALLLPLALLLHAARPEIVLTQSPATLSLSPGERATLSCRASQSVSSYLAWY
QQKPGQAPRLLIYDASNRATGIPARFSGSGSGTDFTLTISSLEPEDFAVYYCQQRSN
WPITFGQGTRLEIKGSTSGGGSGGGSGGGGSSEVQLVESGGGLVQPGRSLRLSCAA
SGFTFNDYAMHWVRQAPGKGLEWVSTISWNSGSIGYADSVKGRFTISRDNAKKSL
YLQMNSLRAEDTALYYCAKDIQYGNYYYGMDVWGQGTTVTVSSGGGGSDIQMTQ
SPSSLSASVGDRVTITCRASQGISNYLNWYQQKPGKAPKPLIYYTSNLQSGVPSRFSG
SGSGTDYTLTISSLQPEDFATYYCMGQTISSYTFGQGTKLEIKGGGGSGGGGSGGG
GSEVQLVESGGGLVQPGGSLRLSCAASGFTFSNFDMAWVRQAPGKGLVWVSSITT
GADHAIYADSVKGRFTISRDNAKNTLYLQMNSLRAEDTAVYYCVRHGYYDGYHLF
DYWGQGTLVTVSSESKYGPPCPPCPMFWVLVVVGGVLACYSLLVTVAFIIFWVKR
GRKKLLYIFKQPFMRPVQTTQEEDGCSCRFPEEEEGGCELRVKFSRSADAPAYQQ
GQNQLYNELNLGRREEYDVLDKRRGRDPEMGGKPRRKNPQEGLYNELQKDKMA
EAYSEIGMKGERRRGKGHDGLYQGLSTATKDTYDALHMQALPPR (SEQ ID NO:98)
***TN-OF-B20-3 (TOB3)***
CD8a SP nucleic acid sequence 
ATGGCCTTACCAGTGACCGCCTTGCTCCTGCCGCTGGCCTTGCTGCTCCACGCCGCC
AGGCCG (SEQ ID NO:99)
OF V_{H} nucleic acid sequence 
GAAGTGCAGCTGGTGGAGTCTGGGGGAGGCTTGGTACAGCCTGGCAGGTCCCTGAG
ACTCTCCTGTGCAGCCTCTGGATTCACCTTTAATGATTATGCCATGCACTGGGTCCGG
CAAGCTCCAGGGAAGGGCCTGGAGTGGGTCTCAACTATTAGTTGGAATAGTGGTTCC
ATAGGCTATGCGGACTCTGTGAAGGGCCGATTCACCATCTCCAGAGACAACGCCAA
GAAGTCCCTGTATCTGCAAATGAACAGTCTGAGAGCTGAGGACACGGCCTTGTATTA
 
CTGTGCAAAAGATATACAGTACGGCAACTACTACTACGGTATGGACGTCTGGGGCC
AAGGGACCACGGTCACCGTCTCCTCA (SEQ ID NO:100)
Linker-1 nucleic acid sequence
   GGCAGTACTAGCGGTGGTGGCTCCGGGGGCGGTTCCGGTGGGGGCGGCAGCAGC (SEQ ID NO:101)
OF V_{L} nucleic acid sequence 
GAAATTGTGTTGACACAGTCTCCAGCCACCCTGTCTTTGTCTCCAGGGGAAAGAGCC
ACCCTCTCCTGCAGGGCCAGTCAGAGTGTTAGCAGCTACTTAGCCTGGTACCAACAG
AAACCTGGCCAGGCTCCCAGGCTCCTCATCTATGATGCATCCAACAGGGCCACTGGC
ATCCCAGCCAGGTTCAGTGGCAGTGGGTCTGGGACAGACTTCACTCTCACCATCAGC
AGCCTAGAGCCTGAAGATTTTGCAGTTTATTACTGTCAGCAGCGTAGCAACTGGCCG
ATCACCTTCGGCCAAGGGACACGACTGGAGATTAAA (SEQ ID NO:102)
Linker-2 nucleic acid sequence
   GGAGGTGGTGGATCC (SEQ ID NO:103)
B20 V_{L} nucleic acid sequence 
Gacatccagatgacccagtcccccctcctccctgtccgcctccgtgggcgaccgggtgaccatcacctgccgggcctcccagggcatctcc
aactacctgaactggtaccagcagaagcccggcaaggcccccaagcccctgatctactacacctccaacctgcagtccggcgtgccctcc
cggttctccggctccggctccggcaccgactacaccctgaccatctcctccctgcagcccgaggacttcgccacctactactgcatgggcc
agaccatctcctcctacaccttcggccagggcaccaagctggagatcaag (SEQ ID NO:104)
Linker-3 nucleic acid sequence ggtggcggtggctcgggcggtggtgggtcgggtggcggcggatct (SEQ ID NO:105)
B20 V_{H} nucleic acid sequence 
Gaggtgcagctggtggagtccggcggcggcctggtgcagcccggcggctccctgcggctgtcctgcgccgcctccggcttcaccttctc
caacttcgacatggcctgggtgcggcaggcccccggcaagggcctggtgtgggtgtcctccatcaccaccggcgccgaccacgccatct
acgccgactccgtgaagggccggttcaccatctcccgggacaacgccaagaacaccctgtacctgcagatgaactccctgcgggccgag
 
gacaccgccgtgtactactgcgtgcggcacggctactacgacggctaccacctgttcgactactggggccagggcaccctggtgaccgtg
tcctcc (SEQ ID NO:106)
hinge nucleic acid sequence
   GAGAGCAAGTACGGACCGCCCTGCCCCCCTTGCCCT (SEQ ID NO:107)
CD28 TM nucleic acid sequence 
ATGTTCTGGGTGCTGGTGGTGGTCGGAGGCGTGCTGGCCTGCTACAGCCTGCTGGTC
ACCGTGGCCTTCATCATCTTTTGGGTG (SEQ ID NO:108)
4-1BB nucleic acid sequence 
AAACGGGGCAGAAAGAAACTCCTGTATATATTCAAACAACCATTTATGAGACCAGT
ACAAACTACTCAAGAGGAAGATGGCTGTAGCTGCCGATTTCCAGAAGAAGAAGAAG
GAGGATGTGAACTG (SEQ ID NO:109)
CD3z nucleic acid sequence 
CGGGTGAAGTTCAGCAGAAGCGCCGACGCCCCTGCCTACCAGCAGGGCCAGAATCA
GCTGTACAACGAGCTGAACCTGGGCAGAAGGGAAGAGTACGACGTCCTGGATAAGC
GGAGAGGCCGGGACCCTGAGATGGGCGGCAAGCCTCGGCGGAAGAACCCCCAGGA
AGGCCTGTATAACGAACTGCAGAAAGACAAGATGGCCGAGGCCTACAGCGAGATCG
GCATGAAGGGCGAGCGGAGGCGGGGCAAGGGCCACGACGGCCTGTATCAGGGCCT
GTCCACCGCCACCAAGGATACCTACGACGCCCTGCACATGCAGGCCCTGCCCCCAA
GG (SEQ ID NO:110)
***TOB3*** nucleic acid sequence (2130nt) 
ATGGCCTTACCAGTGACCGCCTTGCTCCTGCCGCTGGCCTTGCTGCTCCACGCCGCC
AGGCCGGAAGTGCAGCTGGTGGAGTCTGGGGGAGGCTTGGTACAGCCTGGCAGGTC
CCTGAGACTCTCCTGTGCAGCCTCTGGATTCACCTTTAATGATTATGCCATGCACTGG
GTCCGGCAAGCTCCAGGGAAGGGCCTGGAGTGGGTCTCAACTATTAGTTGGAATAG
TGGTTCCATAGGCTATGCGGACTCTGTGAAGGGCCGATTCACCATCTCCAGAGACAA
CGCCAAGAAGTCCCTGTATCTGCAAATGAACAGTCTGAGAGCTGAGGACACGGCCT
 
TGTATTACTGTGCAAAAGATATACAGTACGGCAACTACTACTACGGTATGGACGTCT
GGGGCCAAGGGACCACGGTCACCGTCTCCTCAGGCAGTACTAGCGGTGGTGGCTCC
GGGGGCGGTTCCGGTGGGGGCGGCAGCAGCGAAATTGTGTTGACACAGTCTCCAGC
CACCCTGTCTTTGTCTCCAGGGGAAAGAGCCACCCTCTCCTGCAGGGCCAGTCAGAG
TGTTAGCAGCTACTTAGCCTGGTACCAACAGAAACCTGGCCAGGCTCCCAGGCTCCT
CATCTATGATGCATCCAACAGGGCCACTGGCATCCCAGCCAGGTTCAGTGGCAGTG
GGTCTGGGACAGACTTCACTCTCACCATCAGCAGCCTAGAGCCTGAAGATTTTGCAG
TTTATTACTGTCAGCAGCGTAGCAACTGGCCGATCACCTTCGGCCAAGGGACACGAC
TGGAGATTAAAGGAGGTGGTGGATCCGacatccagatgacccagtcccctcctccctgtccgcctccgtgggc
gaccgggtgaccatcacctgccgggcctcccagggcatctccaactacctgaactggtaccagcagaagcccggcaaggcccccaagc
ccctgatctactacacctccaacctgcagtccggcgtgccctcccggttctccggctccggctccggcaccgactacaccctgaccatctcc
tccctgcagcccgaggacttcgccacctactactgcatgggccagaccatctcctcctacaccttcggccagggcaccaagctggagatca
agggtggcggtggctcgggcggtggtgggtcgggtggcggcggatctgaggtgcagctggtggagtccggcggcggcctggtgcagc
ccggcggctccctgcggctgtcctgcgccgcctccggcttcaccttctccaacttcgacatggcctgggtgcggcaggcccccggcaagg
gcctggtgtgggtgtcctccatcaccaccggcgccgaccacgccatctacgccgactccgtgaagggccggttcaccatctcccgggaca
acgccaagaacaccctgtacctgcagatgaactccctgcgggccgaggacaccgccgtgtactactgcgtgcggcacggctactacgac
ggctaccacctgttcgactactggggccagggcaccctggtgaccgtgtcctccGAGAGCAAGTACGGACCGCCCT
GCCCCCCTTGCCCTATGTTCTGGGTGCTGGTGGTCGGAGGCGTGCTGGCCTGCT
ACAGCCTGCTGGTCACCGTGGCCTTCATCATCTTTTGGGTGAAACGGGGCAGAAAGA
AACTCCTGTATATATTCAAACAACCATTTATGAGACCAGTACAAACTACTCAAGAGG
AAGATGGCTGTAGCTGCCGATTTCCAGAAGAAGAAGGAGGATGTGAACTGCGG
GTGAAGTTCAGCAGAAGCGCCGACGCCCCTGCCTACCAGCAGGGCCAGAATCAGCT
GTACAACGAGCTGAACCTGGGCAGAAGGGAAGAGTACGACGTCCTGGATAAGCGG
AGAGGCCGGGACCCTGAGATGGGCGGCAAGCCTCGGCGGAAGAACCCCCAGGAAG
GCCTGTATAACGAACTGCAGAAAGACAAGATGGCCGAGGCCTACAGCGAGATCGGC
ATGAAGGGCGAGCGGAGGCGGGGCAAGGGCCACGACGGCCTGTATCAGGGCCTGT
CCACCGCCACCAAGGATACCTACGACGCCCTGCACATGCAGGCCCTGCCCCCAAGG
TAA (SEQ ID NO:111)
***TOB3*** amino acid sequence: MALPVTALLLPLALLLHAARPEVQLVESGGGLVQPGRSLRLSCAASGFTFNDYAM 
HWVRQAPGKGLEWVSTISWNSGSIGYADSVKGRFTISRDNAKKSLYLQMNSLRAE
DTALYYCAKDIQYGNYYYGMDVWGQGTTVTVSSGSTSGGGSGGGSGGGGSSEIVL
TQSPATLSLSPGERATLSCRASQSVSSYLAWYQQKPGQAPRLLIYDASNRATGIPAR
FSGSGSGTDFTLTISSLEPEDFAVYYCQQRSNWPITFGQGTRLEIKGGGGSDIQMTQ
SPSSLSASVGDRVTITCRASQGISNYLNWYQQKPGKAPKPLIYYTSNLQSGVPSRFSG
SGSGTDYTLTISSLQPEDFATYYCMGQTISSYTFGQGTKLEIKGGGGSGGGGSGGG
GSEVQLVESGGGLVQPGGSLRLSCAASGFTFSNFDMAWVRQAPGKGLVWVSSITT
GADHAIYADSVKGRFTISRDNAKNTLYLQMNSLRAEDTAVYYCVRHGYYDGYHLF
DYWGQGTLVTVSSESKYGPPCPPCPMFWVLVVVGGVLACYSLLVTVAFIIFWVKR
GRKKLLYIFKQPFMRPVQTTQEEDGCSCRFPEEEEGGCELRVKFSRSADAPAYQQ
GQNQLYNELNLGRREEYDVLDKRRGRDPEMGGKPRRKNPQEGLYNELQKDKMA
EAYSEIGMKGERRRGKGHDGLYQGLSTATKDTYDALHMQALPPR (SEQ ID NO:112)
***TN-OF-B20-4 (TOB4)***
CD8a SP nucleic acid sequence 
ATGGCCTTACCAGTGACCGCCTTGCTCCTGCCGCTGGCCTTGCTGCTCCACGCCGCC
AGGCCG (SEQ ID NO:113)
OF V_{H} nucleic acid sequence 
GAAGTGCAGCTGGTGGAGTCTGGGGGAGGCTTGGTACAGCCTGGCAGGTCCCTGAG
ACTCTCCTGTGCAGCCTCTGGATTCACCTTTAATGATTATGCCATGCACTGGGTCCGG
CAAGCTCCAGGGAAGGGCCTGGAGTGGGTCTCAACTATTAGTTGGAATAGTGGTTCC
ATAGGCTATGCGGACTCTGTGAAGGGCCGATTCACCATCTCCAGAGACAACGCCAA
GAAGTCCCTGTATCTGCAAATGAACAGTCTGAGAGCTGAGGACACGGCCTTGTATTA
CTGTGCAAAAGATATACAGTACGGCAACTACTACTACGGTATGGACGTCTGGGGCC
AAGGGACCACGGTCACCGTCTCCTCA (SEQ ID NO:114)
Linker-1 nucleic acid sequence
   GGCAGTACTAGCGGTGGTGGCTCCGGGGGCGGTTCCGGTGGGGGCGGCAGCAGC (SEQ ID NO:115)
OF V_{L} nucleic acid sequence 
GAAATTGTGTTGACACAGTCTCCAGCCACCCTGTCTTTGTCTCCAGGGGAAAGAGCC
ACCCTCTCCTGCAGGGCCAGTCAGAGTGTTAGCAGCTACTTAGCCTGGTACCAACAG
AAACCTGGCCAGGCTCCCAGGCTCCTCATCTATGATGCATCCAACAGGGCCACTGGC
ATCCCAGCCAGGTTCAGTGGCAGTGGGTCTGGGACAGACTTCACTCTCACCATCAGC
AGCCTAGAGCCTGAAGATTTTGCAGTTTATTACTGTCAGCAGCGTAGCAACTGGCCG
ATCACCTTCGGCCAAGGGACACGACTGGAGATTAAA (SEQ ID NO:116)
Linker-2 nucleic acid sequence
   GGAGGTGGTGGATCC (SEQ ID NO:117)
B20 V_{H} nucleic acid sequence 
Gaggtgcagctggtggagtccggcggcggcctggtgcagcccggcggctccctgcggctgtcctgcgccgcctccggcttcaccttctc
caacttcgacatggcctgggtgcggcaggcccccggcaagggcctggtgtgggtgtcctccatcaccaccggcgccgaccacgccatct
acgccgactccgtgaagggccggttcaccatctcccgggacaacgccaagaacaccctgtacctgcagatgaactccctgcgggccgag
gacaccgccgtgtactactgcgtgcggcacggctactacgacggctaccacctgttcgactactggggccagggcaccctggtgaccgtg
tcctcc (SEQ ID NO:118)
Linker-3 nucleic acid sequence
   ggtggcggtggctcgggcggtggtgggtcgggtggcggcggatct (SEQ ID NO:119)
B20 V_{L} nucleic acid sequence 
Gacatccagatgacccagtcccctcctccctgtccgcctccgtgggcgaccgggtgaccatcacctgccgggcctcccagggcatctcc
aactacctgaactggtaccagcagaagcccggcaaggcccccaagcccctgatctactacacctccaacctgcagtccggcgtgccctcc
cggttctccggctccggctccggcaccgactacaccctgaccatctcctccctgcagcccgaggacttcgccacctactactgcatgggcc
agaccatctcctcctacaccttcggccagggcaccaagctggagatcaag (SEQ ID NO:120)
hinge nucleic acid sequence
   GAGAGCAAGTACGGACCGCCCTGCCCCCCTTGCCCT (SEQ ID NO:121)
CD28 TM nucleic acid sequence 
ATGTTCTGGGTGCTGGTGGTGGTCGGAGGCGTGCTGGCCTGCTACAGCCTGCTGGTC
ACCGTGGCCTTCATCATCTTTTGGGTG (SEQ ID NO:122)
4-1BB nucleic acid sequence 
AAACGGGGCAGAAAGAAACTCCTGTATATATTCAAACAACCATTTATGAGACCAGT
ACAAACTACTCAAGAGGAAGATGGCTGTAGCTGCCGATTTCCAGAAGAAGAAGAAG
GAGGATGTGAACTG (SEQ ID NO:123)
CD3z nucleic acid sequence 
CGGGTGAAGTTCAGCAGAAGCGCCGACGCCCCTGCCTACCAGCAGGGCCAGAATCA
GCTGTACAACGAGCTGAACCTGGGCAGAAGGGAAGAGTACGACGTCCTGGATAAGC
GGAGAGGCCGGGACCCTGAGATGGGCGGCAAGCCTCGGCGGAAGAACCCCCAGGA
AGGCCTGTATAACGAACTGCAGAAAGACAAGATGGCCGAGGCCTACAGCGAGATCG
GCATGAAGGGCGAGCGGAGGCGGGGCAAGGGCCACGACGGCCTGTATCAGGGCCT
GTCCACCGCCACCAAGGATACCTACGACGCCCTGCACATGCAGGCCCTGCCCCCAA
GG (SEQ ID NO:124)
***TOB4*** nucleic acid sequence (2130nt) 
ATGGCCTTACCAGTGACCGCCTTGCTCCTGCCGCTGGCCTTGCTGCTCCACGCCGCC
AGGCCGGAAGTGCAGCTGGTGGAGTCTGGGGGAGGCTTGGTACAGCCTGGCAGGTC
CCTGAGACTCTCCTGTGCAGCCTCTGGATTCACCTTTAATGATTATGCCATGCACTGG
GTCCGGCAAGCTCCAGGGAAGGGCCTGGAGTGGGTCTCAACTATTAGTTGGAATAG
TGGTTCCATAGGCTATGCGGACTCTGTGAAGGGCCGATTCACCATCTCCAGAGACAA
CGCCAAGAAGTCCCTGTATCTGCAAATGAACAGTCTGAGAGCTGAGGACACGGCCT
TGTATTACTGTGCAAAAGATATACAGTACGGCAACTACTACTACGGTATGGACGTCT
GGGGCCAAGGGACCACGGTCACCGTCTCCTCAGGCAGTACTAGCGGTGGTGGCTCC
GGGGGCGGTTCCGGTGGGGGCGGCAGCAGCGAAATTGTGTTGACACAGTCTCCAGC
CACCCTGTCTTTGTCTCCAGGGGAAAGAGCCACCCTCTCCTGCAGGGCCAGTCAGAG
TGTTAGCAGCTACTTAGCCTGGTACCAACAGAAACCTGGCCAGGCTCCCAGGCTCCT
CATCTATGATGCATCCAACAGGGCCACTGGCATCCCAGCCAGGTTCAGTGGCAGTG
GGTCTGGGACAGACTTCACTCTCACCATCAGCAGCCTAGAGCCTGAAGATTTTGCAG
 
TTTATTACTGTCAGCAGCGTAGCAACTGGCCGATCACCTTCGGCCAAGGGACACGAC
TGGAGATTAAAGGAGGTGGTGGATCCgaggtgcagctggtggagtccggcggcggcctggtgcagcccggcg
gctccctgcggctgtcctgcgccgcctccggcttcaccttctccaacttcgacatggcctgggtgcggcaggcccccggcaaggcctggt
gtgggtgtcctccatcaccaccggcgccgaccacgccatctacgccgactccgtgaagggccggttcaccatctcccgggacaacgcca
agaacaccctgtacctgcagatgaactccctgcgggccgaggacaccgccgtgtactactgcgtgcggcacggctactacgacggctac
cacctgttcgactactggggccagggcaccctggtgaccgtgtcctccggtggcggtggctcgggcggtggtgggtcgggtggcggcgg
atctGacatccagatgacccagtccccctcctccctgtccgcctccgtgggcgaccgggtgaccatcacctgccgggcctcccagggcat
ctccaactacctgaactggtaccagcagaagcccggcaaggcccccaagcccctgatctactacacctccaacctgcagtccggcgtgcc
ctcccggttctccggctccggctccggcaccgactacaccctgaccatctcctccctgcagcccgaggacttcgccacctactactgcatgg
gccagaccatctcctcctacaccttcggccagggcaccaagctggagatcaagGAGAGCAAGTACGGACCGCCCTG
CCCCCCTTGCCCTATGTTCTGGGTGCTGGTGGTGGTCGGAGGCGTGCTGGCCTGCTA
CAGCCTGCTGGTCACCGTGGCCTTCATCATCTTTTGGGTGAAACGGGGCAGAAAGAA
ACTCCTGTATATATTCAAACAACCATTTATGAGACCAGTACAAACTACTCAAGAGGA
AGATGGCTGTAGCTGCCGATTTCCAGAAGAAGAAGGAGGATGTGAACTGCGGG
TGAAGTTCAGCAGAAGCGCCGACGCCCCTGCCTACCAGCAGGGCCAGAATCAGCTG
TACAACGAGCTGAACCTGGGCAGAAGGGAAGAGTACGACGTCCTGGATAAGCGGA
GAGGCCGGGACCCTGAGATGGGCGGCAAGCCTCGGCGGAAGAACCCCCAGGAAGG
CCTGTATAACGAACTGCAGAAAGACAAGATGGCCGAGGCCTACAGCGAGATCGGCA
TGAAGGGCGAGCGGAGGCGGGGCAAGGGCCACGACGGCCTGTATCAGGGCCTGTCC
ACCGCCACCAAGGATACCTACGACGCCCTGCACATGCAGGCCCTGCCCCCAAGGTA
A (SEQ ID NO:125)
***TOB4*** amino acid sequence: 
MALPVTALLLPLALLLHAARPEVQLVESGGGLVQPGRSLRLSCAASGFTFNDYAM
HWVRQAPGKGLEWVSTISWNSGSIGYADSVKGRFTISRDNAKKSLYLQMNSLRAE
DTALYYCAKDIQYGNYYYGMDVWGQGTTVTVSSGSTSGGGSGGGSGGGGSSEIVL
TQSPATLSLSPGERATLSCRASQSVSSYLAWYQQKPGQAPRLLIYDASNRATGIPAR
FSGSGSGTDFTLTISSLEPEDFAVYYCQQRSNWPITFGQGTRLEIKGGGGSEVQLVE
SGGGLVQPGGSLRLSCAASGFTFSNFDMAWVRQAPGKGLVWVSSITTGADIIAIYA
DSVKGRFTISRDNAKNTLYLQMNSLRAEDTAVYYCVRHGYYDGYHLFDYWGQGT
LVTVSSGGGGSGGGGSGGGGSDIQMTQSPSSLSASVGDRVTITCRASQGISNYLNW
 
YQQKPGKAPKPLIYYTSNLQSGVPSRFSGSGSGTDYTLTISSLQPEDFATYYCMGQT
ISSYTFGQGTKLEIKESKYGPPCPPCPMFWVLVVVGGVLACYSLLVTVAFIIFWVKR
GRKKLLYIFKQPFMRPVQTTQEEDGCSCRFPEEEEGGCELRVKFSRSADAPAYQQ
GQNQLYNELNLGRREEYDVLDKRRGRDPEMGGKPRRKNPQEGLYNELQKDKMA
EAYSEIGMKGERRRGKGHDGLYQGLSTATKDTYDALHMQALPPR (SEQ ID
NO:126)
OF-V_{H}-CDR1: NDYAMH (SEQ ID NO: 127)
OF-V_{H}-CDR2: TISWNSGSIGYADSVKG (SEQ ID NO: 128)
OF-V_{H}-CDR3: DIQYGNYYYGMDV (SEQ ID NO: 129)
OF-V_{L}-CDR1: RASQSVSSYLA (SEQ ID NO: 130)
OF-V_{L}-CDR2: DASNRAT (SEQ ID NO: 131)
OF-V_{L}-CDR3: QQRSNWPIT (SEQ ID NO: 132)
**BCMA-20 V_{L}**

| Region | Sequence Fragment | Residues of SEQ ID No:12 | Length | SEQ ID No: |
|---|---|---|---|---|
| LFR1 | DIQMTQSPSSLSASVGDRVTITC | 1 - 23 | 23 | SEQ ID No:133 |
| CDR-L1 | RASQGISNYLN | 24 - 34 | 11 | SEQ ID No:134 |
| LFR2 | WYQQKPGKAPKPLIY | 35 - 49 | 15 | SEQ ID No:135 |
| CDR-L2 | YTSNLQS | 50 - 56 | 7 | SEQ ID No:136 |
| LFR3 | GVPSRFSGSGSGTDYTLTISSLQPEDFATYYC | 57 - 88 | 32 | SEQ ID No:137 |
| CDR-L3 | MGQTISSYT | 89 - 97 | 9 | SEQ ID No:138 |
| LFR4 | FGQGTKLEIK | 98 - 107 | 10 | SEQ ID No:139 |

**BCMA-20 V_{H}**

| Region | Sequence Fragment | Residues of SEQ ID No:16 | Length | SEQ ID No: |
|---|---|---|---|---|
| HFR1 | EVQLVESGGGLVQPGGSLRLSCAASGFTFS | 1 - 30 | 30 | SEQ ID No:140 |
| CDR-H1 | NFDMA | 31 - 35 | 5 | SEQ ID No:141 |
| HFR2 | WVRQAPGKGLVWVS | 36 - 49 | 14 | SEQ ID No:142 |
| CDR-H2 | SITTGADHAIYADSVKG | 50 - 66 | 17 | SEQ ID No:143 |
| HFR3 | RFTISRDNAKNTLYLQMNSLRAEDTAVYYCVR | 67 - 98 | 32 | SEQ ID No:144 |
| CDR-H3 | HGYYDGYHLFDY | 99 - 110 | 12 | SEQ ID No:145 |
| HFR4 | WGQGTLVTVSS | 111 - 121 | 11 | SEQ ID No:146 |

The scope of the present invention is not limited by what has been specifically shown and described hereinabove. Those skilled in the art will recognize that there are suitable alternatives to the depicted examples of materials, configurations, constructions and dimensions. Numerous references, including patents and various publications, are cited and discussed in the description of this invention. The citation and discussion of such references is provided merely to clarify the description of the present invention and is not an admission that any reference is prior art to the invention described herein. All references cited and discussed in this specification are incorporated herein by reference in their entirety. Variations, modifications and other implementations of what is described herein will occur to those of ordinary skill in the art without departing from the spirit and scope of the invention. While certain embodiments of the present invention have been shown and described, it will be obvious to those skilled in the art that changes and modifications may be made without departing from the spirit and scope of the invention. The matter set forth in the foregoing description and accompanying drawings is offered by way of illustration only and not as a limitation.

### EMBODIMENTS

1. A bispecific chimeric antigen receptor (CAR), comprising:
   (i) an anti-CD20 antigen-binding region which comprises a light chain variable region (V_{L}1) and a heavy chain variable region (V_{H}1), wherein V_{L}1 comprises three complementarity determining regions (CDRs), CDR1, CDR2 and CDR3, having amino acid sequences about 80% to about 100% identical to the amino acid sequences set forth in SEQ ID NO: 130, SEQ ID NO: 131, SEQ ID NO: 132, respectively, and wherein V_{H}1 comprises three CDRs, CDR1, CDR2 and CDR3, having amino acid sequences about 80% to about 100% identical to the amino acid sequences set forth in SEQ ID NO: 127, SEQ ID NO: 128, SEQ ID NO: 129, respectively; and
   (ii) an anti-BCMA antigen-binding region which comprises a light chain variable region (V_{L}2) and a heavy chain variable region (V_{H}2), wherein V_{L}2 comprises three complementarity determining regions (CDRs), CDR1, CDR2 and CDR3, having amino acid sequences about 80% to about 100% identical to the amino acid sequences set forth in SEQ ID NO: 134, SEQ ID NO: 136, SEQ ID NO: 138, respectively, and wherein V_{H}2 comprises three CDRs, CDR1, CDR2 and CDR3, having amino acid sequences about 80% to about 100% identical to the amino acid sequences set forth in SEQ ID NO: 141, SEQ ID NO: 143, SEQ ID NO: 145, respectively.
2. A bispecific chimeric antigen receptor (CAR), comprising:
   (i) an anti-CD20 antigen-binding region which comprises a light chain variable region (V_{L}1) and a heavy chain variable region (V_{H}1); and
   (ii) an anti-BCMA antigen-binding region which comprises a light chain variable region (V_{L}2) and a heavy chain variable region (V_{H}2).
3. The bispecific CAR of embodiments 1 or 2, wherein V_{L}1 is located at the N-terminus of V_{H}1.
4. The bispecific CAR of embodiments 1 or 2, wherein V_{L}2 is located at the N-terminus of V_{H}2.
5. The bispecific CAR of embodiments 1 or 2, wherein V_{H}1 is located at the N-terminus of V_{L}1.
6. The bispecific CAR of embodiments 1 or 2, wherein V_{H}2 is located at the N-terminus of V_{L}2.
7. The bispecific CAR of any of embodiments 1-6, wherein V_{L}1 and V_{H}1 have amino acid sequences about 80% to about 100% identical to amino acid sequences set forth in SEQ ID NO: 4 and SEQ ID NO: 8, respectively.
8. The bispecific CAR of any of embodiments 1-7, wherein V_{L}2 and V_{H}2 have amino acid sequences about 80% to about 100% identical to amino acid sequences set forth in SEQ ID NO: 12 and SEQ ID NO: 16, respectively.
9. The bispecific CAR of any of embodiments 1-8, wherein the anti-CD20 antigen-binding region is a single-chain variable fragment (scFv) that specifically binds CD20, and wherein the anti-BCMA antigen-binding region is a scFv that specifically binds BCMA.
10. The bispecific CAR of any of embodiments 1-9, wherein the bispecific CAR further comprises one or more of the following:
   (a) a signal peptide,
   (b) a hinge region,
   (c) a transmembrane domain,
   (d) a co-stimulatory region, and
   (e) a cytoplasmic signaling domain.
11. The bispecific CAR of embodiment 10, wherein the co-stimulatory region comprises a co-stimulatory region of 4-1BB (CD137), CD28, OX40, CD2, CD7, CD27, CD30, CD40, CD70, CD134, PD1, Dap10, CDS, ICAM-1, LFA-1 (CD11a/CD18), ICOS (CD278), NKG2D, GITR, TLR2, or combinations thereof.
12. The bispecific CAR of embodiment 10 or 11, wherein the cytoplasmic signaling domain comprises a cytoplasmic signaling domain of CD3ζ.
13. The bispecific CAR of any of embodiments 10-12, wherein the hinge region comprises a hinge region of IgG4, CD8, CD28, CD137, or combinations thereof.
14. The bispecific CAR of any of embodiments 10-13, wherein the transmembrane domain comprises a transmembrane domain of CD8, CD28, CD3ε, CD45, CD4, CD5, CD9, CD16, CD22, CD33, CD37, CD64, CD80, CD86, CD134, CD137, CD154, or combinations thereof.
15. The bispecific CAR of any of embodiments 1-14, comprising an amino acid sequence about 80% to about 100% identical to the amino acid sequence set forth in SEQ ID NO:26, SEQ ID NO:40, SEQ ID NO:54, SEQ ID NO:68, SEQ ID NO:84, SEQ ID NO:98, SEQ ID NO:112, or SEQ ID NO:126.
16. An immune cell expressing the bispecific CAR of any of embodiments 1-15.
17. The immune cell of embodiment 16, wherein the immune cell is a T cell or a natural killer (NK) cell.
18. A nucleic acid encoding the bispecific CAR of any of embodiments 1-15.
19. A vector comprising the nucleic acid of embodiment 18.
20. A pharmaceutical composition, comprising the bispecific CAR of any of embodiments 1-15, the immune cell of embodiments 16 or 17, the nucleic acid of embodiment 18, or the vector of embodiment 19.
21. A method of treating an autoimmune disorder, the method comprising administering the immune cell of embodiments 16 or 17, or the pharmaceutical composition of embodiment 20, to a subject in need thereof.
22. The method of embodiment 21, wherein the autoimmune disorder is systemic lupus erythematosus (SLE), systemic sclerosis, inflammatory myopathy, systemic scleroderma, multiple sclerosis, myasthenia gravis, a myositis autoantibody-driven disease, or neuromyelitis optica.
23. The method of embodiment 22, wherein the inflammatory myopathy is polymyositis, dermatomyositis, or inclusion-body myositis.
24. The method of embodiment 22, wherein the SLE is lupus nephritis.
25. A method of treating a cancer, the method comprising administering the immune cell of embodiments 16 or 17, or the pharmaceutical composition of embodiment 20, to a subject in need thereof.
26. The method of embodiment 25, wherein the cancer is a hematologic cancer.
27. The method of embodiment 25, wherein the cancer is a B-cell malignancy.
28. The method of embodiment 25, wherein the cancer is Hodgkin's lymphoma, non-Hodgkin's lymphoma, leukemia, and/or multiple myeloma.
29. The method of embodiment 25, wherein the cancer is acute myeloid leukemia (AML), multiple myeloma (MM), chronic lymphocytic leukemia (CLL), chronic myelogenous leukemia, acute lymphoblastic leukemia (ALL), diffuse large B cell lymphoma (DLBCL), or combinations thereof.
30. The method of any of embodiments 21-29, wherein the immune cell is allogeneic or autologous.

## Claims

1. A bispecific chimeric antigen receptor (CAR), comprising:
(i) an anti-CD20 antigen-binding region which comprises a light chain variable region (V_{L}1) and a heavy chain variable region (V_{H}1), wherein V_{L}1 comprises three complementarity determining regions (CDRs), CDR1, CDR2 and CDR3, having amino acid sequences about 80% to about 100% identical to the amino acid sequences set forth in SEQ ID NO: 130, SEQ ID NO: 131, SEQ ID NO: 132, respectively, and wherein V_{H}1 comprises three CDRs, CDR1, CDR2 and CDR3, having amino acid sequences about 80% to about 100% identical to the amino acid sequences set forth in SEQ ID NO: 127, SEQ ID NO: 128, SEQ ID NO: 129, respectively; and
(ii) an anti-BCMA antigen-binding region which comprises a light chain variable region (V_{L}2) and a heavy chain variable region (V_{H}2), wherein V_{L}2 comprises three complementarity determining regions (CDRs), CDR1, CDR2 and CDR3, having amino acid sequences about 80% to about 100% identical to the amino acid sequences set forth in SEQ ID NO: 134, SEQ ID NO: 136, SEQ ID NO: 138, respectively, and wherein V_{H}2 comprises three CDRs, CDR1, CDR2 and CDR3, having amino acid sequences about 80% to about 100% identical to the amino acid sequences set forth in SEQ ID NO: 141, SEQ ID NO: 143, SEQ ID NO: 145, respectively.

2. The bispecific CAR of claim 1, wherein V_{L}1 is located at the N-terminus of V_{H}1, or wherein V_{H}1 is located at the N-terminus of V_{L}1; optionally wherein V_{L}2 is located at the N-terminus of V_{H}2, or wherein V_{H}2 is located at the N-terminus of V_{L}2.

3. The bispecific CAR of claims 1 or 2, wherein V_{L}1 and V_{H}1 have amino acid sequences about 80% to about 100% identical to amino acid sequences set forth in SEQ ID NO: 4 and SEQ ID NO: 8, respectively, optionally wherein V_{L}2 and V_{H}2 have amino acid sequences about 80% to about 100% identical to amino acid sequences set forth in SEQ ID NO: 12 and SEQ ID NO: 16, respectively.

4. The bispecific CAR of any of claims 1-3, wherein the anti-CD20 antigen-binding region is a single-chain variable fragment (scFv) that specifically binds CD20, and wherein the anti-BCMA antigen-binding region is a scFv that specifically binds BCMA.

5. The bispecific CAR of any of claims 1-4, wherein the bispecific CAR further comprises one or more of the following:
(a)
a hinge region,
(b) a transmembrane domain,
(c) a co-stimulatory region, and
(d) a cytoplasmic signaling domain,
optionally wherein the co-stimulatory region comprises a co-stimulatory region of 4-1BB (CD137), CD28, OX40, CD2, CD7, CD27, CD30, CD40, CD70, CD134, PD1, Dap10, CDS, ICAM-1, LFA-1 (CD11a/CD18), ICOS (CD278), NKG2D, GITR, TLR2, or combinations thereof;
optionally wherein the cytoplasmic signaling domain comprises a cytoplasmic signaling domain of CD3ζ;
optionally wherein the hinge region comprises a hinge region of IgG4, CD8, CD28, CD137, or combinations thereof;
optionally wherein the transmembrane domain comprises a transmembrane domain of CD8, CD28, CD3ε, CD45, CD4, CD5, CD9, CD16, CD22, CD33, CD37, CD64, CD80, CD86, CD134, CD137, CD154, or combinations thereof.

6. The bispecific CAR of any of claims 1-5, comprising an amino acid sequence about 80% to about 100% identical to the amino acid sequence set forth in SEQ ID NO:26, SEQ ID NO:40, SEQ ID NO:54, SEQ ID NO:68, SEQ ID NO:84, SEQ ID NO:98, SEQ ID NO:112, or SEQ ID NO:126.

7. An immune cell expressing the bispecific CAR of any of claims 1-6, optionally wherein the immune cell is a T cell or a natural killer (NK) cell.

8. A nucleic acid encoding the bispecific CAR of any of claims 1-6.

9. A vector comprising the nucleic acid of claim 8.

10. A pharmaceutical composition, comprising the bispecific CAR of any of claims 1-6, the immune cell of claim 7, the nucleic acid of claim 8, or the vector of claim 9.

11. A method of treating an autoimmune disorder, the method comprising administering the immune cell of claim 7, or the pharmaceutical composition of claim 10, to a subject in need thereof, optionally wherein the autoimmune disorder is systemic lupus erythematosus (SLE), systemic sclerosis, inflammatory myopathy, systemic scleroderma, multiple sclerosis, myasthenia gravis, a myositis autoantibody-driven disease, or neuromyelitis optica; optionally wherein the inflammatory myopathy is polymyositis, dermatomyositis, or inclusion-body myositis; optionally wherein the subject has lupus nephritis.

12. A method of treating a cancer, the method comprising administering the immune cell of claim 7, or the pharmaceutical composition of claim 10, to a subject in need thereof, optionally wherein the cancer is a hematologic cancer; optionally wherein the cancer is a B-cell malignancy; optionally wherein the cancer is Hodgkin's lymphoma, non-Hodgkin's lymphoma, leukemia, and/or multiple myeloma; optionally wherein the cancer is acute myeloid leukemia (AML), multiple myeloma (MM), chronic lymphocytic leukemia (CLL), chronic myelogenous leukemia, acute lymphoblastic leukemia (ALL), diffuse large B cell lymphoma (DLBCL), or combinations thereof.

13. The method of claim 11 or 12, wherein the immune cell is allogeneic or autologous.
